# EUROPEAN PATENT APPLICATION

(11) **EP 3 919 495 A1**
(43) Date of publication of application: **08.12.2021**
(21) Application number: 20749766.0
(22) Date of filing: 03.02.2020
(51) Int. Cl.: C07D 471/06, C07D 487/06, A61K 31/55, A61P 35/00

(54) **INDOLO HEPTAMYL OXIME ANALOGUE AS PARP INHIBITOR**

(30) Priority: 02.02.2019 CN 201910107947; 12.02.2019 CN 201910111576; 26.07.2019 CN 201910684020
(71) Applicant: Chia Tai Tianqing Pharmaceutical Group Co., Ltd., Lianyungang, Jiangsu 222062 (CN)
(72) Inventor: HU, Yanbin, Shanghai 200131 (CN); LI, Gang, Shanghai 200131 (CN); SUN, Fei, Shanghai 200131 (CN); CHI, Zhigang, Shanghai 200131 (CN); LUO, Jin, Shanghai 200131 (CN); DING, Charles Z., Shanghai 200131 (CN); CHEN, Shuhui, Shanghai 200131 (CN)
(74) Representative: Müller Schupfner & Partner Patent- und Rechtsanwaltspartnerschaft mbB
(86) International application number: PCT/CN2020/074220
(87) International publication number: WO 2020/156577

(57) **Abstract**

Disclosed is a type of indolo heptamyl oxime compounds as a PARP inhibitor. Specifically disclosed are a compound as represented by formula (II) and a pharmaceutically acceptable salt thereof.

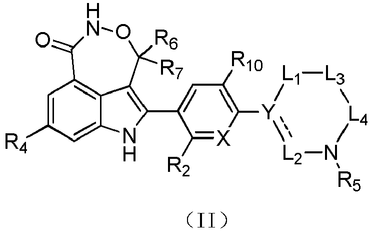

## Description

### CROSS-REFERENCE TO RELATED APPLICATIONS

The present application claims the priority to and the benefit of the Chinese Patent Application No. 201910107947.5 filed with China National Intellectual Property Administration on Feb. 2, 2019, the Chinese Patent Application No. 201910111576.8 filed with China National Intellectual Property Administration on Feb. 12, 2019 and the Chinese Patent Application No. 201910684020.8 filed with China National Intellectual Property Administration on Jul. 26, 2019, the disclosure of each of which is incorporated herein by reference in its entirety.

### TECHNICAL FIELD

The present application relates to a new type of indolo heptamyl oxime compounds as a PARP inhibitor, and in particular to a compound as represented by formula (I), an isomer thereof and a pharmaceutically acceptable salt thereof.

### BACKGROUND

Ploy(ADP-ribose) polymerase (PARP) is a family of enzymes and can be used to catalyze the addition of ADP-ribose residues to a variety of target proteins. To date, a total of 18 subtypes have been identified and characterized. Despite the wide variety of enzymes in the PARP family, PARP-1 is responsible for more than 90% of ADP-ribosylation in cells, and thus PARP-1 inhibitors are the focus of PARP inhibitor research.

In the human living environment, human DNA is always damaged due to the influence of the natural environment (such as oxidative stress, radiotherapy and chemotherapy). PARP-1 is closely related to DNA repair and maintenance of genome function. Upon DNA damage, typically single strand break (SSB), PARP-1 first binds to the DNA break and is then activated, and as the structure of PARP1 enzyme changes, the enzyme begins to recruit NAD+ (coenzyme II) for the synthesis of poly(ADP)ribose, which at the same time serves as a signal for other repair enzymes such as DNA ligase and DNA polymerase β to function. This process of PARP-1 binding and activation is called base excision repair (BER), and contributes to the DNA amplification repair process. When PARP-1 is inhibited by a PARP inhibitor, a broken DNA cannot be repaired through SSB; instead, double strand break (DSB) is activated. The body repairs DSB mainly through two ways: homologous recombination (HR) and non-homologous end joining (NHEJ) of DNA, wherein the homologous recombination is the major way of DSB repair and features high repair reliability. BRCA1 and BRCA2 play important roles in homologous recombination (Nature, 2005, 913-917). Researches show that BRCA1/2 mutation is found in ovarian cancer, breast cancer and prostate cancer, and the PARP inhibitor is a good choice for BRCA1/2-deficient tumors. The PARP inhibitor can be used alone or in combination with chemotherapeutic drugs and radiotherapeutic drugs, thereby reducing the dosage and improving the efficacy. Based on this, a series of different types of compounds (J. Med. Chem. 2010, 4561) have been developed, and among these compounds, olaparib, rucaparib, niraparib (MK-4827) and talazoparib (BMN-673) have been successfully marketed. Nevertheless, as the indications of PARP inhibitors continue to expand, the application of PARP inhibitors is also deepening from treatment of tumor to that of stroke, myocardial ischemia, inflammation and diabetes. A very large number of clinical trials are currently in progress.

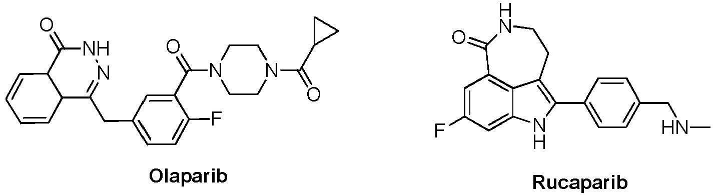

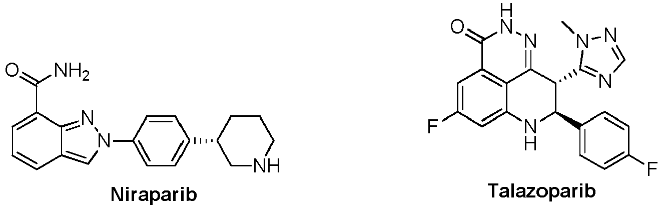

Although efforts to develop PARP inhibitors for the treatment of cancer and other diseases have been ongoing, satisfactory treatment has not been achieved, and thus there is still a pressing need to develop new PARP inhibitors.

### SUMMARY OF THE INVENTION

The present application provides a compound of formula (II), an isomer thereof or a pharmaceutically acceptable salt thereof, 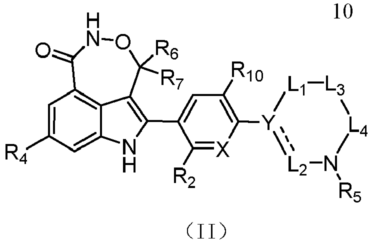 wherein,
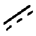 is selected from the group consisting of a single bond and a double bond;
X is selected from the group consisting of CR₃ and N;
Y is selected from the group consisting of CR₁ and C;
L₁ is selected from the group consisting of a single bond and -(CR₈R₉)ₙ-;
L₂ is selected from the group consisting of a single bond, -CR₈R₉- and =CH-;
L₁ and L₂ are not single bonds at the same time;
when L₂ is selected from a single bond, 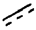 is selected from a single bond;
L₃ and L₄ are each independently selected from -CR₈R₉-;
n is 1 or 2;
R₁ is selected from the group consisting of H, D, F, Cl, Br, I and C₁₋₃ alkyl, wherein the C₁₋₃ alkyl is optionally substituted with 1, 2 or 3 Rₐ, and when L₂ is selected from =CH-, R₁ is absent;
R₂ and R₁₀ are each independently selected from the group consisting of H, F, Cl, Br, I and C₁₋₃ alkyl, wherein the C₁₋₃ alkyl is optionally substituted with 1, 2 or 3 R_{b};
R₃ is selected from the group consisting of H, F, Cl, Br, I, CN and C₁₋₃ alkyl, wherein the C₁₋₃ alkyl is optionally substituted with 1, 2 or 3 R_{c};
R₄ is selected from the group consisting of H and F;
R₅ is selected from the group consisting of H and C₁₋₃ alkyl, wherein the C₁₋₃ alkyl is optionally substituted with 1, 2 or 3 R_{d};
R₆ and R₇ are each independently selected from the group consisting of H and D;
R₈ and R₉ are each independently selected from the group consisting of H, F, Cl, Br, I and C₁₋₃ alkyl, wherein the C₁₋₃ alkyl is optionally substituted with 1, 2 or 3 Rₑ, or
R₈ and R₉, together with a same carbon atom connected thereto, form ring A optionally substituted with 1, 2 or 3 R_{g};
ring A is selected from the group consisting of C₃₋₈ cycloalkyl and 3-8 membered heterocycloalkyl;
Rₐ, R_{b}, R_{c}, R_{d}, Rₑ and R_{g} are each independently selected from the group consisting of F, Cl, Br, I, OH, CN, NH₂, COOH, C(=O)NH₂, CH₃, CH₃CH₂, CF₃, CHF₂, CH₂F, NHCH₃ and N(CH₃)₂;
the 3-8 membered heterocycloalkyl comprises 1, 2, 3 or 4 atoms or groups of atoms each independently selected from the group consisting of O, N, S and NH.

In some embodiments of the present application, provided is the compound, the isomer thereof or the pharmaceutically acceptable salt thereof described above, which is selected from formula (II-1) 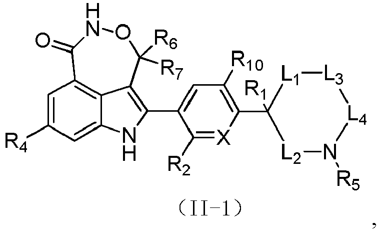 wherein
R₁, R₂, X, R₄, R₅, R₆, R₇, R₁₀, L₁, L₂, L₃ and L₄ are as defined in the compound of formula (II) disclosed herein.

In some embodiments of the present application, provided is the compound, the isomer thereof or the pharmaceutically acceptable salt thereof described above, which is selected from formula (II-1') 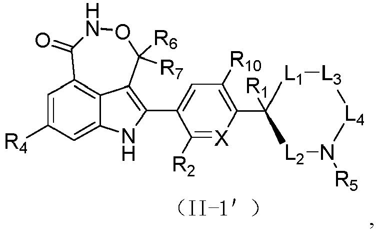 wherein
R₁, R₂, X, R₄, R₅, R₆, R₇, R₁₀, L₁, L₂, L₃ and L₄ are as defined in the compound of formula (II) disclosed herein.

In some embodiments of the present application, provided is the compound, the isomer thereof or the pharmaceutically acceptable salt thereof described above, which is selected from formula (II-1") 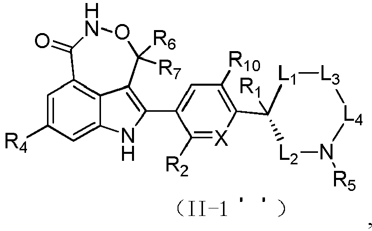 wherein
R₁, R₂, X, R₄, R₅, R₆, R₇, R₁₀, L₁, L₂, L₃ and L₄ are as defined in the compound of formula (II) disclosed herein.

In some embodiments of the present application, provided is the compound, the isomer thereof or the pharmaceutically acceptable salt thereof described above, which is selected from formula (II-1-a) 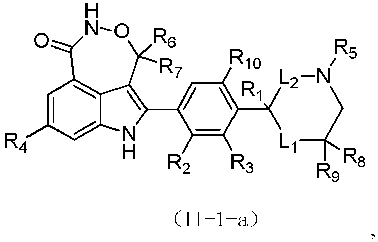 wherein
R₁, R₂, R₃, R₄, R₅, R₆, R₇, R₈, R₉, R₁₀, L₁ and L₂ are as defined in the compound of formula (II) disclosed herein. In some embodiments of the present application, provided is the compound, the isomer thereof or the pharmaceutically acceptable salt thereof described above, which is selected from formula (II-1-a-1) 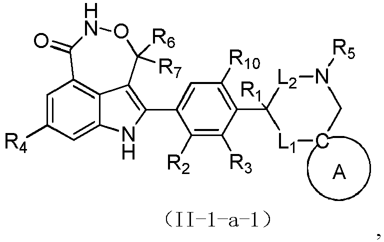 wherein
R₁, R₂, R₃, R₄, R₅, R₆, R₇, R₈, R₉, R₁₀, L₁, L₂ and ring A are as defined in the compound of formula (II) disclosed herein.

In some embodiments of the present application, provided is the compound, the isomer thereof or the pharmaceutically acceptable salt thereof described above, which is selected from formula (II-1-a-2) 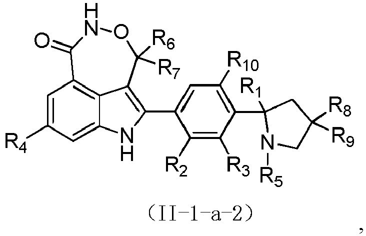 wherein
R₁, R₂, R₃, R₄, R₅, R₆, R₇, R₈, R₉ and R₁₀ are as defined in the compound of formula (II) disclosed herein.

In some embodiments of the present application, provided is the compound, the isomer thereof or the pharmaceutically acceptable salt thereof described above, which is selected from formula (II-1-a-2') 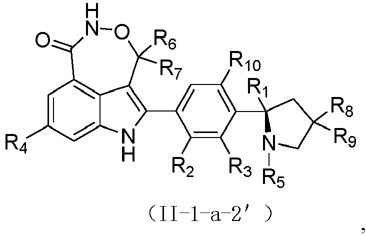 wherein
R₁, R₂, R₃, R₄, R₅, R₆, R₇, R₈, R₉ and R₁₀ are as defined in the compound of formula (II) disclosed herein.

In some embodiments of the present application, provided is the compound, the isomer thereof or the pharmaceutically acceptable salt thereof described above, which is selected from formula (II-1-a-2") 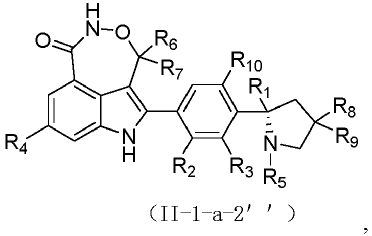 wherein
R₁, R₂, R₃, R₄, R₅, R₆, R₇, R₈, R₉ and R₁₀ are as defined in the compound of formula (II) disclosed herein.

In some embodiments of the present application, provided is the compound, the isomer thereof or the pharmaceutically acceptable salt thereof described above, which is selected from formula (II-2) 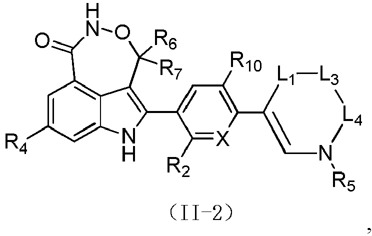 wherein
R₂, X, R₄, R₅, R₆, R₇, R₁₀, L₁, L₃ and L₄ are as defined herein.

In some embodiments of the present application, provided is the compound, the isomer thereof or the pharmaceutically acceptable salt thereof described above, wherein Y is selected from CR₁ and other variables are as defined herein.

In some embodiments of the present application, provided is the compound, the isomer thereof or the pharmaceutically acceptable salt thereof described above, wherein R₁ is selected from the group consisting of H, D, F and CH₃, and other variables are as defined herein.

In some embodiments of the present application, provided is the compound, the isomer thereof or the pharmaceutically acceptable salt thereof described above, wherein R₂ and R₁₀ are each independently selected from the group consisting of H and F, and other variables are as defined herein.

In some embodiments of the present application, provided is the compound, the isomer thereof or the pharmaceutically acceptable salt thereof described above, wherein X is selected from CR₃, and other variables are as defined herein.

In some embodiments of the present application, provided is the compound, the isomer thereof or the pharmaceutically acceptable salt thereof described above, wherein R₃ is selected from the group consisting of H, F, CN, Cl and CF₃, and other variables are as defined herein.

In some embodiments of the present application, provided is the compound, the isomer thereof or the pharmaceutically acceptable salt thereof described above, wherein R₃ is selected from the group consisting of H and F, and other variables are as defined herein.

In some embodiments of the present application, provided is the compound, the isomer thereof or the pharmaceutically acceptable salt thereof described above, wherein R₅ is selected from the group consisting of H, methyl, ethyl, propyl and isopropyl, the methyl, ethyl, propyl and isopropyl being optionally substituted with 1, 2 or 3 R_{d}, and other variables are as defined herein.

In some embodiments of the present application, provided is the compound, the isomer thereof or the pharmaceutically acceptable salt thereof described above, wherein R₅ is selected from the group consisting of H, methyl, 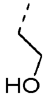 and isopropyl, and other variables are as defined herein.

In some embodiments of the present application, provided is the compound, the isomer thereof or the pharmaceutically acceptable salt thereof described above, wherein L₁ is selected from the group consisting of a single bond and -CR₈R₉-, and other variables are as defined herein.

In some embodiments of the present application, provided is the compound, the isomer thereof or the pharmaceutically acceptable salt thereof described above, wherein L₁ is selected from -CR₈R₉-, L₂ is selected from -CR₈R₉-, and other variables are as defined herein.

In some embodiments of the present application, provided is the compound, the isomer thereof or the pharmaceutically acceptable salt thereof described above, wherein L₁ is selected from a single bond, L₂ is selected from -CR₈R₉-, and other variables are as defined herein.

In some embodiments of the present application, provided is the compound, the isomer thereof or the pharmaceutically acceptable salt thereof described above, wherein L₁ is selected from -CR₈R₉-, L₂ is selected from a single bond, and other variables are as defined herein.

In some embodiments of the present application, provided is the compound, the isomer thereof or the pharmaceutically acceptable salt thereof described above, wherein L₁ is selected from a single bond, L₂ is selected from =CH, and other variables are as defined herein. In some embodiments of the present application, provided is the compound, the isomer thereof or the pharmaceutically acceptable salt thereof described above, wherein L₁ is selected from -(CR₈R₉)₂-, L₂ is selected from a single bond, and other variables are as defined herein.

In some embodiments of the present application, provided is the compound, the isomer thereof or the pharmaceutically acceptable salt thereof described above, wherein R₈ and R₉ are each independently selected from the group consisting of H and F, and other variables are as defined herein.

In some embodiments of the present application, provided is the compound, the isomer thereof or the pharmaceutically acceptable salt thereof described above, wherein R₈ and R₉ are both selected from H, and other variables are as defined herein.

In some embodiments of the present application, provided is the compound, the isomer thereof or the pharmaceutically acceptable salt thereof described above, wherein one of R₈ and R₉ is selected from H and the other is selected from F, and other variables are as defined herein.

In some embodiments of the present application, provided is the compound, the isomer thereof or the pharmaceutically acceptable salt thereof described above, wherein ring A is selected from 5-6 membered heterocycloalkyl, the 5-6 membered heterocycloalkyl being optionally substituted with 1, 2 or 3 R_{g}, and other variables are as defined herein.

In some embodiments of the present application, provided is the compound, the isomer thereof or the pharmaceutically acceptable salt thereof described above, wherein ring A is selected from 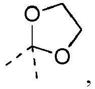 and other variables are as defined herein. In some embodiments of the present application, provided is the compound, the isomer thereof or the pharmaceutically acceptable salt thereof described above, wherein R₆ and R₇ are both H, and other variables are as defined herein.

In some embodiments of the present application, provided is the compound, the isomer thereof or the pharmaceutically acceptable salt thereof described above, wherein R₆ and R₇ are both D, and other variables are as defined herein.

In some embodiments of the present application, provided is the compound, the isomer thereof or the pharmaceutically acceptable salt thereof described above, wherein Rₐ, R_{b}, R_{c}, R_{d}, Rₑ and R_{g} are each independently selected from the group consisting of F and OH.

In some embodiments of the present application, provided is the compound, the isomer thereof or the pharmaceutically acceptable salt thereof described above, wherein the structural unit 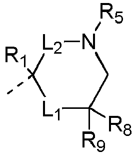 is selected from the group consisting of 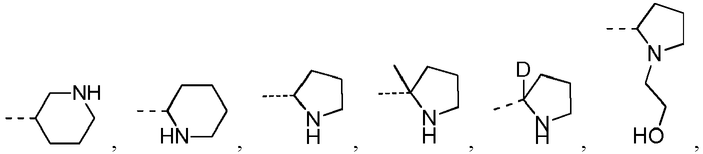 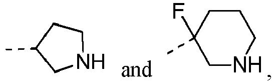 and other variables are as defined herein.

In some embodiments of the present application, provided is the compound, the isomer thereof or the pharmaceutically acceptable salt thereof described above, wherein the structural unit 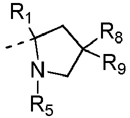 is selected from the group consisting of 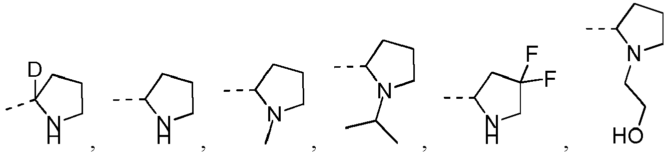 and 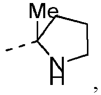 and other variables are as defined herein.

In some embodiments of the present application, provided is the compound, the isomer thereof or the pharmaceutically acceptable salt thereof described above, wherein the structural unit 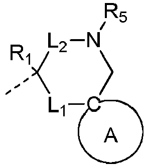 is selected from 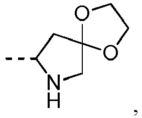 and other variables are as defined herein.

In some embodiments of the present application, provided is the compound, the isomer thereof or the pharmaceutically acceptable salt thereof described above, wherein the structural unit 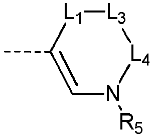 is selected from 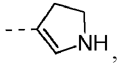 and other variables are as defined herein.

In some embodiments of the present application, provided is the compound, the isomer thereof or the pharmaceutically acceptable salt thereof described above, wherein the structural unit 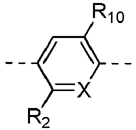 is selected from the group consisting of 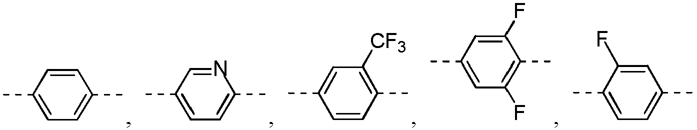 and 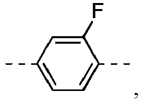 and other variables as defined herein.

In some embodiments of the present application, provided is the compound, the isomer thereof or the pharmaceutically acceptable salt thereof described above, wherein the structural unit 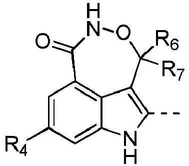 is selected from the group consisting of 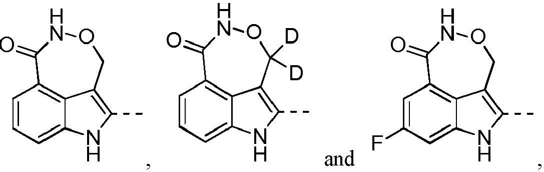 and other variables are as defined herein.

The present application provides a compound of formula (I), an isomer thereof or a pharmaceutically acceptable salt thereof, 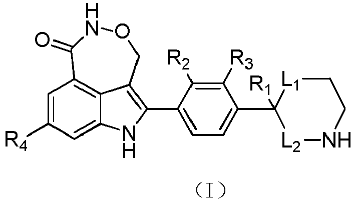 wherein,
L₁ and L₂ are each independently selected from the group consisting of a single bond and -CH₂-, and L₁ and L₂ are not single bonds at the same time;
R₁ is selected from the group consisting of H, D and C₁₋₃ alkyl, wherein the C₁₋₃ alkyl is optionally substituted with 1, 2 or 3 R_{d};
R₂ is selected from the group consisting of H, halogen and C₁₋₃ alkyl, wherein the C₁₋₃ alkyl is optionally substituted with 1, 2 or 3 R_{b};
R₃ is selected from the group consisting of H, halogen and C₁₋₃ alkyl, wherein the C₁₋₃ alkyl is optionally substituted with 1, 2 or 3 R_{c};
R₄ is selected from the group consisting of H and F;
Rₐ, R_{b} and R_{c} are each independently selected from the group consisting of F, Cl, Br, I, OH, CN, NH₂, COOH, C(=O)NH₂, CH₃, CH₃CH₂, CF₃, CHF₂, CH₂F, NHCH₃ and N(CH₃)₂.

In some embodiments of the compound of formula (I) disclosed herein, R₁ is selected from the group consisting of H, D and CH₃.

In some embodiments of the compound of formula (I) disclosed herein, R₂ is selected from the group consisting of H and F.

In some embodiments of the compound of formula (I) disclosed herein, R₃ is selected from the group consisting of H and F.

In some embodiments of the compound of formula (I) disclosed herein, the structural unit 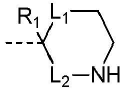 is selected from the group consisting of 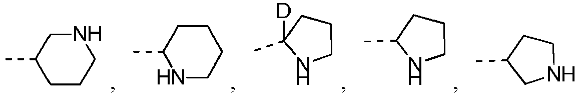 and

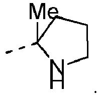

In some embodiments of the compound of formula (I) disclosed herein, the structural unit 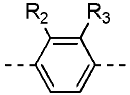 is selected from the group consisting of

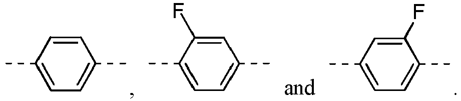

In some embodiments of the compound of formula (I) disclosed herein, R₁ is selected from the group consisting of H, D and CH₃, and other variables are as defined herein.

In some embodiments of the compound of formula (I) disclosed herein, R₂ is selected from the group consisting of H and F, and other variables are as defined herein.

In some embodiments of the compound of formula (I) disclosed herein, R₃ is selected from the group consisting of H and F, and other variables are as defined herein.

In some embodiments of the compound of formula (I) disclosed herein, the structural unit 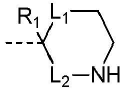 is selected from the group consisting of 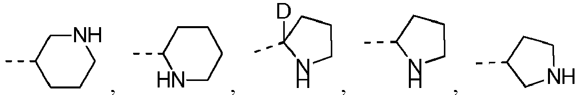 and 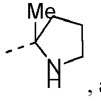 and other variables are as defined herein.

In some embodiments of the compound of formula (I) disclosed herein, the structural unit 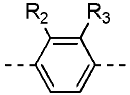 is selected from the group consisting of 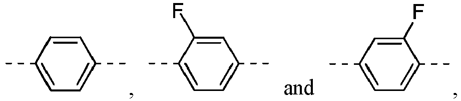 and other variables are as defined herein.

In some embodiments of the compound of formula (I) disclosed herein, provided is the compound, the isomer thereof, or the pharmaceutically acceptable salt thereof described above, wherein the compound is selected from the group consisting of 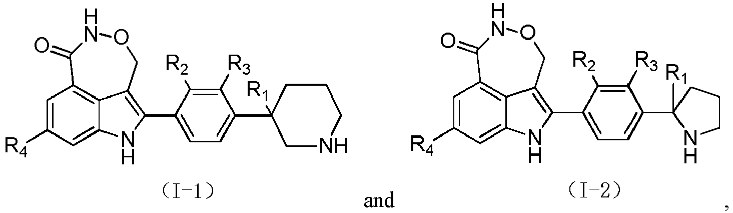 wherein R₁, R₂, R₃ and R₄ are as defined herein.

The present application also provides a compound of a formula below, an isomer thereof or a pharmaceutically acceptable salt thereof, selected from the group consisting of

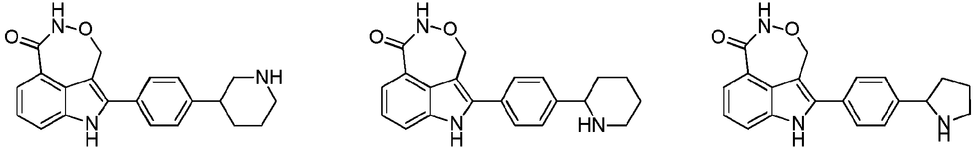

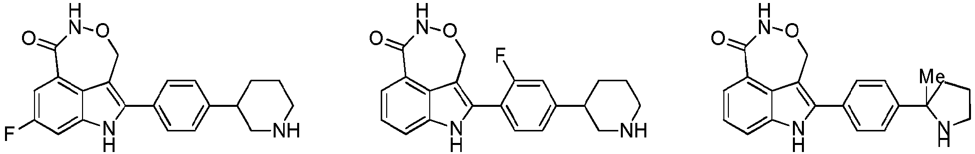

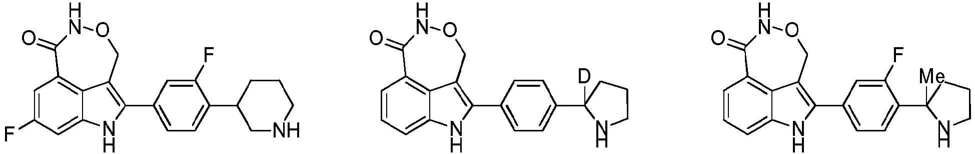

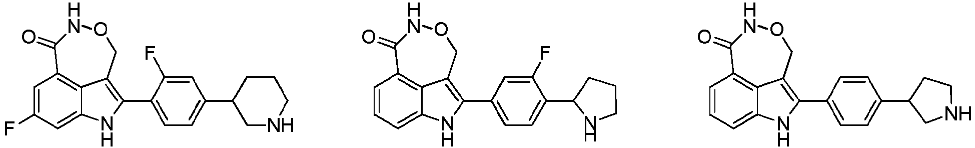

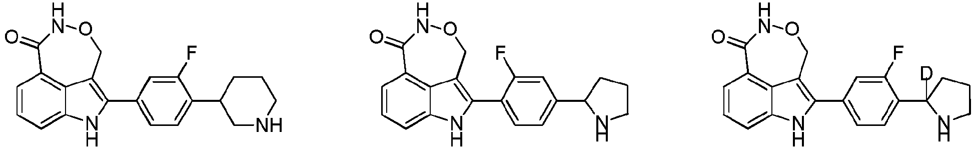

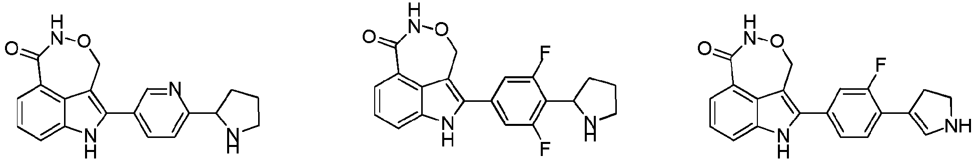

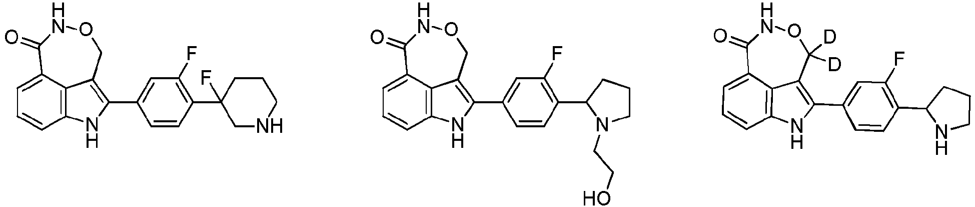

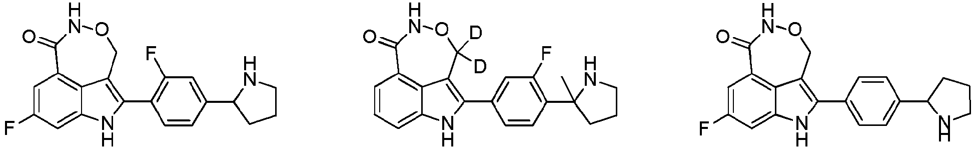

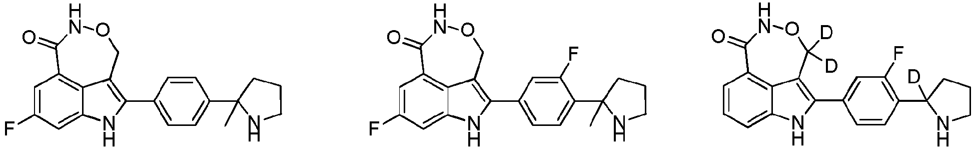

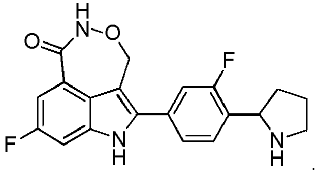

In some embodiments of the present application, provided is the compound, the isomer thereof or the pharmaceutically acceptable salt thereof, selected from the group consisting of

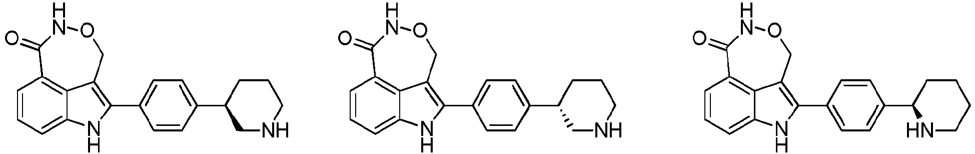

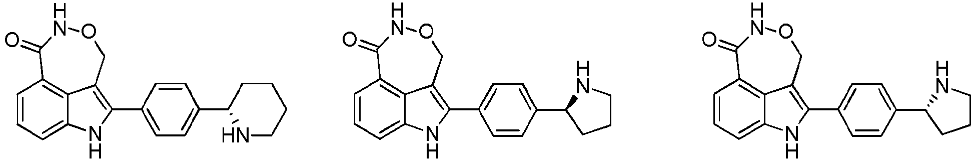

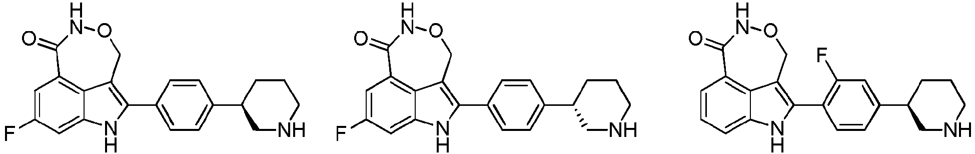

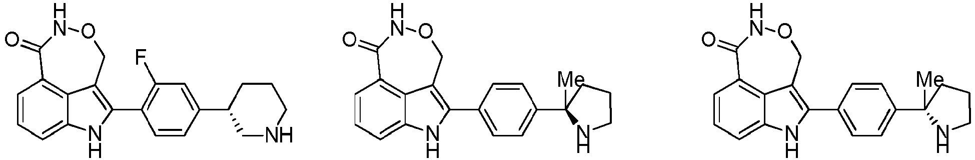

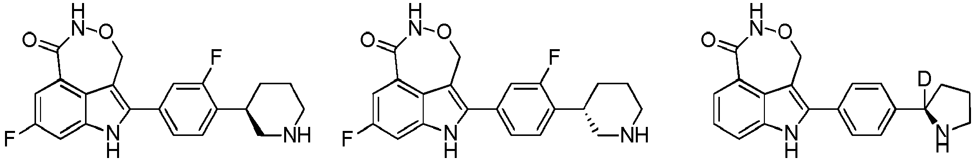

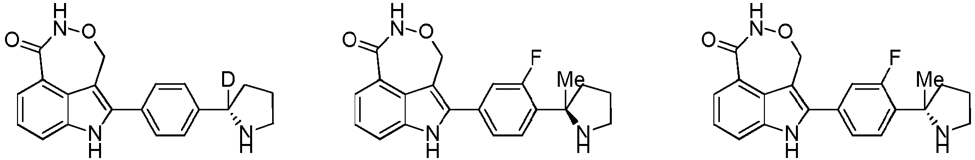

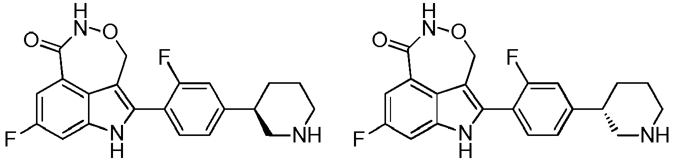

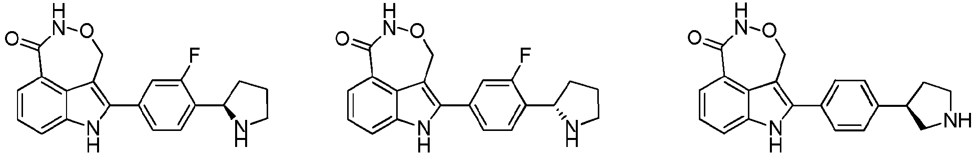

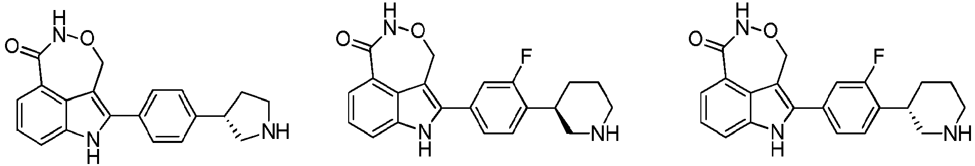

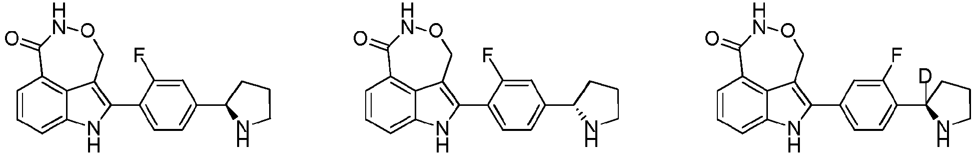

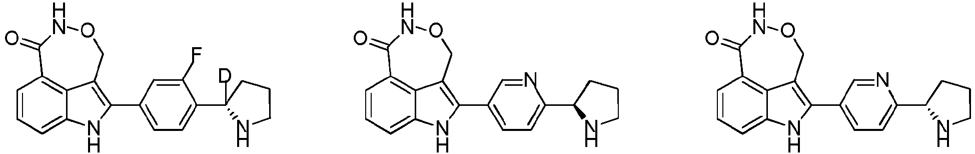

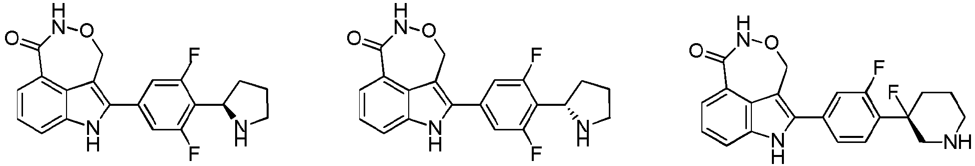

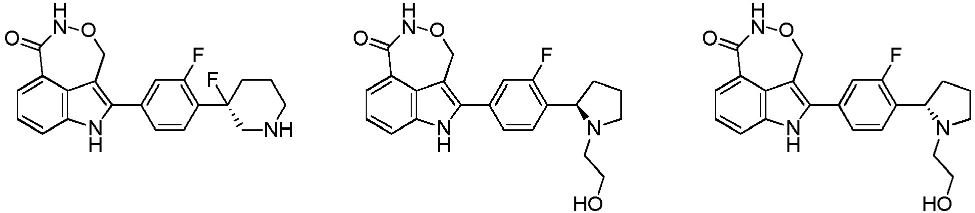

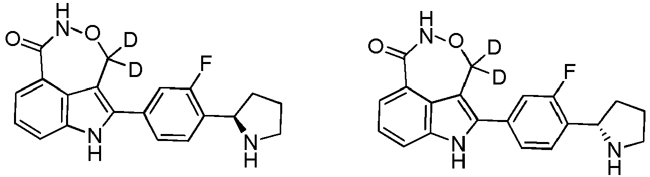

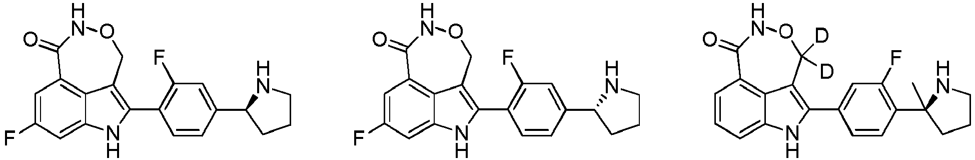

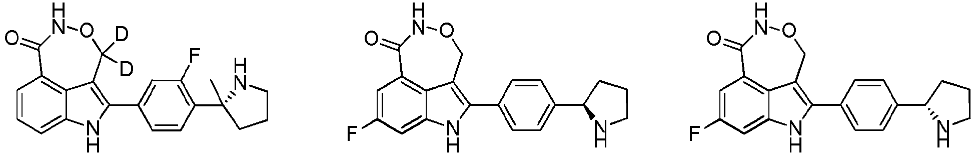

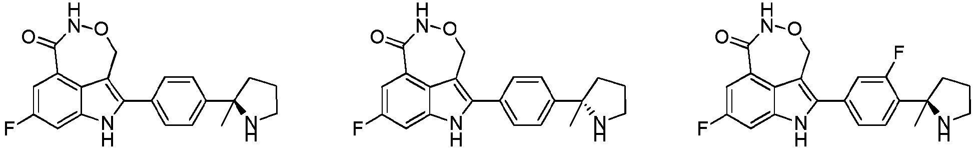

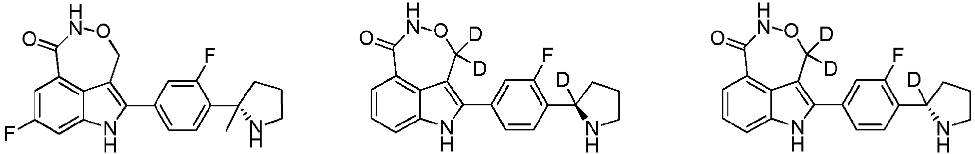

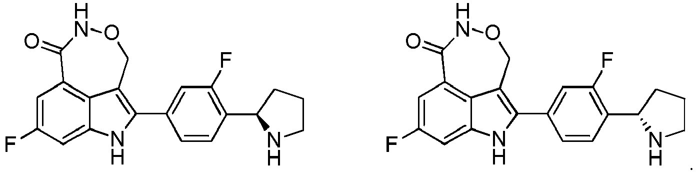

The present application further provides a pharmaceutical composition comprising a therapeutically effective amount of the compound, the isomer thereof or the pharmaceutically acceptable salt thereof disclosed herein and a pharmaceutically acceptable carrier.

The present application further provides use of the compound, the isomer thereof or the pharmaceutically acceptable salt thereof disclosed herein in preparing a medicament for use in treating a disease related to PARP receptor.

The present application further provides a method for treating a disease related to PARP receptor, comprising administering to a mammal, preferably a human, in need of such treatment a therapeutically effective amount of the compound, the isomer thereof or the pharmaceutically acceptable salt thereof disclosed herein.

The present application further provides use of the compound, the isomer thereof or the pharmaceutically acceptable salt thereof disclosed herein in treating a disease related to PARP receptor.

The present application further provides the compound of formula (II), the isomer thereof or the pharmaceutically acceptable salt thereof disclosed herein for use in treating a disease related to PARP receptor.

In some embodiments of the present application, the disease related to PARP receptor is selected from the group consisting of a tumor and a cancer.

In some embodiments of the present application, the disease related to PARP receptor is selected from breast cancer.

Some other embodiments of the present application are derived from any combination of the variables as described above.

### Technical Effects

The compound disclosed herein has strong inhibitory activity against PARP1 kinase and excellent anti-proliferative activity against BRCA1-mutated MDA-MB-436 cells, and meanwhile, it has no inhibitory activity against BRCA-wild type MDA-MB-231 cells, showing that the compound disclosed herein is excellent in selectivity and safety. The compound disclosed herein also has certain inhibitory effect against poly ADP-ribosylation. In addition, the compound disclosed herein has excellent pharmacokinetic properties as it is stable in metabolism *in vivo* and high in bioavailability. In general, the compound disclosed herein is not only excellent in activity and easy to synthesize, but also excellent in pharmacokinetic properties.

### Definitions and Description

Unless otherwise stated, the following terms and phrases used herein are intended to have the following meanings. A particular term or phrase, unless otherwise specifically defined, should not be considered as uncertain or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound disclosed herein, which is prepared from the compound having particular substituents disclosed herein and a relatively nontoxic acid or base. When the compound disclosed herein contains a relatively acidic functional group, a base addition salt can be obtained by contacting such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound disclosed herein contains a relatively basic functional group, an acid addition salt can be obtained by contacting such a compound with a sufficient amount of an acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid and phosphorous acid; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid and methanesulfonic acid. Also included are salts of amino acids (e.g., arginine) and salts of organic acids such as glucuronic acid. Certain specific compounds disclosed herein contain both basic and acidic functional groups that allow the compounds to be converted into either base or acid addition salts.

The pharmaceutically acceptable salts disclosed herein can be synthesized from a parent compound having an acidic or basic group by conventional chemical methods. In general, such salts are prepared by the following method: the free acid or base form of the compound reacting with a stoichiometric amount of the appropriate base or acid in water or an organic solvent or a mixture thereof.

The compounds disclosed herein can be in the form of a geometric isomer or stereoisomer. All such compounds are contemplated herein, including cis and trans isomers, (-)- and (+)- enantiomers, (R)- and (S)- enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present application. Substituents such as alkyl may have an additional asymmetric carbon atom. All these isomers and mixtures thereof are encompassed within the scope of the present application.

Unless otherwise stated, the absolute configuration of a stereogenic center is represented by a wedged solid bond ( 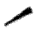 ) and a wedged dashed bond ( 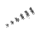 ), and the relative configuration of a stereogenic center is represented by a straight solid bond ( 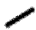 ) and a straight dashed bond ( 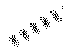 ). A wavy line ( 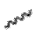 ) represents a wedged solid bond ( 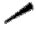 ) or a wedged dashed bond ( 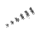 ), or a wavy line ( 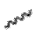 ) represents a straight solid bond ( 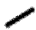 ) and a straight dashed bond ( 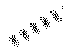 ).

Optically active (R)- and (S)-isomers and *D* and *L* isomers can be prepared by chiral synthesis or chiral reagents or other conventional techniques. An enantiomer of certain compound disclosed herein can be prepared by asymmetric synthesis or derivatization using a chiral auxiliary, wherein the resulting diastereoisomeric mixture is separated and the auxiliary group is cleaved so as to provide the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to get the pure enantiomer. Furthermore, the enantiomer and the diastereoisomer are generally isolated through chromatography using a chiral stationary phase, optionally in combination with chemical derivatization (e.g., carbamate generated from amines).

The compound disclosed herein may contain an unnatural proportion of atomic isotope at one or more of the atoms that constitute the compound. For example, the compound may be labeled with a radioisotope, such as tritium (D, ³H), iodine-125 (¹²⁵I), or C-14 (¹⁴C). For another example, hydrogen can be substituted by deuterium to form a deuterated drug, and the bond formed by deuterium and carbon is firmer than that formed by common hydrogen and carbon. Compared with an un-deuterated drug, the deuterated drug has the advantages of reduced toxic side effect, increased stability, enhanced efficacy, prolonged biological half-life and the like. All isotopic variations of the compound described herein, whether radioactive or not, are encompassed within the scope of the present application.

"Optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted by substituents which may include deuterium and hydrogen variants, as long as the valence of the specific atom is normal and the substituted compound is stable. When the substituent is an oxygen (i.e., =O), it means that two hydrogen atoms are substituted. Substitution by oxygen does not occur on aromatic groups. The term "optionally substituted" means that an atom can be substituted by a substituent or not. Unless otherwise specified, the type and number of the substituent may be arbitrary as long as being chemically achievable.

When any variable (e.g., R) occurs more than once in the constitution or structure of a compound, the variable is independently defined in each case. Thus, for example, if a group is substituted by 0-2 R, the group can be optionally substituted by two R at most, and the definition of R in each case is independent. Furthermore, a combination of a substituent and/or a variant thereof is permissible only if the combination can result in a stable compound.

When the number of a linking group is 0, for example, -(CRR)₀-, it means that the linking group is a single bond. When one of variants is selected from single bond, then two groups bonding by this variant are bonded directly. For example, in A-L-Z, when L represents a single bond, it means that the structure is actually A-Z.

When a substituent is absent, it means that the substituent does not exist. For example, when X in A-X is absent, the structure is actually A.

Unless otherwise specified, when a group has one or more connectable sites, any one or more of the sites of the group may be connected to other groups by chemical bonds. The chemical bond that connects the site to another group may be represented by a straight solid bond ( 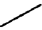 ), a straight dashed line bond ( 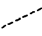 ), or a wavy line 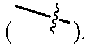 For example, the solid straight line in -OCH₃ indicates that the group is connected to another group through the oxygen atom; in 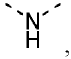 the straight dashed line indicates that the group is connected to another group through the two ends of the nitrogen atom; in 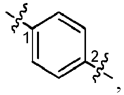 the wavy line indicates that the phenyl group is connected to other groups through the carbon atoms on positions 1 and 2.

Unless otherwise specified, the number of atoms on a ring is generally defined as the member number of the ring. For example, "5-7 membered ring" refers to a "ring" on which 5 to 7 atoms are arranged in a circle.

Unless otherwise specified, "C₃₋₈ cycloalkyl" refers to a saturated cyclic hydrocarbon group consisting of 3 to 8 carbon atoms. This includes monocyclic and bicyclic systems, wherein the bicyclic system includes spirocyclic, fused and bridged rings. The C₃₋₈ cycloalkyl includes C₃₋₆, C₃₋₅, C₄₋₈, C₄₋₆, C₄₋₅, C₅₋₈, C₅₋₆ cycloalkyl, or the like, and may be monovalent, divalent, or polyvalent. Examples of C₃₋₈ cycloalkyl include, but are not limited to, cyclopropyl, cyclobutyl, cyclopentyl, cyclohexyl, cycloheptyl, norbornyl, [2.2.2]bicyclooctane, and the like.

Unless otherwise specified, the term "3-8 membered heterocycloalkyl", by itself or in combination with other terms, refers to a saturated cyclic group consisting of 3 to 8 ring atoms, of which 1, 2, 3, or 4 ring atoms are heteroatoms independently selected from the group consisting of O, S, and N, with the remaining being carbon atoms. The nitrogen atom is optionally quaternized, and the carbon, nitrogen and sulfur heteroatoms can be optionally oxidized (i.e., C=O, NO and S(O)ₚ, wherein p is 1 or 2). This includes monocyclic and bicyclic systems, wherein the bicyclic system includes spirocyclic, fused, and bridged rings. Furthermore, with respect to the "3-8 membered heterocycloalkyl", a heteroatom may occupy the position where the heterocycloalkyl is connected to the rest of the molecule. The 3-8 membered heterocycloalkyl includes 3-6 membered, 3-5 membered, 4-6 membered, 5-6 membered, 4 membered, 5 membered and 6 membered heterocycloalkyl, and the like. Examples of 3-8 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, or the like.

Unless otherwise specified, the term "5-6 membered heterocycloalkyl", by itself or in combination with other terms, refers to a saturated cyclic group consisting of 5 to 6 ring atoms, of which 1, 2, 3, or 4 ring atoms are heteroatoms independently selected from the group consisting of O, S, and N, with the remaining being carbon atoms. The nitrogen atom is optionally quaternized, and the carbon, nitrogen and sulfur heteroatoms can be optionally oxidized (i.e., C=O, NO and S(O)ₚ, wherein p is 1 or 2). This includes monocyclic and bicyclic systems, wherein the bicyclic system includes spirocyclic, fused, and bridged rings. Furthermore, with respect to the "5-6 membered heterocycloalkyl", a heteroatom may occupy the position where the heterocycloalkyl is connected to the rest of the molecule. The 5-6 membered heterocycloalkyl includes 5-membered heterocycloalkyl and 6-membered heterocycloalkyl. Examples of 5-6 membered heterocycloalkyl include, but are not limited to, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, or the like.

Unless otherwise specified, the term "C₁₋₃ alkyl" refers to a linear or branched saturated hydrocarbon group consisting of 1 to 3 carbon atoms. The C₁₋₃ alkyl includes, but is not limited to, C₁₋₂ and C₂₋₃ alkyl, etc., and may be monovalent (e.g., methyl), divalent (e.g., methylene), or polyvalent (e.g., methenyl). Examples of C₁₋₃ alkyl include, but are not limited to, methyl (Me), ethyl (Et), propyl (including *n*-propyl and isopropyl), and the like.

The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

The solvents used herein can be commercially available.

The following abbreviations are used in the present application: Boc represents *tert*-butyloxycarbonyl, an amine protecting group; pht represents phthaloyl, a protecting group for a primary amine; Ms represents methylsulfonyl.

### DETAILED DESCRIPTION

The present application is described in detail below by way of examples. However, this is by no means disadvantageously limiting the scope of the present application. The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein. It will be apparent to those skilled in the art that various changes and modifications can be made to the specific embodiments without departing from the spirit and scope of the present application.

### Example 1 (1_A and 1_B)

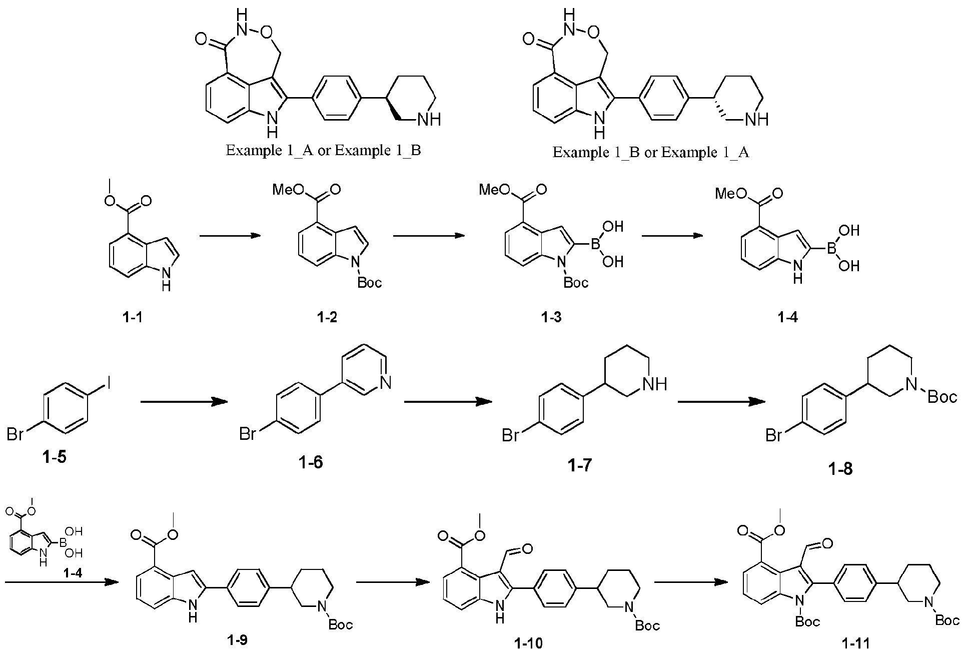

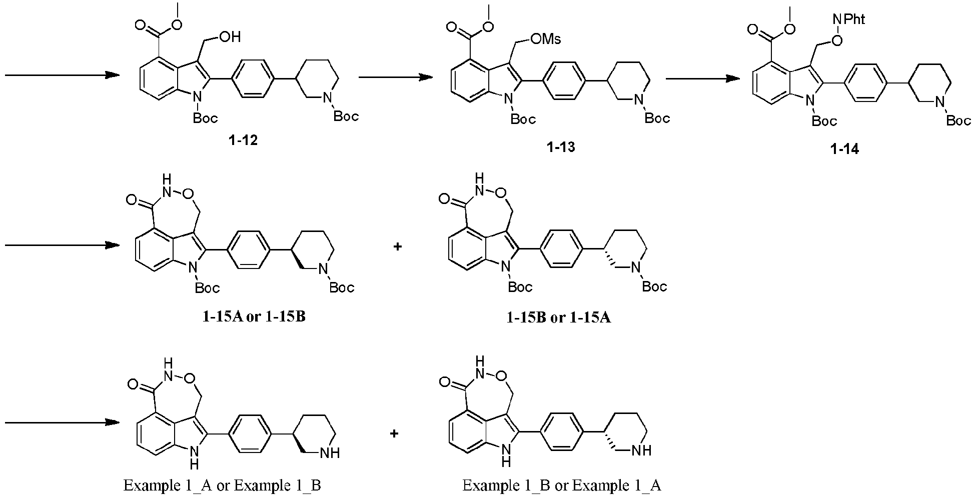

Step A: 1-1 (50 g, 285.41 mmol) was dissolved in dichloromethane (500 mL), and triethylamine (43.32 g, 428.12 mmol), 4-dimethylaminopyridine (3.49 g, 28.54 mmol), di-*tert*-butyl dicarbonate (68.52 g, 313.96 mmol) were added at 0 °C. The reaction system was stirred at 25 °C for 0.5 h. The reaction system was concentrated by rotary evaporation under reduced pressure, and then added with ethyl acetate (500 mL). The organic phase was washed with saturated aqueous ammonium chloride solution (300 mL × 3) and saturated brine (300 mL × 2), dried over anhydrous sodium sulfate, filtered and concentrated under reduced pressure to give 1-2.

Step B: Diisopropylamine (46.1 g, 456.23 mmol) was dissolved in tetrahydrofuran (250 mL), and *n*-butyllithium (2.5 M, 159.68 mL) was added dropwise to the reaction system at -78 °C under nitrogen atmosphere, and the dropwise addition was completed within half an hour. The reaction system was stirred at 0 °C for half an hour, and then added dropwise to another three-necked flask containing a solution of 1-2 (78.5 g, 285.14 mmol) and triisopropyl borate (80.44 g, 427.72 mmol) in tetrahydrofuran (750 mL) at 0 °C under nitrogen atmosphere, and the dropwise addition was completed within one and a half hours. The resulting reaction system was stirred at 0 °C for 1 h. The reaction system was then added with acetic acid solution (200 mL) to quench the reaction, diluted with water (600 mL) and extracted with ethyl acetate (500 mL × 2). The organic phases were combined, washed with saturated aqueous ammonium chloride solution (200 mL × 3) and saturated brine (200 mL × 2), dried over anhydrous sodium sulfate, filtered and concentrated under reduced pressure to give a crude product. The crude product was slurried with acetonitrile (100 mL) and aqueous solution (500 mL), and the filter cake was dried with an oil pump to give 1-3.

Step C: 1-3 was added to a solution of trifluoroacetic acid (556.25 mL) at 0 °C in three portions, and the reaction system was stirred at 0 °C for one hour under nitrogen atmosphere. The reaction system was then poured into ice water (600 mL) to precipitate a solid, and a filter cake was obtained and concentrated under reduced pressure with an oil pump to give 1-4.

Step D: 1-5 (60 g, 212.09 mmol) and 3-pyridineboronic acid (26.07 g, 212.09 mmol) were dissolved in 1,4-dioxane (600 mL) and water (120 mL), and then [1,1-bis(triphenylphosphino)ferrocene]palladium dichloride (573.14 mg, 879.39 µmol, 44.64 mL) and potassium carbonate (1.48 g, 17.59 mmol) were added. The reaction system was stirred at 90 °C for 16 h under nitrogen atmosphere. After the reaction was completed, the reaction system was filtered, and the filtrate was extracted with ethyl acetate (500 mL × 3). The organic phases were combined, dried over anhydrous sodium sulfate, filtered, concentrated under reduced pressure and purified by a silica gel column (eluent (V/V): petroleum ether/ethyl acetate =10/1 to 2/1) to give 1-6.

Step E: 1-6 (20 g, 85.44 mmol) was dissolved in methanol (200 mL), and platinum dioxide (3.88 g, 17.09 mmol) and aqueous hydrochloric acid solution (1 N, 85.44 mL) were added at room temperature, and the reaction system was stirred at 10 °C for 16 h under hydrogen atmosphere of 50 Psi. After the reaction was completed, the reaction system was filtered to give a filtrate, which was concentrated under reduced pressure to give 1-7.

Step F: 1-7 (23.63 g, 85.43 mmol) was dissolved in methanol (300 mL), and *N*,*N*-diisopropylethylamine (33.12 g, 256.29 mmol, 44.64 mL) and Boc anhydride (37.29 g, 170.86 mmol, 39.25 mL) were added at room temperature. The reaction system was stirred at room temperature for 1 h. The reaction system was then concentrated under reduced pressure to remove the solvent and extracted with ethyl acetate (300 mL × 3). The organic phases were combined, washed with saturated brine (100 mL × 1), dried over anhydrous sodium sulfate, filtered and concentrated under reduced pressure to give 1-8.

Step G: 1-8 (4 g, 8.79 mmol) and 4-methoxycarbonylindole-2-boronic acid (1.93 g, 8.79 mmol) were dissolved in ethylene glycol dimethyl ether (40 mL) and water (8 mL), and [1,1-bis(di-*tert*-butylphosphino)ferrocene]palladium dichloride (573.14 mg, 879.39 µmol, 44.64 mL) and sodium bicarbonate (1.48 g, 17.59 mmol) were added at room temperature. The reaction system was stirred at 80 °C for 16 h. After the reaction was completed, the reaction system was added with water (60 mL) and extracted with ethyl acetate (50 mL × 2). The organic phases were combined, washed with saturated brine (30 mL × 1), dried over anhydrous sodium sulfate, filtered, concentrated under reduced pressure and purified by a silica gel column (eluent (V/V): petroleum ether/ethyl acetate =10/1 to 3/1) to give 1-9.

Step H: Oxalyl chloride (1.87 g, 14.73 mmol, 1.29 mL) was dissolved in dichloromethane (20 mL) while controlling the temperature at 0 °C. *N, N*-dimethylformamide (1.61 g, 22.09 mmol, 1.7 mL) was added at 0 °C under nitrogen atmosphere. The reaction system was stirred at 0 °C for 0.25 h. 1-9 (3.2 g, 7.36 mmol) was dissolved in dichloromethane (10 mL) and added dropwise to the reaction system while controlling the temperature at 0 °C. The reaction system was reacted at 0-15 °C for 0.5 h. After the reaction was completed, the reaction system was added with an ammonium acetate solution (10%, 150 mL) and stirred at 15 °C for 1 h. The reaction system was concentrated under reduced pressure to remove the solvent, and then extracted with ethyl acetate (60 mL × 2). The organic phases were combined, washed with saturated ammonium chloride (30 mL × 2) and saturated brine (30 mL × 3), dried over anhydrous sodium sulfate, filtered and concentrated under reduced pressure to give 1-10.

Step I: 1-10 (3.5 g, 7.57 mmol) was dissolved in dichloromethane (50 mL), and Boc anhydride\di-*tert*-butyl carbonate (1.98 g, 9.08 mmol, 2.09 mL), triethylamine (1.53 g, 15.13 mmol, 2.11 mL) and 4-dimethylaminopyridine (92.44 mg, 756.70 µmol) were added with stirring while controlling the temperature at 20 °C. The reaction system was stirred at 20 °C for 1 h. After the reaction was completed, the organic phase was washed with saturated ammonium chloride solution (150 mL × 2), dried over anhydrous sodium sulfate, filtered and concentrated under reduced pressure to give 1-11.

Step J: 1-11 (4.3 g, 7.64 mmol) was dissolved in tetrahydrofuran (40 mL) and methanol (10 mL), and sodium borohydride (578.22 mg, 15.28 mmol) was added under nitrogen atmosphere while controlling the temperature at 0 °C. The reaction system was reacted at 0 °C for 0.5 h. After the reaction was completed, the reaction system was added with saturated ammonium chloride (80 mL) to quench the reaction and extracted with ethyl acetate (100 mL × 2). The organic phases were combined, washed with saturated brine (50 mL × 1), dried over anhydrous sodium sulfate, filtered and concentrated under reduced pressure to give 1-12.

Step K: 1-12 (4.0 g, 7.08 mmol) was dissolved in dichloromethane (50 mL), and triethylamine (2.15 g, 21.25 mmol, 2.96 mL) and methanesulfonyl chloride (1.62 g, 14.17 mmol) were added at 0 °C. The reaction system was stirred at 15 °C for 16 h. After the reaction was completed, the reaction system was added with dichloromethane (100 mL). The organic phase was washed with saturated ammonium chloride solution (100 mL × 2), dried over anhydrous sodium sulfate, filtered and concentrated under reduced pressure to give 1-13.

Step L: 1-13 (4.0 g, 6.22 mmol) was dissolved in *N*,*N-*dimethylformamide (50 mL), and sodium carbonate (1.32 g, 12.45 mmol) and *N*-hydroxyphthalimide (2.03 g, 12.45 mmol) were added. The reaction system was stirred at 50 °C for 16 h. After the reaction was completed, the reaction system was washed with ethyl acetate (150 mL) and saturated brine (180 mL × 4), dried over anhydrous sodium sulfate, filtered and concentrated under reduced pressure to give 1-14.

Step M: 1-14 (4.0 g, 5.64 mmol) was dissolved in methanol (40 mL), and hydrazine hydrate (1.15 g, 22.54 mmol, 1.12 mL, 98% purity) was added. The reaction system was stirred at 70 °C for 2 h under nitrogen atmosphere. After the reaction was completed, the reaction system was concentrated under reduced pressure to remove the organic solvent, and the resulting solid was purified by preparative high performance liquid chromatography (prep-HPLC) (column: Phenomenex Synergi Max-RP (250 mm×50 mm, 10 µm); mobile phase: water (0.225% formic acid)-acetonitrile; elution gradient: 60%-90%, 29 min) to give a yellow solid. The solid was then separated by chiral chromatography column (separation column: AD-H (250 mm×30 mm, 5 µm); mobile phase: 0.1% ammonia in isopropanol; elution gradient: 30%-30%, 2.1 min; 300 min) to give 1-15A (retention time = 1.704 min, ee value (enantiomeric excess): 100%) and 1-15B (retention time = 1.782 min, ee value (enantiomeric excess): 97%).

Step N: 1-15A (420 mg, 764.09 µmol) was dissolved in dichloromethane (6 mL), and trifluoroacetic acid (2 mL) was added. The reaction system was stirred at 20 °C for 1 h under nitrogen atmosphere. After the reaction was completed, the reaction system was concentrated under reduced pressure to remove the organic solvent, and the resulting solid was purified by prep-HPLC (column: Phenomenex Gemini (150 mm×25 mm, 10 µm); mobile phase: water (10 mM ammonium bicarbonate)-acetonitrile; elution gradient: 30%-51%, 7 min) to give Example 1_A (retention time = 6.63 min, ee value (enantiomeric excess): 94%). SFC (supercritical fluid chromatography) method: separation column: Chiralpak AD-3 (100 mm×4.6 mm, I.D. 3 µm); mobile phase: 40% isopropanol (0.05% diethylamine) in CO₂; flow rate: 3 mL/min; wavelength: 220 nm.
**¹HNMR (400 MHz, deuterated methanol)** δ 7.80 (dd, *J =* 0.86, 7.58 Hz, 1H), 7.62-7.70 (m, 1H), 7.48-7.54 (m, 2H), 7.39-7.46 (m, 2H), 7.31 (t*, J* = 7.83 Hz, 1H), 5.41-5.50 (m, 1H), 5.26-5.36 (m, 1H), 3.16 (br t, *J* = 12.90 Hz, 2H), 2.68-2.86 (m, 3H), 2.04 (br s, 1H), 1.83-1.94 (m, 1H), 1.68-1.80 (m, 2H).

Step O: 1-15B (440 mg, 803.45 µmοl) was dissolved in dichloromethane (6 mL), and trifluoroacetic acid (2 mL) was added. The reaction system was stirred at 20 °C for 1 h under nitrogen atmosphere. After the reaction was completed, the reaction system was concentrated under reduced pressure to remove the organic solvent, and the resulting solid was purified by prep-HPLC (column: Phenomenex Gemini (150 mm×25 mm, 10 µm); mobile phase: water (10 mM ammonium bicarbonate)-acetonitrile; elution gradient: 30%-51%, 7 min) to give Example 1_B (retention time = 5.62 min, ee value (enantiomeric excess): 94%). SFC (supercritical fluid chromatography) method: separation column: Chiralpak AD-3 (100 mm×4.6 mm, I.D. 3 µm); mobile phase: 40% isopropanol (0.05% diethylamine) in CO₂; flow rate: 3 mL/min; wavelength: 220 nm.
**¹HNMR (400 MHz, deuterated methanol)** δ 7.80 (dd, *J* = 0.86, 7.58 Hz, 1H), 7.64-7.70 (m, 1H), 7.48-7.53 (m, 2H), 7.41-7.46 (m, 2H), 7.31 (t, *J* = 7.83 Hz, 1H), 5.41-5.50 (m, 1H), 5.27-5.35 (m, 1H), 3.13-3.19 (m, 2H), 2.71-2.84 (m, 3H), 2.04 (br s, 1H), 1.84-1.91 (m, 1H), 1.71-1.79 (m, 2H)

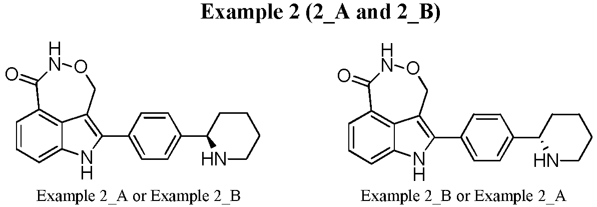

Reference was made to Example 1 for synthesis method.

For Example 2, prior to deprotection of Boc, the compound was separated by chiral HPLC column (separation column: WHELK-O1 (250 mm×50 mm, 10 µm); mobile phase: 0.1% ammonia in methanol; elution gradient: 40%-40%, 4 min; 260 min) to give two isomers with different configurations: Example 2_AA (retention time = 4.453 min, ee value (enantiomeric excess): 100%) and Example 2_BB (retention time = 4.735 min, ee value (enantiomeric excess): 98%), which were deprotected with trifluoroacetic acid to give Example 2_A (retention time = 1.276 min, ee value (enantiomeric excess): 100%) and Example 2_B (retention time = 1.632 min, ee value (enantiomeric excess): 98%), respectively.

SFC (supercritical fluid chromatography) method: separation column: Chiralcel OJ-3 (50 mm×4.6 mm, I.D. 3 µm); mobile phase: 40% isopropanol (0.05% diethylamine) in CO₂; flow rate: 3 mL/min; wavelength: 220 nm.

**Example 2_A**: **¹HNMR (400 MHz, deuterated methanol)** δ 8.52 (s, 1H), 7.82 (d, *J* = 7.21 Hz, 1H), 7.60-7.72 (m, 5H), 7.34 (t*, J* = 7.76 Hz, 1H), 5.43-5.52 (m, 1H), 5.25-5.35 (m, 1H), 4.32 (br d, *J* = 11.86 Hz, 1H), 3.51 (br d, *J=* 12.72 Hz, 1H), 3.16-3.29 (m, 1H), 1.95-2.21 (m, 4H), 1.73-1.93 (m, 2H).

**Example 2_B: ¹HNMR (400 MHz, deuterated methanol)** δ 8.55 (s, 1H), 7.82 (d, *J* = 7.34 Hz, 1H), 7.59-7.72 (m, 5H), 7.34 (t*, J* = 7.83 Hz, 1H), 5.43-5.56 (m, 1H), 5.24-5.36 (m, 1H), 4.28 (br d, *J* = 11.86 Hz, 1H), 3.49 (br d, *J=* 11.98 Hz, 1H), 3.11-3.26 (m, 1H), 1.92-2.21 (m, 4H), 1.71-1.90 (m, 2H).

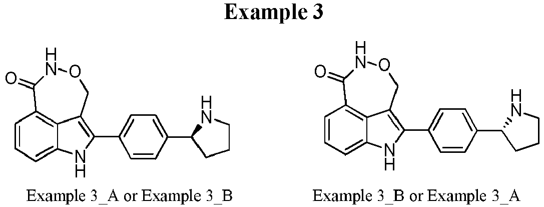

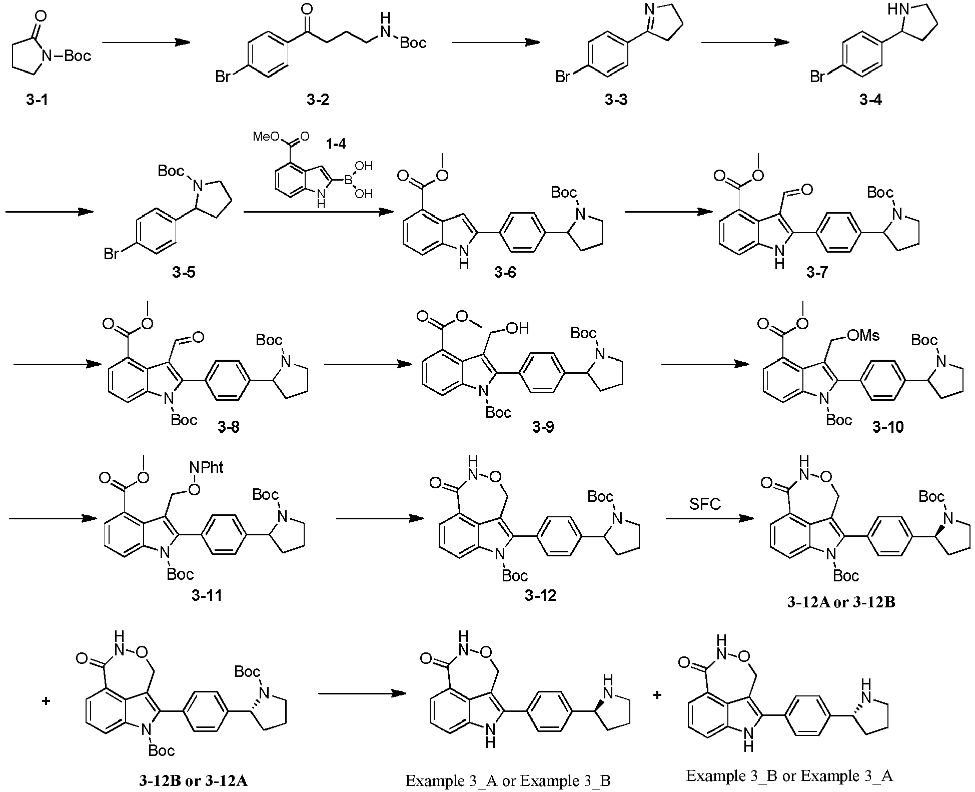

Step A: 1,4-dibromobenzene (8.92 g, 37.79 mmol) was dissolved in tetrahydrofuran (35.00 mL) and then added dropwise to a three-necked flask containing magnesium chips (918.56 mg, 37.79 mmol) and iodine (137.03 mg, 539.9 µmol) at 70 °C under nitrogen atmosphere, and the dropwise addition was completed within half an hour. The reaction system was stirred at 70 °C for 1 h and then cooled to 20 °C. The reaction system was added dropwise into another three-necked flask containing a solution of 3-1 (5 g, 26.99 mmol) in tetrahydrofuran (15 mL) at -70 °C under nitrogen atmosphere, and the dropwise addition was completed within half an hour. The resulting reaction system was stirred at -70 °C for 2 h, and then heated to 15 °C and stirred for 1 h. The reaction system was added with saturated aqueous ammonium chloride solution (60 mL) to quench the reaction and extracted with ethyl acetate (50 mL × 3). The organic phases were combined, washed with saturated brine (50 mL), dried over anhydrous sodium sulfate, filtered and concentrated under reduced pressure. The residue was purified by a silica gel column (eluent (V/V): petroleum ether/ethyl acetate =30/1 to 10/1) to give 3-2.

Step B: 3-2 (5 g, 14.61 mmol) was added to trifluoroacetic acid (25 mL). The reaction system was stirred at 25 °C for 4 h. The reaction system was then concentrated by rotary evaporation under reduced pressure and added with water (40 mL). The mixture was adjusted to pH = 14 with 40% aqueous sodium hydroxide solution and a white solid was precipitated. The resulting mixture was filtered, and the filter cake was washed with a small amount of water and subjected to rotary evaporation to give 3-3.

Step C: 3-3 (2.8 g, 4.97 mmol) was dissolved in methanol (30 mL) and water (7 mL). The reaction system was cooled to -41 °C and added with sodium borohydride (945.34 mg, 24.99 mmol). The reaction system was stirred at -41 °C for 4 h. Then the reaction system was added with 2 mol/L hydrochloric acid (50 mL) at 0 °C to quench the reaction, and added with ethyl acetate (100 mL). The organic phase was washed with water (50 mL), followed by liquid separation. The aqueous phase was adjusted to pH =14 with 4 mol/L sodium hydroxide and extracted with ethyl acetate (150 mL × 3). The organic phases were combined, washed with water (50 mL × 2), dried over anhydrous sodium sulfate, filtered and concentrated under reduced pressure to give 3-4.

Step D: 3-4 (3 g, 13.15 mmol) was dissolved in dichloromethane (30 mL), and triethylamine (3.99 g, 39.45 mmol) and di-*tert*-butyl dicarbonate (3.16 g, 14.47 mmol) were added. The reaction system was stirred at 25 °C for 1 h. The reaction system was concentrated by rotary evaporation under reduced pressure, and then added with ethyl acetate (60 mL). The organic phase was washed with saturated aqueous ammonium chloride solution (30 mL × 3) and saturated brine (50 mL), dried over anhydrous sodium sulfate, filtered and concentrated under reduced pressure to give 3-5.

Referring to the method in Example 1, Compound 3-5 was separated by chiral HPLC (separation column: AD-H (250 mm×30 mm, 5 µm); mobile phase: 0.1% ammonia in isopropanol; elution gradient: 30%-30%, 2.1 min; 85 min) to 3-12A (retention time = 1.627 min, ee value (enantiomeric excess): 100%) and 3-12B (retention time = 1.711 min, ee value (enantiomeric excess): 98%), which were deprotected to give Example 3_A (retention time = 0.732 min, ee value (enantiomeric excess): 100%) and Example 3_B (retention time = 1.402 min, ee value (enantiomeric excess): 97.32%), respectively.

Method for measuring ee value (enantiomeric excess): separation column: Chiralcel OJ-3 (50 mm×4.6 mm, I.D. 3 µm); mobile phase: 40% ethanol (0.05% diethylamine) in CO₂; flow rate: 3 mL/min; wavelength: 220 nm.

**Example 3_A: ¹H NMR (400 MHz, DMSO-*d*₆**) δ ppm 1.76-2.00 (m, 3H) 2.23-2.34 (m, 1H) 3.07-3.15 (m, 1H) 3.21 (dt, *J*=10.18, 7.26 Hz, 1H) 4.36 (br t, *J*=8.01 Hz, 1H) 5.17-5.27 (m, 1H) 5.36-5.49 (m, 1H) 7.29 (t, *J =* 7.76 Hz, 1H) 7.58 (d, *J*=0.86 Hz, 4H) 7.64-7.71 (m, 2H) 8.33 (s, 1H) 11.06 (br s, 1H) 11.85 (s, 1H).

**Example 3_B: ¹H NMR (400 MHz, DMSO-*d*₆**) δ ppm 1.73-1.97 (m, 3H) 2.19-2.35 (m, 1H) 3.04-3.24 (m, 2H) 4.33 (br t, J=8.07 Hz, 1H) 5.14-5.26 (m, 1H) 5.36-5.50 (m, 1H) 7.29 (t, J = 7.76 Hz, 1H) 7.58 (d, J = 0.98 Hz, 4H) 7.63-7.71 (m, 2H) 8.31 (s, 1H) 11.06 (br s, 1H) 11.83 (s, 1H).

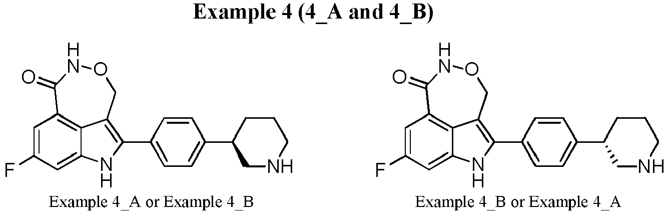

Reference was made to Example 1 for synthesis method.

The Example 4 was separated by chiral HPLC column (separation column: IC (250 mm×30 mm, 10 µm); mobile phase: 0.1% ammonia in ethanol; elution gradient: 55%-55%, 3.6 min; 80 min) to give Example 4_A (retention time = 2.353 min, ee value (enantiomeric excess): 100 %) and Example 4_B (retention time = 3.177 min, ee value (enantiomeric excess): 100%).

SFC (supercritical fluid chromatography) method: separation column: Chiralpak IC-3 (50 mm×4.6 mm, I.D. 3 µm); mobile phase: 40% isopropanol (0.05% diethylamine) in CO₂; flow rate: 3 mL/min; wavelength: 220 nm.

**Example 4_A: ¹HNMR (400 MHz, deuterated methanol)** δ 7.46-7.59 (m, 5H), 7.38 (br d, *J* = 7.70 Hz, 1H), 5.38-5.50 (m, 1H), 5.23-5.34 (m, 1H), 3.49 (br d*, J* = 11.00 Hz, 2H), 3.02-3.24 (m, 3H), 2.12 (br d*, J* = 10.39 Hz, 2H), 1.79-2.02 (m, 2H).

**Example 4_B: ¹HNMR (400 MHz, deuterated methanol)** δ 7.30-7.55 (m, 6H), 5.38-5.48 (m, 1H), 5.24-5.34 (m, 1H), 3.11 (br t, *J* = 13.82 Hz, 2H), 2.57-2.89 (m, 3H), 2.03 (br d, *J* = 8.80 Hz, 1H), 1.85 (br d, *J* = 10.27 Hz, 1H), 1.62-1.78 (m, 2H)

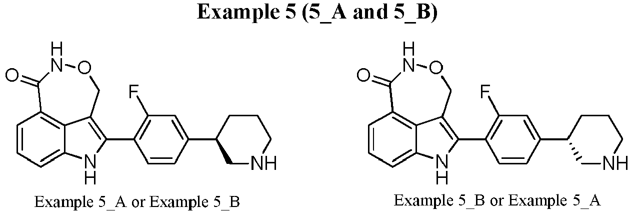

Reference was made to Example 1 for synthesis method.

For Example 5, prior to deprotection of Boc, the compound was separated by chiral HPLC column (separation column: AD-H (250 mm×30 mm, 5 µm); mobile phase: 0.1% ammonia in isopropanol; elution gradient: 30%-30%, 6.0 min; 350 min) to give two isomers with different configurations: 5_AA (retention time = 1.669 min, ee value (enantiomeric excess): 98%) and 5_BB (retention time = 1.725 min, ee value (enantiomeric excess): 97%), which were deprotected with trifluoroacetic acid to give Example 5_A (retention time = 4.468 min, ee value (enantiomeric excess): 97%) and Example 5_B (retention time = 3.784 min, ee value (enantiomeric excess): 94%), respectively. SFC (supercritical fluid chromatography) method: separation column: Chiralpak IC-3 (50 mm×4.6 mm, I.D. 3 µm); mobile phase: 40% ethanol (0.05% diethylamine) in CO₂; flow rate: 3 mL/min; wavelength: 220 nm.

**Example 5_A: ¹HNMR (400 MHz, deuterated methanol)** δ 8.54 (s, 1H), 7.82 (d, *J* = 7.50 Hz, 1H), 7.69 (d, *J* = 7.63 Hz, 1H), 7.47-7.59 (m, 1H), 7.22-7.39 (m, 3H), 5.26-5.38 (m, 1H), 5.05-5.17 (m, 1H), 3.41-3.57 (m, 2H), 3.00-3.22 (m, 3H), 2.10 (br d*, J* = 13.51 Hz, 2H), 1.77-2.01 (m, 2H).

**Example 5_B: ¹ HNMR (400 MHz, deuterated methanol)** δ 8.54 (s, 1H), 7.78-7.88 (m, 1H), 7.65-7.74 (m, 1H), 7.51-7.60 (m, 1H), 7.24-7.40 (m, 3H), 5.27-5.40 (m, 1H), 5.05-5.20 (m, 1H), 3.42-3.55 (m, 2H), 2.99-3.23 (m, 3H), 2.05-2.17 (m, 2H), 1.79-2.01 (m, 2H)

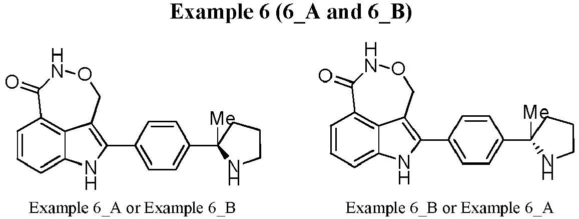

Reference was made to Example 3 for synthesis method.

For Example 6, prior to deprotection of Boc, the compound was separated by chiral HPLC column (separation column: WHELK-O1 (250 mm×50 mm, 10 µm); mobile phase: 0.1% ammonia in methanol; elution gradient: 40%-40%, 3.7 min; 450 min) to give two isomers with different configurations: Example 6_AA (retention time = 2.483 min, ee value (enantiomeric excess): 100%) and Example 6_BB (retention time = 2.691 min, ee value (enantiomeric excess): 94%), which were deprotected with trifluoroacetic acid to give Example 6_A (retention time = 1.955 min, ee value (enantiomeric excess): 100%) and Example 6_B (retention time = 3.339 min, ee value (enantiomeric excess): 94%), respectively.

SFC (supercritical fluid chromatography) method: separation column: OJ-3 (50 mm×4.6 mm, I.D. 3 µm); mobile phase: 30% isopropanol (0.05% diethylamine) in CO₂; flow rate: 3 mL/min; wavelength: 220 nm.

**Example 6_A: ¹H NMR (400 MHz, deuterated dimethyl sulfoxide)** δ ppm 1.48 (s, 3H) 1.81 (br s, 1H) 1.93-2.17 (m, 3H) 2.94-3.35 (m, 2H) 5.23 (br dd, *J* = 14.67, 3.55 Hz, 1H) 5.38-5.48 (m, 1H) 7.29 (t, *J* = 7.76 Hz, 1H) 7.54-7.61 (m, 2H) 7.62-7.73 (m, 4H) 8.18-8.33 (m, 1H) 11.07 (s, 1H) 11.80 (br s, 1H)

**Example 6_B: ¹H NMR (400 MHz, deuterated dimethyl sulfoxide)** δ ppm 1.48 (br d, *J* = 2.69 Hz, 3H) 1.79 (br s, 1H) 1.95-2.18 (m, 3H) 2.93-3.30 (m, 2H) 5.23 (br dd, *J =* 14.61, 3.12 Hz, 1H) 5.43 (br d, *J* = 14.79 Hz, 1H) 7.19-7.35 (m, 1H) 7.53-7.60 (m, 2H) 7.61-7.77 (m, 4H) 8.19-8.35 (m, 1H) 11.06 (s, 1H) 11.80 (br d, *J =* 4.65 Hz, 1H)

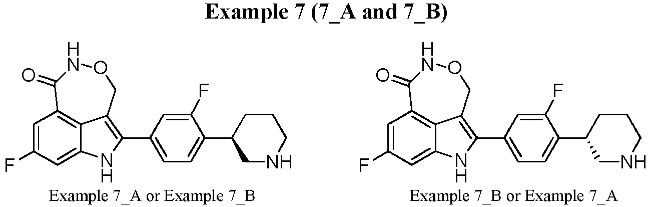

Reference was made to Example 1 for synthesis method.

The Example 7 was separated by chiral HPLC column (separation column: IC (250 mm×30 mm, 10 µm); mobile phase: 0.1% ammonia in ethanol; elution gradient: 50%-50%, 4.3 min; 120 min) to give two isomers of different configurations: Example 7_A (retention time = 1.889 min, ee value (enantiomeric excess): 100 %) and Example 7_B (retention time = 2.411 min, ee value (enantiomeric excess): 94%).

SFC (supercritical fluid chromatography) method: separation column: IC-3 (50 mm×4.6 mm, I.D. 3 µm); mobile phase: 40% isopropanol (0.05% diethylamine) in CO₂; flow rate: 3 mL/min; wavelength: 220 nm.

**Example 7_A**: **¹HNMR (400 MHz, deuterated methanol)** δ 7.49-7.64 (m, 2H), 7.26-7.45 (m, 3H), 5.40-5.48 (m, 1H), 5.25-5.34 (m, 1H), 3.37-3.56 (m, 3H), 2.98-3.24 (m, 2H), 2.04-2.18 (m, 2H), 1.81-2.01 (m, 2H).

**Example 7_B**: **¹HNMR (400 MHz, deuterated methanol)** δ 7.43-7.57 (m, 2H), 7.38 (dd, *J* = 2.14, 8.99 Hz, 1H), 7.23-7.32 (m, 2H), 5.39-5.47 (m, 1H), 5.26-5.35 (m, 1H), 3.02-3.21 (m, 3H), 2.60-2.82 (m, 2H), 1.95-2.08 (m, 1H), 1.64-1.89 (m, 3H).

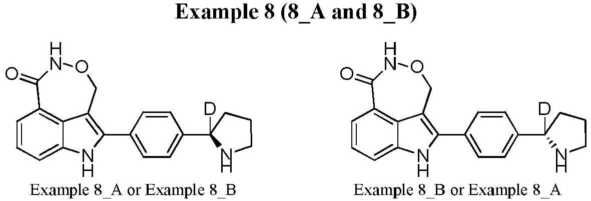

Reference was made to Example 1 for synthesis method.

For Example 8, prior to deprotection of Boc, the compound was separated by chiral HPLC column (separation column: AD-H (250 mm×30 mm, 5 µm); mobile phase: 0.1% ammonia in isopropanol; elution gradient: 30%-30%, 5.0 min; 110 min) to give two isomers with different configurations: 8_AA (retention time = 1.570 min, ee value (enantiomeric excess): 100%) and 8_BB (retention time = 1.674 min, ee value (enantiomeric excess): 100%), which were deprotected with trifluoroacetic acid to give Example 8_A (retention time = 1.262 min, ee value (enantiomeric excess): 100%) and Example 8_B (retention time = 2.511 min, ee value (enantiomeric excess): 100%), respectively.

SFC (supercritical fluid chromatography) method: separation column: OJ-3 (50 mm×4.6 mm, I.D. 3 µm); mobile phase: 30% methanol (0.05% diethylamine) in CO₂; flow rate: 3 mL/min; wavelength: 220 nm.

**Example 8_A: ¹H NMR (400 MHz, DMSO-*d*₆)** δ ppm 1.71-1.80 (m, 1H) 1.82-1.99 (m, 2H) 2.22-2.31 (m, 1H) 3.05-3.28 (m, 2H) 5.22 (br d, *J*=14.55 Hz, 1H) 5.43 (br d, *J*=14.55 Hz, 1H) 7.29 (t*, J* = 7.76 Hz, 1H) 7.53-7.71 (m, 6H) 8.30 (s, 1H) 11.06 (br s, 1H) 11.82 (s, 1H)

**Example 8_B: ¹H NMR (400 MHz, DMSO-*d*₆)** δ ppm 1.70-1.79 (m, 1H) 1.86-1.97 (m, 2H) 2.21-2.35 (m, 1H) 3.02-3.23 (m, 2H) 5.16-5.27 (m, 1H) 5.37-5.48 (m, 1H) 7.29 (t, *J* = 7.76 Hz, 1H) 7.52-7.72 (m, 6H) 8.29 (s, 1H) 11.06 (br s, 1H) 11.81 (s, 1H)

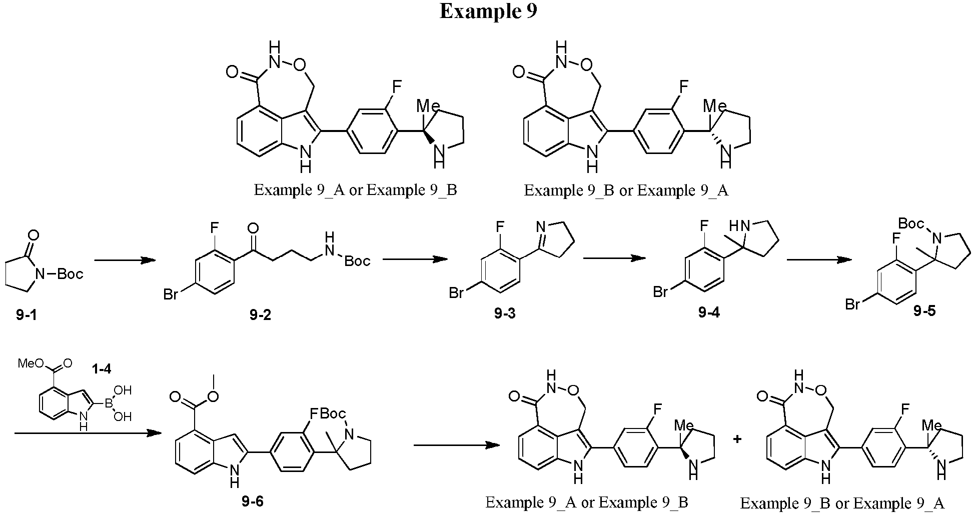

After obtaining Compound 9-5 by referring to the preparation method of Example 3, 9-5 was reacted with 1-4, referring to Example 1, to give 9-6, which was separated by chiral HPLC column (separation column: AD (250 mm×30 mm, 10 µm); mobile phase: 0.1% ammonia in methanol; elution gradient: 60%-60%, 6.6 min; 190 min) to give two isomers with different configurations: 9-6_AA (retention time = 0.625 min, ee value (enantiomeric excess): 100%) and 9-6_BB (retention time = 1.657 min, ee value (enantiomeric excess): 100%), which were subjected to similar procedure in Example 1 to give Example 9_A (retention time = 0.716 min, ee value (enantiomeric excess): 100%) and Example 9_B (retention time = 0.559 min, ee value (enantiomeric excess): 100%), respectively.

SFC (supercritical fluid chromatography) method: separation column: AD-3 (50 mm×4.6 mm, I.D. 3 µm); mobile phase: 40% methanol (0.05% diethylamine) in CO₂; flow rate: 3 mL/min; wavelength: 220 nm.

**Example 9_A: ¹HNMR (400 MHz, deuterated methanol)** δ 8.51 (s, 1H), 7.83 (d, J = 7.46 Hz, 1H), 7.69 (d, J = 8.07 Hz, 1H), 7.61 (t, J = 8.25 Hz, 1H), 7.41-7.51 (m, 2H), 7.37 (t, J = 7.83 Hz, 1H), 5.44-5.53 (m, 1H), 5.27-5.38 (m, 1H), 3.52-3.62 (m, 1H), 3.40-3.50 (m, 1H), 2.43-2.62 (m, 2H), 2.18-2.41 (m, 2H), 1.72 (s, 3H).

**Example 9_B: ¹HNMR (400 MHz, deuterated methanol)** δ 8.49 (s, 1H), 7.83 (d, J = 7.46 Hz, 1H), 7.69 (d, J = 8.07 Hz, 1H), 7.61 (t, J = 8.19 Hz, 1H), 7.41-7.52 (m, 2H), 7.36 (t, J = 7.82 Hz, 1H), 5.42-5.58 (m, 1H), 5.25-5.38 (m, 1H), 3.53-3.63 (m, 1H), 3.41-3.52 (m, 1H), 2.43-2.64 (m, 2H), 2.18-2.42 (m, 2H), 1.73 (s, 3H).

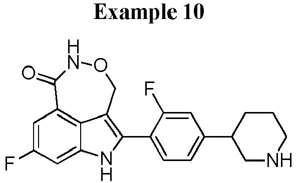

Reference was made to Example 1 for synthesis method; however, the racemate Example 10 was obtained directly without chiral resolution.

**Example 10: ¹H NMR (400 MHz, DMSO-*d*₆)** δ ppm 1.57-1.82 (m, 3H) 1.93 (br s, 1H) 2.68 (br s, 1H) 2.75-2.91 (m, 2H) 2.99-3.23 (m, 2H) 5.03-5.13 (m, 1H) 5.14-5.28 (m, 1H) 7.24-7.39 (m, 2H) 7.41-7.57 (m, 3H) 8.34 (br s, 1H) 11.27 (br s, 1H) 11.87 (br s, 1H).

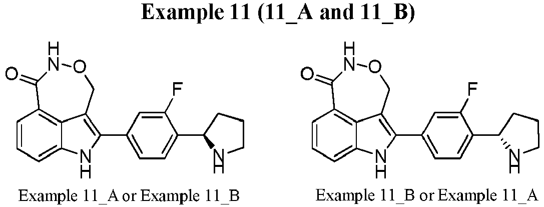

Reference was made to Example 1 for synthesis method.

After deprotection with trifluoroacetic acid, Example 11_A (retention time = 2.020 min, ee value (enantiomeric excess): 96.33%) and Example 11_B (retention time = 1.402 min, ee value (enantiomeric excess): 97.32%) were obtained.

Method for measuring ee value (enantiomeric excess): separation column: OJ-3 (50 mm×4.6 mm, I.D. 3 µm); mobile phase: 5%-40% ethanol (0.05% diethylamine) in CO₂; flow rate: 3 mL/min; wavelength: 220 nm.

**Example 11_A: ¹H NMR (400 MHz, DMSO-*d*₆)** δ ppm 1.74-2.00 (m, 3H) 2.23-2.36 (m, 1H) 3.07-3.22 (m, 2 H) 4.57 (br t, *J* = 7.95 Hz, 1H) 5.17-5.32 (m, 1H) 5.38-5.52 (m, 1H) 7.28-7.52 (m, 3H) 7.63-7.76 (m, 3H) 8.25 (s, 1H) 11.09 (br s, 1H) 11.90 (s, 1H).

**Example 11_B: ¹H NMR (400 MHz, DMSO-*d*₆)** δ ppm 1.68-2.05 (m, 3H) 2.19-2.37 (m, 1H) 3.05-3.22 (m, 2H) 4.42-4.60 (m, 1H) 5.17-5.32 (m, 1H) 5.38-5.51 (m, 1H) 7.29-7.52 (m, 3H) 7.56-7.75 (m, 3H) 8.29 (s, 1H) 11.09 (br s, 1H) 11.92 (s, 1H).

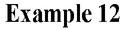

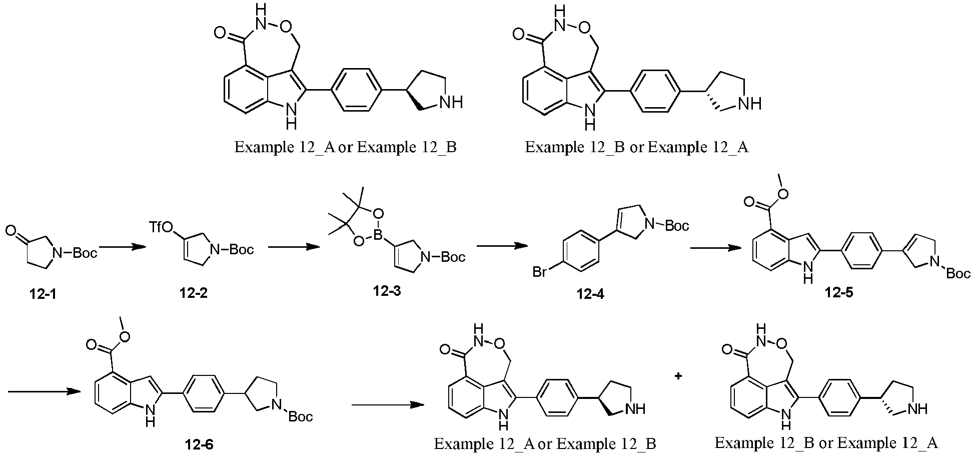

Step A: 12-1 (5.0 g, 26.99 mmol) was dissolved in tetrahydrofuran (50 mL), and lithium hexamethyldisilazide (29.69 mmol, 1 mol/L) was added with stirring while controlling the temperature at -78 °C. The reaction system was stirred at -78 °C for 0.5 h. N-phenylbis(trifluoromethanesulfonyl)imide (11.57 g, 32.39 mmol) was then dissolved in tetrahydrofuran (100 mL) and slowly added dropwise to the reaction system. The resulting reaction system was stirred at -78 °C to 0 °C for 2 h. After the reaction was completed, the reaction system was added with saturated sodium bicarbonate (500 mL) to quench the reaction and extracted with ethyl acetate (200 mL × 3). The organic phases were combined, washed with saturated brine (200 mL × 2), dried over anhydrous sodium sulfate, filtered and concentrated under reduced pressure to give 12-2.

Step B: 12-2 (6.0 g, 18.91 mmol) was dissolved in dioxane (60 mL) and bis(pinacolato)diboron (4.8 g, 18.91 mmol), potassium acetate (3.71 g, 37.82 mmol) and [1,1-bis(triphenylphosphino)ferrocene]palladium dichloride dichloromethane complex (1.54 g, 1.89 mmol) were added at room temperature. The reaction system was stirred at 80 °C for 16 h. After the reaction was completed, no further treatment was performed on the reaction system and 12-3 was obtained, which was directly used in the next step.

Step C: 12-3 (1.2 g, 3.70 mmol) and 1-bromo-4-iodobenzene (4.79 g, 16.94 mmol) were dissolved in dioxane (60 mL) and water (12 mL), and [1,1-bis(triphenylphosphino)ferrocene]palladium dichloride dichloromethane complex (1.38 g, 1.69 mmol) and potassium carbonate (4.68 g, 33.88 mmol) were added. The reaction system was stirred at 80 °C for 5 h. After the reaction was completed, the reaction system was added with water (200 mL) and extracted with ethyl acetate (100 mL × 3). The organic phases were combined, washed with saturated brine (100 mL × 1), dried over anhydrous sodium sulfate, filtered, concentrated under reduced pressure and purified by a silica gel column (eluent (V/V): petroleum ether/ethyl acetate (volume ratio) = 50:1 to 10:1) to give 12-4.

Step D: 12-4 (1.2 g, 3.70 mmol) and 4-methoxycarbonylindole-2-boronic acid (810.59 mg, 3.70 mmol) were dissolved in ethylene glycol dimethyl ether (15 mL) and water (3 mL), and [1,1-bis(di-*tert*-butylphosphino)ferrocene]palladium dichloride (241.23 mg, 370.13 µmοl) and sodium bicarbonate (621.89 mg, 7.40 mmol) were added. The reaction system was stirred at 80 °C for 16 h. After the reaction was completed, the reaction system was added with water (100 mL) and extracted with ethyl acetate (100 mL × 2). The organic phases were combined, washed with saturated brine (100 mL × 1), dried over anhydrous sodium sulfate, filtered, concentrated under reduced pressure and purified by a silica gel column (eluent (V/V): petroleum ether/ethyl acetate (volume ratio) = 10:1 to 2:1) to give 12-5.

Step E: 12-5 (1.1 g, 32.43 mmol) was dissolved in methanol (10 mL), and palladium on carbon (100 mg, 10% purity) was added at room temperature. The reaction system was stirred at 25 °C for 16 h under hydrogen atmosphere of 15 Psi. After the reaction was completed, the reaction system was filtered and concentrated under reduced pressure to give 12-6.

For Compound 12-6, reference was made to the method of Example 1 to give the compound before deprotection of Boc, which was then separated by chiral HPLC column (separation column: AD-H (250 mm×30 mm, 5 µm); mobile phase: 0.1% ammonia in ethanol; elution gradient: 35%-35%, 2.3 min; 50 min) to give two isomers with different configurations: 12_AA (retention time = 2.771 min, ee value (enantiomeric excess): 98%) and 12_BB (retention time = 2.875 min, ee value (enantiomeric excess): 98%), which were deprotected with trifluoroacetic acid to give Example 12_A (retention time = 4.564 min, ee value (enantiomeric excess): 100%) and Example 12_B (retention time = 4.148 min, ee value (enantiomeric excess): 100%), respectively.

SFC (supercritical fluid chromatography) method: separation column: AS-3 (100 mm×4.6 mm, I.D. 3 µm); mobile phase: 30%-45% isopropanol (0.05% isopropylamine) in CO₂; flow rate: 3 mL/min; wavelength: 220 nm.

**Example 12_A: ¹HNMR (400 MHz, deuterated methanol)** δ 8.56 (s, 1H), 7.81 (d, *J* = 7.09 Hz, 1H), 7.67 (d, *J* = 7.95 Hz, 1H), 7.48-7.59 (m, 4H), 7.32 (t, *J* = 7.82 Hz, 1H), 5.42-5.52 (m, 1H), 5.25-5.35 (m, 1H), 3.77 (br s, 1H), 3.60 (br s, 2H), 3.42 (br d, *J* = 7.58 Hz, 1H), 3.28 (br d, *J* = 9.66 Hz, 1H), 2.52 (br s, 1H), 2.08-2.30 (m, 1H).

**Example 12_B: ¹HNMR (400 MHz, deuterated methanol)** δ 8.55 (br s, 1H), 7.78-7.85 (m, 1H), 7.67 (d, *J* = 7.58 Hz, 1H), 7.49-7.60 (m, 4H), 7.33 (t*, J* = 7.83 Hz, 1H), 5.41-5.51 (m, 1H), 5.25-5.36 (m, 1H), 3.72-3.83 (m, 1H), 3.58-3.65 (m, 2H), 3.38-3.52 (m, 1H), 3.27 (br s, 1H), 2.53 (br d, *J* = 4.16 Hz, 1H), 2.11-2.26 (m, 1H).

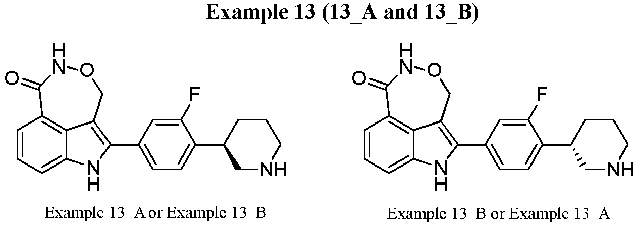

Reference was made to Example 1 for synthesis method.

For Example 13, Prior to deprotection of Boc, the compound was separated by chiral HPLC column (separation column: AD-H (250 mm×30 mm, 5 µm); mobile phase: 0.1% ammonia in isopropanol; elution gradient: 25%-25%, 2.0 min; 500 min) to give two isomers with different configurations: 13_AA (retention time = 3.090 min, ee value (enantiomeric excess): 85 %) and 13_BB (retention time = 3.357 min, ee value (enantiomeric excess): 86%), which were deprotected with trifluoroacetic acid to give Example 13_A (retention time = 1.477 min, ee value (enantiomeric excess): 93 %) and Example 13_B (retention time = 1.162 min, ee value (enantiomeric excess): 50%), respectively.

SFC (supercritical fluid chromatography) method: separation column: Chiralpak AD-3 (50 mm×4.6 mm, I.D. 3 µm); mobile phase: 40% isopropanol (0.05% diethylamine) in CO₂; flow rate: 3 mL/min; wavelength: 220 nm.

**Example 13_A**: **HNMR (400 MHz, deuterated methanol)** δ 7.69 (d, *J* = 7.46 Hz, 1H), 7.55 (d, *J* = 7.95 Hz, 1H), 7.32-7.38 (m, 1H), 7.15-7.22 (m, 3H), 5.30-5.38 (m, 1H), 5.16-5.25 (m, 1H), 3.02 (br d, *J* = 10.03 Hz, 2H), 2.48-2.70 (m, 3H), 1.88 (br d, *J=* 12.10 Hz, 1H), 1.66-1.78 (m, 3H).

**Example 13_B: HNMR (400 MHz, deuterated methanol)** δ 7.69 (d, *J* = 6.85 Hz, 1H), 7.57 (d, *J* = 7.95 Hz, 1H), 7.36-7.43 (m, 1H), 7.18-7.27 (m, 3H), 5.30-5.38 (m, 1H), 5.16-5.24 (m, 1H), 3.15 (br t, *J* = 11.92 Hz, 3H), 2.68-2.88 (m, 2H), 1.83-1.96 (m, 2H), 1.69-1.78 (m, 2H).

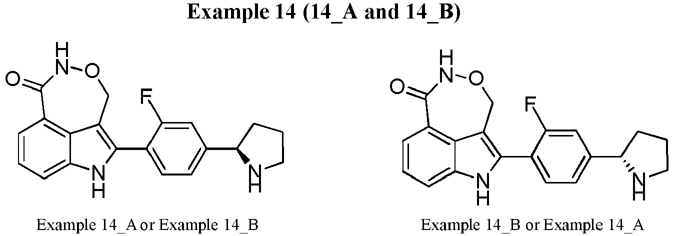

Reference was made to Example 3 for synthesis method.

For Example 14, prior to deprotection of Boc, the compound was separated by chiral HPLC column (separation column: Whelk-O1 (250 mm×50 mm, 10 µm); mobile phase: 0.1% ammonia in methanol; elution gradient: 40%-40%, 3.6 min; 150 min) to give two isomers with different configurations: 14_AA (retention time = 4.092 min, ee value (enantiomeric excess): 100%) and 14_BB (retention time = 4.411 min, ee value (enantiomeric excess): 98%), which were deprotected with trifluoroacetic acid to give Example 14_A (retention time = 1.489 min, ee value (enantiomeric excess): 100%) and Example 14_B (retention time = 2.700 min, ee value (enantiomeric excess): 91%), respectively.

SFC (supercritical fluid chromatography) method: separation column: Chiralpak AD-3 (50 mm×4.6 mm, I.D. 3 µm); mobile phase: 40% isopropanol (0.05% diethylamine) in CO₂; flow rate: 3 mL/min; wavelength: 220 nm.

**Example 14_A: HNMR (400 MHz, deuterated dimethyl sulfoxide)** δ = 11.82 (s, 1H), 11.25-10.87 (m, 1H), 8.30 (s, 1H), 7.74-7.65 (m, 2H), 7.60-7.54 (m, 1H), 7.52-7.45 (m, 1H), 7.44-7.39 (m, 1H), 7.36-7.28 (m, 1H), 5.30-5.17 (m, 1H), 5.13-4.99 (m, 1H), 4.37 (br t, *J=* 7.9 Hz, 1H), 3.21-3.07 (m, 2H), 2.34-2.25 (m, 1H), 1.97-1.72 (m, 3H)

Example 14 B: HNMR (400 MHz, deuterated dimethyl sulfoxide) δ = 11.80-11.74 (m, 1H), 11.15-11.03 (m, 1H), 8.26 (s, 1H), 7.73-7.65 (m, 2H), 7.58-7.52 (m, 1H), 7.48-7.38 (m, 2H), 7.31 (s, 1H), 5.28-5.18 (m, 1H), 5.11-5.02 (m, 1H), 4.32-4.27 (m, 1H), 3.15-3.10 (m, 1H), 3.08-3.03 (m, 1H), 2.30-2.22 (m, 1H), 1.92-1.80 (m, 2H), 1.72-1.62 (m, 1H)

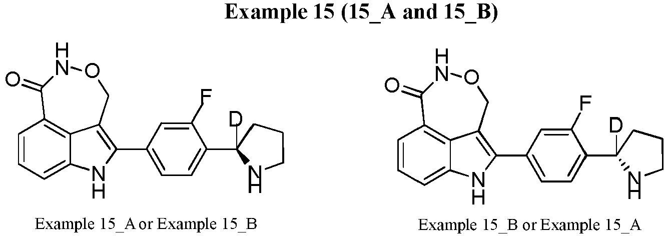

Reference was made to Example 8 for synthesis method.

For Example 15, prior to deprotection of Boc, the compound was separated by chiral HPLC column (separation column: AD-H (250 mm×30 mm, 5 µm); mobile phase: 0.1% ammonia in isopropanol; elution gradient: 20%-20%, 3.1 min; 250 min) to give two isomers with different configurations: 15_AA (retention time = 3.191 min, ee value (enantiomeric excess): 100 %) and 15_BB (retention time = 3.511 min, ee value (enantiomeric excess): 97%), which were deprotected with trifluoroacetic acid to give Example 15_A (retention time = 2.28 min, ee value (enantiomeric excess): 100 %) and Example 15_B (retention time = 2.101 min, ee value (enantiomeric excess): 87%), respectively.

SFC (supercritical fluid chromatography) method: separation column: Chiralpak OD-3 (50 mm×4.6 mm, I.D. 3 µm); mobile phase: 5%-40% ethanol (0.05% diethylamine) in CO₂; flow rate: 3 mL/min; wavelength: 220 nm.

**Example 15_A: HNMR (400 MHz, deuterated dimethyl sulfoxide)** δ ppm 1.64 (br s, 1H) 1.75-1.89 (m, 2H) 2.17-2.30 (m, 1H) 3.07 (br d, *J* = 17.69 Hz, 2H) 5.14-5.32 (m, 1H) 5.37-5.52 (m, 1H) 7.31 (t, *J* = 7.72 Hz, 1H) 7.39 (br s, 2H) 7.65 (d*, J* = 8.03 Hz, 1H) 7.68-7.75 (m, 2H) 8.23 (br s, 1H) 11.08 (s, 1H) 11.85 (br s, 1H)

**Example 15_B: HNMR (400 MHz, deuterated dimethyl sulfoxide)** δ ppm 1.66 (br s, 1H) 1.86 (br s, 2H) 2.25 (br s, 1H) 3.09 (br s, 2H) 5.24 (br d, *J* = 14.05 Hz, 1H) 5.39-5.49 (m, 1H) 7.31 (t*, J* = 7.78 Hz, 1H) 7.39 (br s, 2H) 7.66 (d*, J* = 8.03 Hz, 1H) 7.69-7.74 (m, 2H) 8.16 (br s, 1H) 11.08 (s, 1H) 11.83 (s, 1H)

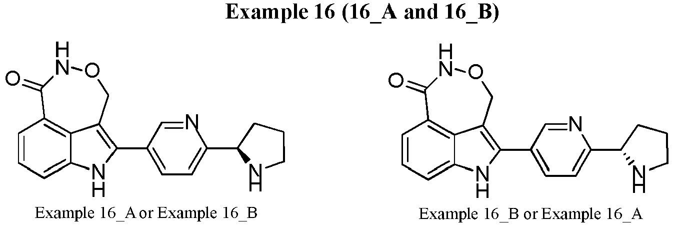

Reference was made to Example 3 for synthesis method.

For Example 16, prior to deprotection of Boc, the compound was separated by chiral HPLC column (separation column: AD (250 mm×30 mm, 10 µm); mobile phase: 0.1% ammonia in isopropanol; elution gradient: 50%-50%, 3.7 min; 52 min) to give two isomers with different configurations: 16_AA (retention time = 0.567 min, ee value (enantiomeric excess): 100 %) and 16_BB (retention time = 0.835 min, ee value (enantiomeric excess): 99%), which were deprotected with trifluoroacetic acid to give Example 16_A (retention time = 0.702 min, ee value (enantiomeric excess): 100 %) and Example 16_B (retention time = 1.301 min, ee value (enantiomeric excess): 100%), respectively.

SFC (supercritical fluid chromatography) method: separation column: Amycoat (50 mm×4.6 mm, I.D. 3 µm); mobile phase: 60% isopropanol (0.05% diethylamine) in CO₂; flow rate: 3 mL/min; wavelength: 220 nm.

**Example 16_A: HNMR (400 MHz, deuterated dimethyl sulfoxide**) δ = 11.98 (s, 1H), 11.11 (s, 1H), 8.79-8.73 (m, 1H), 8.04-7.98 (m, 1H), 7.74-7.66 (m, 3H), 7.33 (s, 1H), 5.47 (d, *J* = 14.7 Hz, 1H), 5.31-5.19 (m, 1H), 4.56-4.46 (m, 1H), 3.23-3.09 (m, 3H), 1.95-1.83 (m, 3H).

**Example 16_B: HNMR (400 MHz, deuterated dimethyl sulfoxide)** δ = 11.99 (s, 1H), 11.23-11.04 (m, 1H), 8.77 (s, 1H), 8.27-8.17 (m, 1H), 8.08-7.99 (m, 1H), 7.73-7.66 (m, 3H), 7.34 (*t, J* = 7.8 Hz, 1H), 5.53-5.42 (m, 1H), 5.29-5.20 (m, 1H), 4.64-4.52 (m, 1H), 3.27-3.14 (m, 3H), 1.86 (br s, 3H).

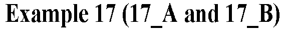

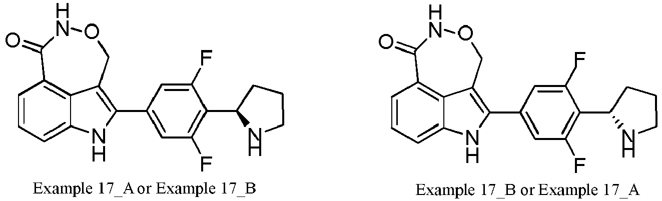

Reference was made to Example 3 for synthesis method.

For Example 17, prior to deprotection of Boc, the compound was separated by chiral HPLC column (separation column: WHELK-O1 (250 mm×50 mm, 10 µm); mobile phase: 0.1% ammonia in ethanol; elution gradient: 45%-45%, 3.2 min; 150 min) to give two isomers with different configurations: 17 AA (retention time = 2.145 min, ee value (enantiomeric excess): 100%) and 17_BB (retention time = 2.396 min, ee value (enantiomeric excess): 93%), which were deprotected with trifluoroacetic acid to give Example 17_A (retention time = 0.622 min, ee value (enantiomeric excess): 100%) and Example 17_B (retention time = 1.114 min, ee value (enantiomeric excess): 95%), respectively.

SFC (supercritical fluid chromatography) method: separation column: Chiralpak OD-3 (50 mm×4.6 mm, I.D. 3 µm); mobile phase: 40% ethanol (0.05% diethylamine) in CO₂; flow rate: 3 mL/min; wavelength: 220 nm.

**Example 17_A: HNMR (400 MHz, deuterated dimethyl sulfoxide)** δ = 11.96 (s, 1H), 11.16-11.06 (m, 1H), 8.31-8.17 (m, 1H), 7.69 (dd, *J =* 7.6, 15.4 Hz, 2H), 7.37-7.28 (m, 3H), 5.51-5.40 (m, 1H), 5.31-5.19 (m, 1H), 4.67-4.56 (m, 1H), 3.17-3.11 (m, 1H), 3.09-3.03 (m, 1H), 2.28-2.18 (m, 1H), 2.03-1.82 (m, 3H).

**Example 17_B: HNMR (400 MHz, deuterated dimethyl sulfoxide)** δ = 11.94-11.87 (m, 1H), 11.14-11.05 (m, 1H), 8.24-8.21 (m, 1H), 7.74-7.63 (m, 2H), 7.37-7.25 (m, 3H), 5.44 (s, 1H), 5.30-5.21 (m, 1H), 4.54-4.47 (m, 1H), 3.10-3.05 (m, 1H), 2.96-2.91 (m, 1H), 2.15 (br s, 1H), 1.98-1.77 (m, 3H).

### Example 18

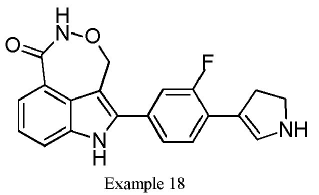

Reference was made to Example 12 for synthesis method.

**Example 18: HNMR (400 MHz, deuterated methanol)** δ 8.53 (s, 1H), 7.79-7.89 (m, 1H), 7.57-7.73 (m, 2H), 7.23-7.50 (m, 3H), 6.54 (br s, 1H), 5.41-5.54 (m, 1H), 5.25-5.38 (m, 1H), 4.51 (br s, 2H), 4.30 (br d, *J =* 2.08 Hz, 2H).

### Example 19 (19_A and 19_B

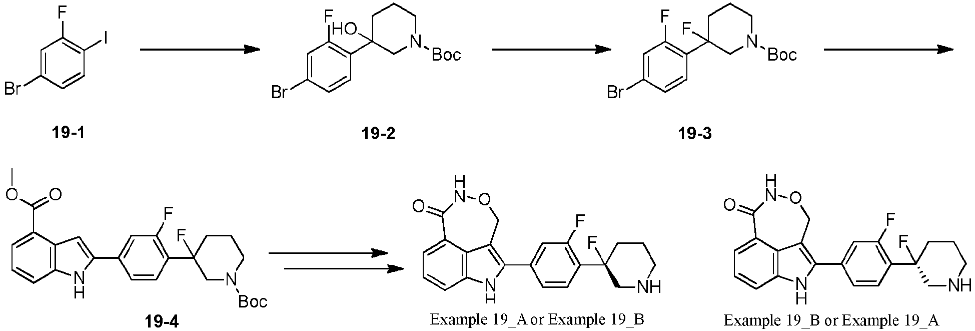

Step A: 19-1 (10 g, 33.23 mmol) was dissolved in tetrahydrofuran (100.00 mL), and *n*-butyllithium (2.5 M, 13.29 mL) was added dropwise to the reaction system at -78 °C under nitrogen atmosphere, and the dropwise addition was completed within half an hour. A solution of N-*tert*-butoxycarbonyl-3-piperidone (6.62 g, 33.23 mmol) in tetrahydrofuran (20 mL) was added dropwise to the reaction system at-78 °C under nitrogen atmosphere. The resulting reaction system was stirred at -78 °C for 1.5 h. The reaction system was then added with saturated aqueous ammonium chloride solution (100 mL) to quench the reaction and extracted with ethyl acetate (100 mL × 3). The organic phases were combined, washed with saturated brine (100 mL), dried over anhydrous sodium sulfate, filtered and concentrated under reduced pressure. The residue was purified by a silica gel column (eluent: petroleum ether/ethyl acetate = 15/1-3/1) to give 19-2.

Step B: 19-2 (9.56 g, 22.83 mmol) was dissolved in dichloromethane (100 mL), and diethylaminosulfur trifluoride (18.40 g, 114.14 mmol) was added to the reaction system at -78 °C under nitrogen atmosphere. The reaction system was stirred at -78 °C for 2 h. The reaction system was then added with aqueous sodium bicarbonate solution (10 mL, pH = 7-8) to quench the reaction and extracted with ethyl acetate (10 mL × 3). The organic phases were combined, washed with saturated brine (10 mL × 3), dried over anhydrous sodium sulfate, filtered and concentrated under reduced pressure. The residue was purified by a silica gel column (eluent: petroleum ether/ethyl acetate = 30/1-5/1) to give 19-3.

For synthesis of 19-4 and subsequent compounds, reference was made to Example 1.

For Example 19, prior to deprotection of Boc, the compound was separated by chiral HPLC column (separation column: AD-H (250 mm×30 mm, 5 µm); mobile phase: 0.1% ammonia in ethanol; elution gradient: 25%-25%, 2.7 min; 570 min) to give two isomers with different configurations: 19 AA (retention time = 1.400 min, ee value (enantiomeric excess): 100 %) and 19_BB (retention time = 1.489 min, ee value (enantiomeric excess): 91.2%), which were deprotected with trifluoroacetic acid to give Example 19_A (retention time = 0.901 min, ee value (enantiomeric excess): 100 %) and Example 19_B (retention time = 1.325 min, ee value (enantiomeric excess): 92%), respectively.

SFC (supercritical fluid chromatography) method: separation column: AD-3 (50 mm×4.6 mm, I.D. 3 µm); mobile phase: 40% isopropanol (0.05% diethylamine) in CO₂; flow rate: 3 mL/min; wavelength: 220 nm.

**Example 19_A: HNMR (400 MHz, deuterated dimethyl sulfoxide)** δ ppm 11.92 (s, 1H), 11.09 (s, 1H), 8.24 (s, 1H), 7.72-7.67 (m, 1H), 7.65-7.64 (m, 2H), 7.474-7.465 (m, 2H), 7.34-7.32 (m, 1H), 5.46-5.42 (d, J = 14.80 Hz, 1H), 5.28-5.25 (d, J = 14.80 Hz, 1H), 3.29-3.22 (dd, J = 23.20 Hz, 4.40 Hz, 1H), 3.08-3.04 (d, J = 12.8 Hz, 1H), 2.73 (s, 1H), 2.51-2.11 (m, 1H), 1.68-1.64 (m, 1H).

**Example 19_B: HNMR (400 MHz, deuterated dimethyl sulfoxide)** δ ppm 11.90 (s, 1H), 11.09 (s, 1H), 8.24 (s, 1H), 7.71-7.69 (m, 1H), 7.67-7.64 (m, 2H), 7.463-7.457 (m, 2H), 7.34-7.32 (m, 1H), 5.45-5.42 (d, J = 14.80 Hz, 1H), 5.28-5.25 (d, J = 14.80 Hz, 1H), 3.22-3.15 (m, 2H), 3.03-3.00 (m, 2H), 2.67 (s, 1H), 2.50-2.09 (m, 2H), 1,83-1.79 (m, 1H), 1.63-1.60 (m, 1H).

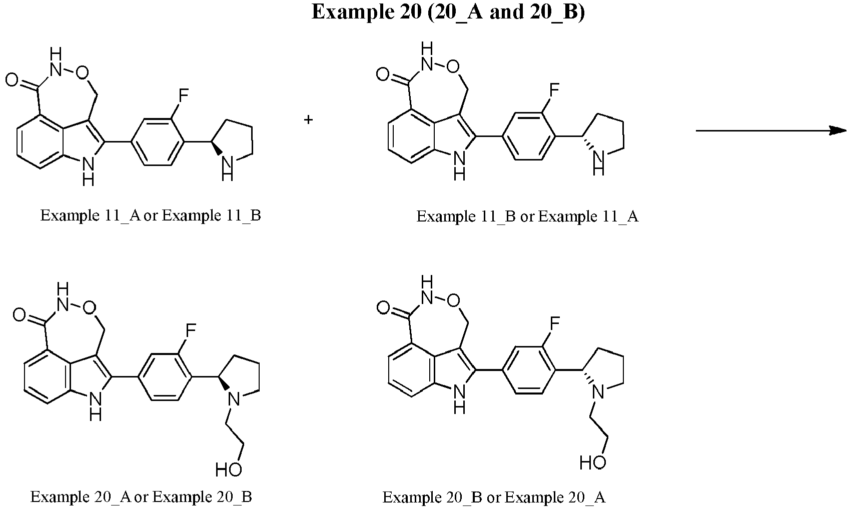

Example 11_A (50 mg, 142.3 µmol) and 2-bromoethanol (21.34 mg, 170.76 µmol) were dissolved in *N*,*N*-dimethylformamide, and then potassium carbonate (23.60 mg, 0.17 mmol) was added. The resulting reaction system was stirred at 60 °C for 16 h. After detection of the reaction completion, the reaction system was filtered to remove insoluble material, and the remaining liquid was dried by rotary evaporation and subjected to preparative separation (column: Phenomenex Synergi C18 (150 µm×25 µm, 10 µm); mobile phase: water (0.225% formic acid)-acetonitrile; B%: 15%-45%, 9 min) to give Example 20_A (retention time = 2.523 min, ee value (enantiomeric excess): 99%). Example 20_B (retention time = 2.130 min, ee value (enantiomeric excess): 99%) can be obtained in the same way.

SFC (supercritical fluid chromatography) method: separation column: Cellucoat (50 mm×4.6 mm, I.D. 3 µm); mobile phase: 5%-40% ethanol (0.05% diethylamine) in CO₂; flow rate: 3 mL/min; wavelength: 220 nm.

**Example 20_A: HNMR (400 MHz, deuterated dimethyl sulfoxide)** δ = 11.91-11.77 (m, 1H), 11.08 (br s, 1H), 7.75-7.63 (m, 3H), 7.43-7.28 (m, 3H), 5.50-5.37 (m, 1H), 5.30-5.19 (m, 1H), 4.50-4.38 (m, 1H), 3.74-3.65 (m, 1H), 3.51-3.43 (m, 1H), 3.40-3.23 (m, 1H), 3.16-2.94 (m, 2H), 2.31-2.13 (m, 2H), 2.04-1.71 (m, 3H), 1.71-1.58 (m, 1H).

**Example 20_B: HNMR (400 MHz, deuterated dimethyl sulfoxide)** δ = 11.84 (s, 1H), 11.42-10.86 (m, 1H), 8.22 (s, 1H), 7.77-7.62 (m, 3H), 7.46-7.24 (m, 3H), 5.51-5.37 (m, 1H), 5.34-5.18 (m, 1H), 3.67 (s, 1H), 3.47 (br d, *J =* 3.2 Hz, 2H), 3.33 (br s, 1H), 2.37-2.14 (m, 5H), 1.88-1.79 (m, 2H), 1.58-1.49 (m, 1H).

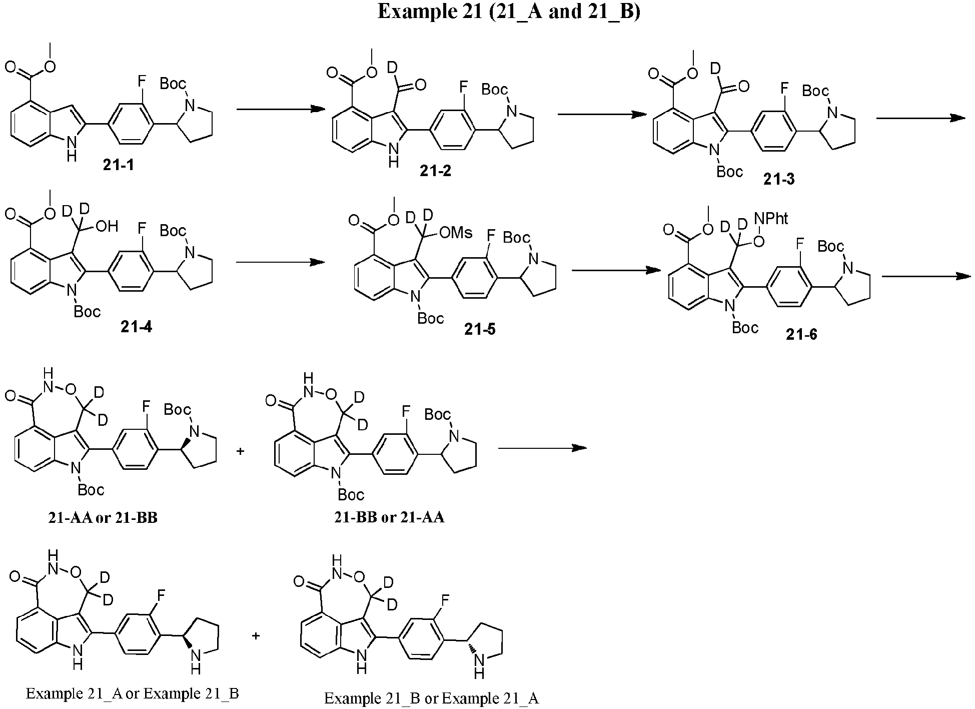

Step A: Oxalyl chloride (0.579 g, 4.56 mmol) was added to dichloromethane (15 mL), and deuterated *N*,*N-*dimethylformamide (0.5 g, 6.84 mmol) was slowly added dropwise at 0 °C under nitrogen atmosphere. The reaction system was stirred at 0 °C for 15 min. 21-1 (1 g, 2.28 mmol) was then dissolved in dichloromethane (5 mL) and added to the reaction system at 0 °C. The reaction system was stirred at 15 °C for 0.5 h. After detection of the reaction completion, the reaction system added with 10% aqueous ammonium acetate solution (30 mL) and tetrahydrofuran (20 mL) to quench the reaction. The organic solvent was removed by rotary evaporation, and the remainder was extracted with ethyl acetate (30 mL × 2). The organic phases were combined, washed with saturated aqueous ammonium chloride solution (30 mL × 3) and saturated brine (30 mL × 3), dried over anhydrous sodium sulfate, filtered and concentrated under reduced pressure to give 21-2.

Step B: 21-2 (2.15 g, 5.08 mmol) was dissolved in dichloromethane (10 mL), and triethylamine (0.65 g, 6.42 mmol), di-*tert*-butyl dicarbonate (0.7 g, 3.21 mmol) and 4-dimethylaminopyridine (26 mg, 0.214 mmol) were added. The reaction system was stirred at 15 °C for 1 h. The reaction system was concentrated by rotary evaporation under reduced pressure, and then added with ethyl acetate (60 mL). The organic phase was washed with saturated aqueous ammonium chloride solution (30 mL × 3) and saturated brine (50 mL), dried over anhydrous sodium sulfate, filtered and concentrated under reduced pressure to give 21-3.

Step C: 21-3 (1.2 g, 2.11 mmol) was dissolved in tetrahydrofuran (8 mL) and methanol (2 mL). The reaction system was cooled to 0 °C and added with deuterated sodium borohydride (120 mg, 3.17 mmol). The reaction system was stirred at 0 °C for 0.5 h. The reaction system was added with saturated aqueous ammonium chloride solution (30 mL) to quench the reaction and extracted with ethyl acetate (30 mL × 2). The organic phases were combined, washed with water (30 mL), dried over anhydrous sodium sulfate, filtered and concentrated under reduced pressure to give 21-4.

Reference can be made to the method of Example 3 to obtain, from Compound 21-4, two isomers with different configurations: 21_AA (retention time = 1.489 min, ee value (enantiomeric excess): 100 %) and 21_BB (retention time = 1.558 min, ee value (enantiomeric excess): 97%), which were deprotected with trifluoroacetic acid to give Example 21_A (retention time = 1.857 min, ee value (enantiomeric excess): 100%) and Example 21_B (retention time = 2.663 min, ee value (enantiomeric excess): 97%), respectively.

SFC (supercritical fluid chromatography) method: separation column: Chiralpak IG-3 (50 mm×4.6 mm, I.D. 3 µm); mobile phase: 40% methanol (0.05% diethylamine) in CO₂; flow rate: 3 mL/min; wavelength: 220 nm.

**Example 21_A: HNMR (400 MHz, deuterated dimethyl sulfoxide)** δ ppm 1.58-1.74 (m, 1H), 1.85 (br dd, *J* = 13.63, 7.27 Hz, 2H), 2.25 (td, *J* = 12.17, 7.09 Hz, 1H), 2.99-3.16 (m, 2H), 4.38-4.53 (m, 1H), 7.24-7.48 (m, 3H), 7.60-7.80 (m, 3H), 8.22 (br s, 1H), 11.07 (br s, 1H), 11.85 (br s, 1H).

**Example 21_B: HNMR (400 MHz, deuterated dimethyl sulfoxide)** δ ppm 1.61-2.01 (m, 3H), 2.21-2.35 (m, 1H), 3.04-3.22 (m, 2H), 4.54 (br s, 1H), 7.27-7.47 (m, 3H), 7.60-7.81 (m, 3H), 8.26 (br s, 1H), 11.08 (br s, 1H), 11.89 (br s, 1H).

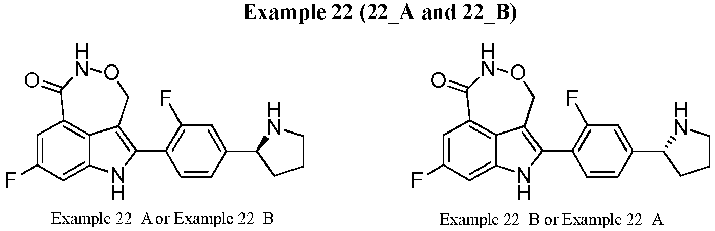

Reference was made to Example 3 for synthesis method.

For Example 22, prior to deprotection of Boc, the compound was separated by chiral HPLC column (separation column: WHELK-O1 (250 mm×50 mm, 10 µm); mobile phase: 0.1% ammonia in ethanol; elution gradient: 40%-40%, 2.8 min; 120 min) to give two isomers with different configurations: 22_AA (retention time = 1.411 min, ee value (enantiomeric excess): 100%) and 22_BB (retention time = 1.613 min, ee value (enantiomeric excess): 99%), which were deprotected with trifluoroacetic acid to give Example 22_A (retention time = 1.523 min, ee value (enantiomeric excess): 100%) and Example 22_B (retention time = 3.001 min, ee value (enantiomeric excess): 100%), respectively.

SFC (supercritical fluid chromatography) method: separation column: Chiralpak AD-3 (50 mm×4.6 mm, I.D. 3 µm); mobile phase: 40% methanol (0.05% diethylamine) in CO₂; flow rate: 3 mL/min; wavelength: 220 nm.

**Example 22_A: HNMR (400 MHz, deuterated methanol)** δ ppm 8.49 (s, 1H), 7.63-7.61 (m, 1H), 7.55-7.53 (m, 1H), 7.473-7.468 (m, 2H), 7.45-7.38 (m, 1H), 5.32-5.28 (d, J = 14.4 Hz, 1H), 5.11-5.08 (d, J = 14.4 Hz, 1H), 4.70-4.68 (m, 1H), 3.50-3.44 (m, 2H), 2.57-2.51 (m. 1H), 2.26-2.18 (m, 3H).

**Example 22_B: HNMR (400 MHz, deuterated methanol)** δ ppm 8.53 (s, 1H), 7.63-7.57 (m, 1H), 7.553-7.547 (m, 1H), 7.49-7.47 (m, 2H), 7.43-7.40 (m, 1H), 5.34-5.31 (d, J = 14.8 Hz, 1H), 5.14-5.10 (d, J = 14.8 Hz, 1H), 4.70-4.66 (m, 1H), 3.49-3.44 (m, 2H), 2.55 (s. 1H), 2.30-2.22 (m, 3H).

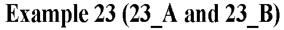

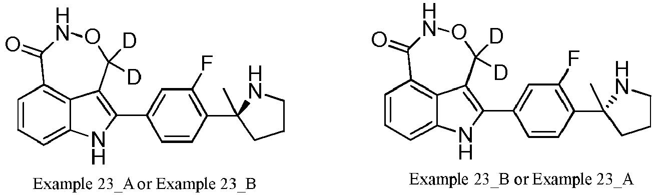

Reference was made to Example 21 for synthesis method.

For Example 23, after deprotection with trifluoroacetic acid, Example 23_A (retention time = 3.712 min, ee value (enantiomeric excess): 99 %) and Example 23_B (retention time = 3.397 min, ee value (enantiomeric excess): 97%) were obtained.

SFC (supercritical fluid chromatography) method: separation column: Cellucoat (50 mm×4.6 mm, I.D. 3 µm); mobile phase: 10%-40% isopropanol (0.05% diethylamine) in CO₂; flow rate: 3 mL/min; wavelength: 220 nm.

**Example 23_A: HNMR (400 MHz, deuterated dimethyl sulfoxide)** δ ppm 1.50 (s, 3H) 1.68-1.79 (m, 1H) 1.87-1.98 (m, 1H) 2.07-2.18 (m, 2H) 2.91-3.01 (m, 1H) 3.13-3.28 (m, 1H) 7.22-7.49 (m, 3H) 7.61-7.82 (m, 3H) 8.20 (s, 1H) 11.07 (s, 1H) 11.85 (s, 1H)

**Example 23_B: HNMR (400 MHz, deuterated dimethyl sulfoxide)** δ ppm 1.50 (s, 3H) 1.69-1.81 (m, 1H) 1.88-1.98 (m, 1H) 2.08-2.18 (m, 2H) 2.89-3.06 (m, 1H) 3.14-3.31 (m, 1H) 7.25-7.48 (m, 3H) 7.62-7.82 (m, 3H) 8.23 (s, 1H) 11.08 (s, 1H) 11.87 (s, 1H).

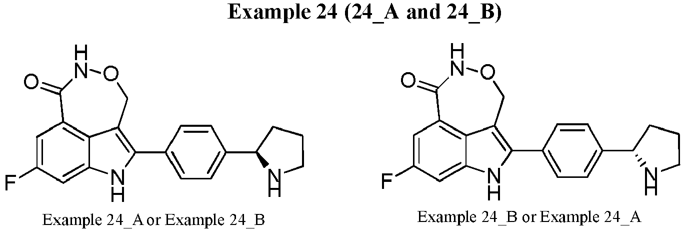

Reference was made to Example 3 for synthesis method.

For Example 24, prior to deprotection of Boc, the compound was separated by chiral HPLC column (separation column: AD-H (250 mm×30 mm, 5 µm); mobile phase: 0.1% ammonia in isopropanol; elution gradient: 30%-30%, 1.5 min; 45 min) to give two isomers with different configurations: 24_AA (retention time = 1.474 min, ee value (enantiomeric excess): 99 %) and 24_BB (retention time = 1.560 min, ee value (enantiomeric excess): 94%), which were deprotected with trifluoroacetic acid to give Example 24_A (retention time = 2.298 min, ee value (enantiomeric excess): 99 %) and Example 24_B (retention time = 2.115 min, ee value (enantiomeric excess): 88%), respectively.

SFC (supercritical fluid chromatography) method: separation column: Cellucoat (50 mm×4.6 mm, I.D. 3 µm); mobile phase: 5%-40% isopropanol (0.05% diethylamine) in CO₂; flow rate: 3 mL/min; wavelength: 220 nm.

**Example 24_A: HNMR (400 MHz, deuterated methanol)** δ 8.54 (br s, 1H), 7.60-7.70 (m, 4H), 7.54 (dd, *J* = 2.26, 10.54 Hz, 1H), 7.40 (dd, *J* = 2.26, 9.03 Hz, 1H), 5.40-5.55 (m, 1H), 5.23-5.37 (m, 1H), 4.70 (br dd, *J* = 6.90, 9.54 Hz, 1H), 3.40-3.61 (m, 2H), 2.48-2.64 (m, 1H), 2.14-2.43 (m, 3H).

**Example 24_B: HNMR (400 MHz, deuterated methanol)** δ 8.52 (br s, 1H), 7.63 (q, *J* = 8.41 Hz, 4H), 7.53 (dd, *J =* 2.20, 10.48 Hz, 1H), 7.38 (dd, *J =* 2.26, 9.03 Hz, 1H), 5.38-5.53 (m, 1H), 5.24-5.35 (m, 1H), 4.71 (br dd, *J* = 6.90, 9.41 Hz, 1H), 3.43-3.61 (m, 2H), 2.48-2.62 (m, 1H), 2.18-2.39 (m, 3H).

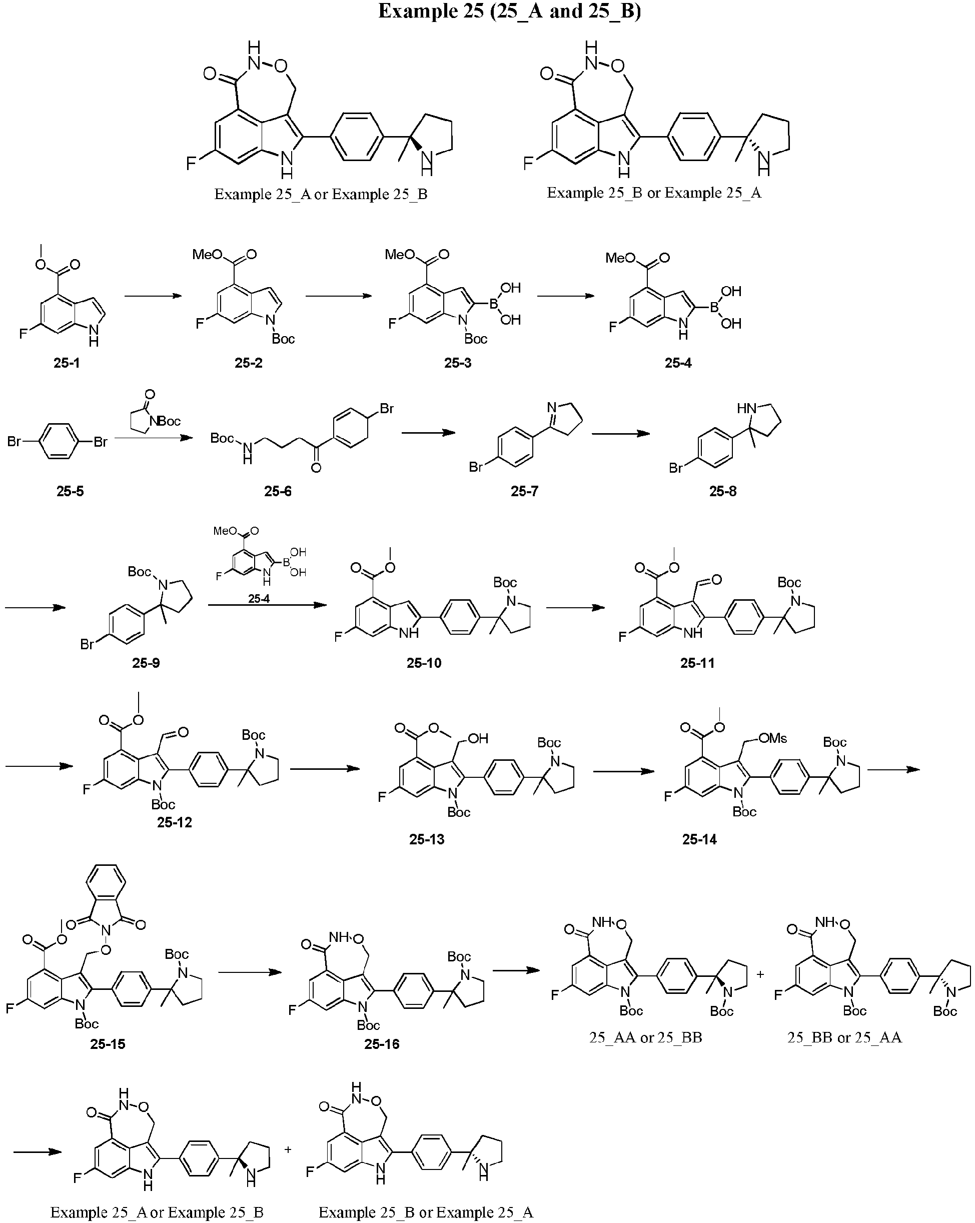

Step A: 25-1 (25 g, 129.42 mmol) was dissolved in dichloromethane (250 mL), and triethylamine (26.19 g, 258.83 mmol), 4-dimethylaminopyridine (3.16 g, 25.88 mmol), di-*tert*-butyl dicarbonate (31.07 g, 142.36 mmol) were added at 0 °C. The reaction system was stirred at 25 °C for 2 h. The reaction system was washed with saturated aqueous ammonium chloride solution (80 mL × 3) and saturated brine (50 mL × 2), dried over anhydrous sodium sulfate, filtered and concentrated under reduced pressure to give 25-2.

Step B: Diisopropylamine (13.80 g, 136.38 mmol) was dissolved in tetrahydrofuran (60 mL), and *n*-butyllithium (2.5 M, 47.73 mL) was added dropwise to the reaction system at -78 °C under nitrogen atmosphere, and the dropwise addition was completed within half an hour. The reaction system was stirred at 0 °C for half an hour, and then added dropwise to another three-necked flask containing a solution of 25-2 (25 g, 285.14 mmol) and triisopropyl borate (24.05 g, 127.86 mmol) in tetrahydrofuran (200 mL) at 0 °C under nitrogen atmosphere, and the dropwise addition was completed quickly at 0 °C. The resulting reaction system was stirred at 0 °C for 1 h. The reaction system was then added with acetic acid solution (50 mL) to quench the reaction, diluted with water (60 mL) and extracted with ethyl acetate (60 mL × 3). The organic phases were combined, washed with saturated aqueous ammonium chloride solution (50 mL × 3) and saturated brine (40 mL × 2), dried over anhydrous sodium sulfate, filtered and concentrated under reduced pressure to give a crude product. The crude product was slurried with acetonitrile (100 mL) and aqueous solution (300 mL), and the filter cake was dried with an oil pump to give 25-3.

Step C: 25-3 (45 g, 133.49 mmol) was added to a solution of trifluoroacetic acid (200 mL) at 0 °C in three portions, and the reaction system was stirred at 0 °C for 1 h under nitrogen atmosphere. The reaction system was then poured into ice water (300 mL) to precipitate a solid, and a filter cake was obtained and concentrated under reduced pressure with an oil pump to give 25-4.

Step D: 25-5 (25 g, 105.98 mmol) was dissolved in tetrahydrofuran (100.00 mL) and then added dropwise to a three-necked flask containing magnesium chips (2.58 g, 105.98 mmol) and iodine (489.05 mg, 1.93 µmol) at 70 °C under nitrogen atmosphere, and the dropwise addition was completed within half an hour. The reaction system was stirred at 70 °C for 1 h and then cooled to 20 °C. The reaction system was added dropwise into another three-necked flask containing a solution of *tert*-butyl 2-oxopyrrolidine-1-carboxylate (17.84 g, 96.34 mmol) in tetrahydrofuran (150 mL) at -70 °C under nitrogen atmosphere, and the dropwise addition was completed within half an hour. The resulting reaction system was stirred at -70 °C for 2 h, and then heated to 10 °C and stirred for 1 h. The reaction system was added with saturated aqueous ammonium chloride solution (60 mL) to quench the reaction and extracted with ethyl acetate (60 mL × 3). The organic phases were combined, washed with saturated brine (50 mL), dried over anhydrous sodium sulfate, filtered and concentrated under reduced pressure. The residue was purified by a silica gel column to give 25-6.

Step E: 25-6 (50 g, 146.10 mmol) was added to trifluoroacetic acid (250 mL) at 0 °C. The reaction system was stirred at 15 °C for 12 h. The reaction system was adjusted to pH = 14 with 40% aqueous sodium hydroxide solution and a yellow solid was precipitated. The resulting mixture was filtered, and the filter cake was washed with a small amount of water and subjected to rotary evaporation to give 25-7.

Step F: 25-7 (15 g, 66.94 mmol) was dissolved in tetrahydrofuran (150 mL). The reaction system was cooled to -78 °C under nitrogen atmosphere, and boron trifluoride diethyl etherate (19 g, 133.87 mmol) was added dropwise to the reaction system, and the dropwise addition was completed within half an hour. The reaction system was stirred at -78 °C for half an hour. Methyllithium solution (1.6 M, 83.67 mL) was then added dropwise to the reaction system. The reaction system was slowly heated to 78 °C and stirred for 19.5 h. The reaction system was cooled to room temperature, added with saturated aqueous sodium bicarbonate solution (100 mL) to quench the reaction, added with water (30 mL) and extracted with ethyl acetate (80 mL × 3). The organic phases were combined, washed with saturated brine (30 mL × 2), dried over anhydrous sodium sulfate, filtered and concentrated under reduced pressure to give 25-8.

Step G: 25-8 (16 g, 66.63 mmol) was dissolved in dichloromethane (150 mL), and triethylamine (20.23 g, 199.88 mmol) was added at 0 °C, followed by addition of di-*tert*-butyl dicarbonate (29.08 g, 133.26 mmol). The reaction system was stirred at 15 °C for 1 h. The reaction system was concentrated by rotary evaporation under reduced pressure, added with saturated aqueous ammonium chloride solution (60 mL) and extracted with ethyl acetate (80 mL × 3). The organic phases were combined, washed with saturated brine (30 mL × 3), dried over anhydrous sodium sulfate, filtered and concentrated under reduced pressure to give 25-9.

Step H: 25-9 (9 g, 26.45 mmol) and 25-4 (7.52 g, 31.74 mmol) were dissolved in ethylene glycol dimethyl ether (100 mL) and water (20 mL), and sodium bicarbonate (6.67 g, 79.35 mmol) and [1,1-bis(di-*tert*-butylphosphino)ferrocene]palladium dichloride (1.72 g, 2.65 mmol) were added. After purge with nitrogen three times, the reaction system was stirred at 80 °C for 12 h under nitrogen atmosphere. The reaction system was concentrated by rotary evaporation under reduced pressure, added with saturated brine (60 mL) and extracted with ethyl acetate (60 mL × 3). The organic phases were combined, washed with saturated brine (50 mL), dried over anhydrous sodium sulfate, filtered and concentrated under reduced pressure to give a residue. The residue was purified by a silica gel column to give 25-10.

Step I: Oxalyl chloride (5.61 g, 44.20 mmol) was added to dichloromethane (150 mL), and *N*,*N-*dimethylformamide (4.85 g, 66.30 mmol) was slowly added dropwise at 0 °C under nitrogen atmosphere. The reaction system was stirred at 0 °C for 15 min. 25-10 (10 g, 22.10 mmol) was then dissolved in dichloromethane (50 mL) and added to the reaction system at 0 °C. The reaction system was stirred at 15 °C for 0.5 h. The reaction system was added with 10% aqueous ammonium acetate solution (100 mL) and tetrahydrofuran (100 mL) to quench the reaction and extracted with ethyl acetate (45 mL × 2). The organic phases were combined, washed with saturated aqueous ammonium chloride solution (50 mL × 3) and saturated brine (50 mL × 3), dried over anhydrous sodium sulfate, filtered and concentrated under reduced pressure to give 25-11.

Step J: 25-11 (10.62 g, 22.10 mmol) was dissolved in dichloromethane (80 mL), and triethylamine (6.71 g, 66.30 mmol), di-*tert*-butyl dicarbonate (9.65 g, 44.20 mmol) and 4-dimethylaminopyridine (810.01 mg, 6.63 mmol) were added at 0 °C. The reaction system was stirred at 15 °C for 1 h. The reaction system was concentrated by rotary evaporation under reduced pressure, added with saturated aqueous ammonium chloride solution (60 mL) and extracted with ethyl acetate (60 mL × 3). The organic phases were combined, washed with saturated brine (30 mL × 3), dried over anhydrous sodium sulfate, filtered and concentrated under reduced pressure to give 25-12.

Step K: 25-12 (12.75 g, 21.96 mmol) was dissolved in tetrahydrofuran (100 mL) and methanol (25 mL). The reaction system was cooled to 0 °C and added with sodium borohydride (1.25 g, 32.94 mmol). The reaction system was stirred at 0 °C for 40 min. The reaction system was added with saturated aqueous ammonium chloride solution (80 mL) to quench the reaction and extracted with ethyl acetate (80 mL × 2). The organic phases were combined, washed with water (50 mL), dried over anhydrous sodium sulfate, filtered and concentrated under reduced pressure to give 25-13.

Step L: 25-13 (12.79 g, 21.95 mmol) was dissolved in dichloromethane (150 mL), and triethylamine (4.44 g, 43.90 mmol) was added, followed by addition of methanesulfonyl chloride (3.02 g, 26.34 mmol) at 0 °C under nitrogen atmosphere. The reaction system was stirred at 0 °C for 1 h. The reaction system was concentrated by rotary evaporation under reduced pressure, and then added with ethyl acetate (80 mL). The organic phase was washed with saturated aqueous ammonium chloride solution (30 mL × 2) and saturated brine (20 mL × 2), dried over anhydrous sodium sulfate, filtered and concentrated under reduced pressure to give 25-14.

Step M: 25-14 (14.45 g, 21.87 mmol) was dissolved in *N*,*N-*dimethylformamide (150 mL), and sodium carbonate (4.64 g, 43.74 mmol) and *N*-hydroxyphthalimide (5.35 g, 32.80 mmol) were added. The reaction system was stirred at 65 °C for 12 h. The reaction system was added with aqueous solution (60 mL) and extracted with ethyl acetate (80 mL × 2). The organic phase was washed with saturated aqueous ammonium chloride solution (50 mL × 3) and saturated brine (50 mL × 3), dried over anhydrous sodium sulfate, filtered and concentrated under reduced pressure to give a residue. The residue was purified by a silica gel column to give 25-15.

Step N: 25-15 (15 g, 20.61 mmol) was dissolved in methanol (200 mL), and 98% hydrazine hydrate (3.10 g, 61.83 mmol) was added. The reaction system was stirred at 65 °C for 2 h under nitrogen atmosphere. The reaction system was filtered and concentrated under reduced pressure to give a residue. The residue was purified by silica gel column to give 25-16.

Step O: Compound 25-16 was separated by chiral HPLC column (separation column: AD-H (250 mm×30 mm, 5 µm); mobile phase: 0.1% ammonia in isopropanol; elution gradient: 25%-25%, 2.7 min; 400 min) to give two isomers with different configurations: 25_AA (retention time = 2.161 min, ee value (enantiomeric excess): 100 %) and 25_BB (retention time = 2.353 min, ee value (enantiomeric excess): 97%), which were deprotected with trifluoroacetic acid to give Example 25_A (time = 3.461 min, ee value (enantiomeric excess): 98%) and Example 25_B (time = 3.128 min, ee value (enantiomeric excess): 90%), respectively.

Method for measuring ee value (enantiomeric excess): separation column: Chiralcel Cellucoat (50 mm×4.6 mm, I.D. 3 µm); mobile phase: 10%-40% isopropanol (0.05% diethylamine) in CO₂; flow rate: 3 mL/min; wavelength: 220 nm.

**Example 25_A: ¹H NMR (400 MHz, deuterated dimethyl sulfoxide)** δ = 12.00 (br s, 1H), 11.30 (br s, 1H), 8.23 (br s, 1H), 7.62 (br d, *J* = 7.75 Hz, 4H), 7.45 (br d, *J* = 9.13 Hz, 2H), 5.44 (br d, *J* = 14.51 Hz, 1H), 5.14-5.31 (m, 1H), 2.99-3.42 (m, 2H), 1.89-2.23 (m, 4H), 1.53 (br s, 3H)

**Example 25_B: ¹H NMR (400 MHz, deuterated dimethyl sulfoxide)** δ = 12.01 (br s, 1H), 11.30 (s, 1H), 8.17 (s, 1H), 7.63 (s, 4H), 7.40-7.49 (m, 2H), 5.45 (br d, *J* = 14.55 Hz, 1H), 5.22 (dd, *J =* 7.40, 14.73 Hz, 1H), 3.28 (br d, *J* = 8.44 Hz, 2H), 1.98-2.31 (m, 4H), 1.56 (s, 3H).

### Example 26 (26_A and 26_B

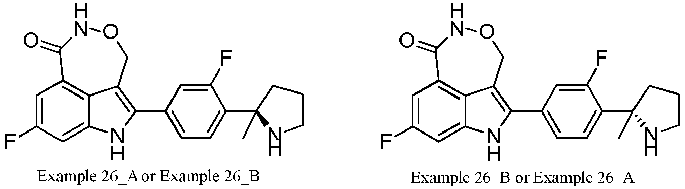

Reference was made to Example 6 for synthesis method.

Finally, Example 26_A (retention time = 11.430 min, ee value (enantiomeric excess): 100%) and Example 26_B (retention time = 8.159 min, ee value (enantiomeric excess): 100%) were obtained through deprotection with trifluoroacetic acid.

Chiral HPLC method: separating column: Chiralpak IA-3 (50 mm×4.6 mm, 3 µm); mobile phase: phase A, *n*-heptane (0.05% diethylamine), phase B, 8% isopropanol + acetonitrile (4:1) (0.05% diethylamine); flow rate: 1 mL/min; wavelength: 220 nm.

**Example 26_A: HNMR (400 MHz, deuterated methanol)** δ 8.54 (s, 1H), 7.63 (t, J = 8.19 Hz, 1H), 7.55 (dd, J = 2.25, 10.51 Hz, 1H), 7.37-7.48 (m, 3H), 5.41-5.52 (m, 1H), 5.27-5.37 (m, 1H), 3.47-3.56 (m, 1H), 3.37-3.45 (m, 1H), 2.39-2.58 (m, 2H), 2.11-2.38 (m, 2H), 1.70 (s, 3H).

**Example 26_B: HNMR (400 MHz, deuterated methanol)** δ 8.52 (s, 1H), 7.63 (t, J = 8.25 Hz, 1H), 7.56 (dd, J = 2.31, 10.44 Hz, 1H), 7.37-7.50 (m, 3H), 5.43-5.51 (m, 1H), 5.28-5.36 (m, 1H), 3.55 (ddd, J = 4.82, 9.35, 11.85 Hz, 1H), 3.38-3.48 (m, 1H), 2.41-2.62 (m, 2H), 2.16-2.39 (m, 2H), 1.72 (s, 3H).

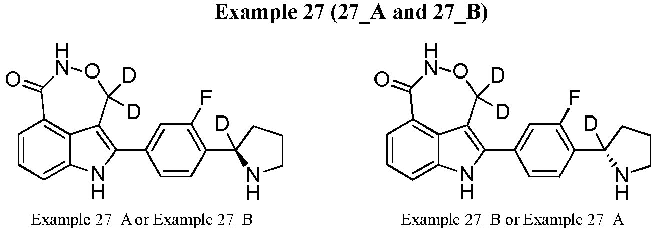

Reference was made to Example 21 and Example 8 for synthesis method.

For Example 27, prior to deprotection of Boc, the compound was separated by chiral HPLC column (separation column: AD-H (250 mm×30 mm, 5 µm); mobile phase: 0.1% ammonia in isopropanol; elution gradient: 25%-25%, 4.1 min; 104 min) to give two isomers with different configurations: 27_AA (retention time = 1.391 min, ee value (enantiomeric excess): 98%) and 27_BB (retention time = 1.482 min, ee value (enantiomeric excess): 94%), which were deprotected with trifluoroacetic acid to give Example 27_A (retention time = 2.294 min, ee value (enantiomeric excess): 99%) and Example 27_B (retention time = 2.116 min, ee value (enantiomeric excess): 93%), respectively.

SFC (supercritical fluid chromatography) method: separation column: Cellucoat (50 mm×4.6 mm, I.D. 3 µm); mobile phase: 5%-40% ethanol (0.05% diethylamine) in CO₂; flow rate: 3 mL/min; wavelength: 220 nm.

**Example 27_A: HNMR (400 MHz, deuterated dimethyl sulfoxide)** δ ppm 1.76-2.06 (m, 3H), 2.30 (ddd, *J* = 11.57, 7.13, 4.19 Hz, 1H), 3.11-3.23 (m, 2H), 7.32 (t, *J* = 7.75 Hz, 1H), 7.40-7.53 (m, 2H), 7.63-7.79 (m, 3H), 8.26 (s, 1H), 11.11 (br s, 1H), 11.93 (s, 1H).

**Example 27_B: HNMR (400 MHz, deuterated dimethyl sulfoxide)** δ ppm 1.77-2.04 (m, 3H), 2.24-2.40 (m, 1H), 3.09-3.33 (m, 2H), 7.33 (t, *J* = 7.75 Hz, 1H), 7.40-7.51 (m, 2H), 7.64-7.78 (m, 3H), 8.21 (s, 1H), 11.11 (s, 1H), 11.92 (s, 1H).

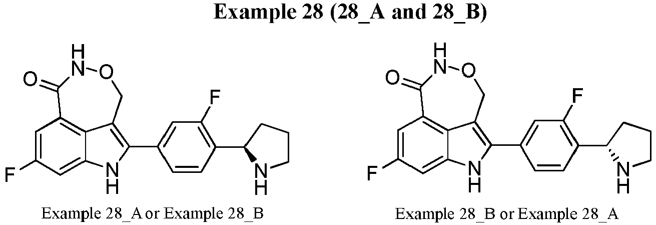

Reference was made to Example 3 for synthesis method.

For Example 28, prior to deprotection of Boc, the compound was separated by chiral HPLC column (separation column: AD-H (250 mm×30 mm, 5 µm); mobile phase: 0.1% ammonia in isopropanol; elution gradient: 20%-20%, 2.3 min; 960 min) to give two isomers with different configurations: 28_AA (retention time = 1.474 min, ee value (enantiomeric excess): 99 %) and 28_BB (retention time = 1.560 min, ee value (enantiomeric excess): 94%), which were deprotected with trifluoroacetic acid to give Example 28_A (retention time = 2.619 min, ee value (enantiomeric excess): 100%) and Example 28_B (retention time = 2.350 min, ee value (enantiomeric excess): 96%), respectively.

SFC (supercritical fluid chromatography) method: separation column: Chiralpak AD-3 (50 mm×4.6 mm, I.D. 3 µm); mobile phase: 40% isopropanol (0.05% diethylamine) in CO₂; flow rate: 3 mL/min; wavelength: 220 nm.

**Example 28_A: HNMR (400 MHz, deuterated dimethyl sulfoxide)** δ ppm 1.56-1.70 (m, 1H) 1.77-1.93 (m, 2H) 2.18-2.32 (m, 1H) 2.93-3.18 (m, 2H) 4.45 (br t, *J* = 7.47 Hz, 1 H) 5.18-5.50 (m, 2H) 7.31-7.52 (m, 4H) 7.72 (t, *J*= 7.97 Hz, 1H) 8.25 (s, 1H) 11.29 (br s, 1H) 12.01 (s, 1H).

**Example 28_B: HNMR (400 MHz, deuterated dimethyl sulfoxide)** δ ppm 1.67 (dq, *J* = 12.25, 8.17 Hz, 1H), 1.78-1.98 (m, 2H), 2.18-2.33 (m, 1H), 2.99-3.15 (m, 2H), 4.48 (br t, *J* = 7.65 Hz, 1H), 5.15-5.30 (m, 1H), 5.35-5.50 (m, 1H), 7.31-7.52 (m, 4H), 7.64-7.79 (m, 1H), 8.27 (s, 1H), 11.30 (br s, 1H), 12.04 (s, 1H).

### Experimental Example 1: PARP-1 Enzymatic Experiment

**Experimental materials:** test compounds; HT Universal Chemiluminescent PARP Assay kit (purchased from TREVIGEN); PBS (purchased from Wisent); Triton X-100 (purchased from Macklin); Envision multi-marker analyzer (PerkinElmer).

### Experimental procedures:

### (I) Preparation of reagents:

1. Washing solution: Triton X-100 was added to 1-fold PBS, and the final concentration of Triton X-100 was 0.1%.
2. 1-fold PARP buffer: the 20-fold PARP buffer in the kit was subjected to 20-fold dilution with water to prepare a 1-fold PARP buffer. This buffer was used to prepare the compound, enzyme solution and substrate solution.
3. 1-fold Strep-Diluent solution: the 10-fold Strep-diluent in the kit was subjected to 10-fold dilution with water to prepare a 1-fold Strep-diluent solution.

### (II) Preparation of test compounds:

Test compounds were serially 5-fold diluted to the 8th concentration with a multichannel pipette, i.e. from 200 µM to 2.56 nM, with the DMSO concentration being 100%. 2 µL of each of the inhibitors at various concentration gradients was added to a compound intermediate plate, and 38 µL of the 1-fold PARP buffer was then added; the two were mixed well for use, and the DMSO concentration was 5%.

### (III) Experimental method:

a) The 1-fold PARP buffer was added to a test plate at 50 µL per well and incubated at 25 °C for 30 min.
b) After the incubation was completed, the liquid in the test plate was discarded, and each of the compounds at various concentration gradients was pipetted from the compound intermediate plate and added into the test plate at 10 µL per well. A duplicate-well experiment was performed.
c) The test plate was added with the enzyme solution (0.5 IU) at 15 µL per well. The compound and enzyme were incubated together at 25 °C for 10 min.
d) After the incubation was completed, 25 µL of the 1-fold PARP Cocktail (comprising 2.5 µL of 10-fold PARP Cocktail, 2.5 µL of 10-fold Activated DNA and 20 µL of 1-fold PARP buffer) was added to each well of the test plate. The test plate was incubated at 25 °C for 1 h. The final concentration of the compound was 2 µM to 0.0256 nM, and the DMSO concentration was 1%.
e) After the incubation was completed, the test plate was washed twice with 200 µL of washing solution per well and then washed twice with 200 µL of PBS per well.
f) Strep-HRP in the kit was subjected to 500-fold dilution with the 1-fold Strep-Diluent solution, added to the test plate at 50 µL per well, and then incubated at 25 °C for 1 h.
g) After the incubation was completed, the test plate was washed twice with 200 µL of washing solution per well and then washed twice with 200 µL of PBS per well.

PeroxyGlow A and B in the kit were mixed at ratio of 1:1, and the mixed solution was added to the test plate at 100 µL per well. Chemiluminescence was immediately read using a PerkinElmer Envision multi-marker analyzer with an integration time of 0.5 s.

**Data analysis:** the original data was converted to inhibition using the equation (sample - Min)/(Max - Min) × 100%, and the IC₅₀ value was then curve fitted using four parameters (obtained from the "log(inhibitor) vs. response-variable slope" model in GraphPad Prism). Table 1 provides the enzymatic inhibitory activity of the compounds disclosed herein against PARP1.

### Experimental results:

The PARP-1 kinase inhibitory activities of the compounds disclosed herein were determined by the above experimental method and the *in vitro* enzymatic inhibitory activities (IC₅₀) of the compounds are shown in Table 1.

**Experimental conclusion:** the compounds disclosed herein show excellent inhibitory activity against PARP1.

**Table 1. PARP-1 kinase activities of compounds**

| **Compound number** | **PARP1 (IC₅₀, nM)** | **Compound number** | **PARP1 (IC₅₀, nM)** |
|---|---|---|---|
| Example 6_A | 2.8 | Example 23_A | 3.7 |
| Example 8_A | 2.7 | Example 24_A | 2.0 |
| Example 11_A | 3.6 | Example 24_B | 2.5 |
| Example 15_B | 7.1 | Example 25_A | 2.3 |
| Example 16_B | 2.8 | Example 25_B | 2.8 |
| Example 19_B | 3.5 | Example 26_A | 3.4 |
| Example 20_B | 3.8 | Example 26_B | 3.6 |
| Example 21_B | 5.6 | Example 28_A | 2.9 |
| Example 22_A | 4.9 | Example 28_B | 3.4 |
| Example 22_B | 4.2 | / | / |

### Experimental Example 2: Anti-Proliferation Experiment on MDA-MB-436 CTG Cells

**Experimental materials:** test compounds; RPMI-1640 medium; fetal bovine serum; penicillin/streptomycin antibiotic; MDA-MB-436 cell line; Envision multi-marker analyzer (PerkinElmer).

### Experimental procedures:

### 1. Experimental method:

MDA-MB-436 cells were seeded in a white 96-well plate by adding 80 µL of cell suspension (containing 3000 MDA-MB-436 cells) to each well. The cell plate was incubated in a CO₂ incubator overnight.

Test compounds were serially 5-fold diluted to the 8th concentration with a multichannel pipette, i.e. from 2 mM to 26 nM, and a duplicate well experiment was performed. 78 µL of medium was added to a intermediate plate, 2 µL of each of serially diluted compounds was transferred to corresponding wells of the intermediate plate, and after mixing, the mixture was transferred to the cell plate at 20 µL per well. The cell plate was incubated in a CO₂ incubator for 7 days. Another cell plate was provided for reading signal values on the day of compound addition and these signal values were used as Max values in data analysis. Promega CellTiter-Glo was added to this cell plate at 25 µL per well and the luminescence signals were stabilized by incubation for 10 min at room temperature. Readings were taken using a PerkinElmer Envision multi-marker analyzer.

Promega CellTiter-Glo was added to the cell plate at 25 µL per well and the luminescence signals were stabilized by incubation for 10 min at room temperature. Readings were taken using a PerkinElmer Envision multi-marker analyzer.

2. Data analysis: the original data was converted to inhibition using the equation (sample - Min)/(Max - Min) × 100%, and the IC₅₀ value was then curve fitted using four parameters (obtained from the "log(inhibitor) vs. response-variable slope" model in GraphPad Prism). Table 2 provides the inhibitory activity of the compounds disclosed herein against MDA-MB-436 cell proliferation.

**Experimental results:** the anti-proliferative activities of the compounds disclosed herein against BRCA1-mutated MDA-MB-436 cells were determined by the experimental method above, and the half maximal inhibitory concentrations (IC₅₀) of the compounds for *in vitro* anti-proliferation are shown in Table 2.

**Experimental conclusion:** the compounds disclosed herein have excellent anti-proliferative activity against BRCA1-mutated MDA-MB-436 cells.

**Table 2. Inhibitory activity of compounds disclosed herein against MDA-MB-436 cell proliferation**

| **Compound number** | **MDA-MB-436 (IC₅₀, nM)** | **Compound number** | **MDA-MB-436 (IC₅₀, nM)** |
|---|---|---|---|
| Example 1_A | 18.8 | Example 15_A | 134.8 |
| Example 1_B | 60.5 | Example 15_B | 89.6 |
| Example 2_A | 75.2 | Example 16_B | 44.6 |
| Example 2_B | 50.2 | Example 17_B | 163.6 |
| Example 3_A | 28.1 | Example 18 | 134.3 |
| Example 3_B | 26.5 | Example 19_B | 23.97 |
| Example 4_A | 40.5 | Example 20_B | 61.6 |
| Example 4_B | 11.0 | Example 21_A | 80.6 |
| Example 6_A | 16.9 | Example 21_B | 69.8 |
| Example 6_B | 47.6 | Example 22_A | 125.0 |
| Example 7_A | 52.6 | Example 22_B | 61.6 |
| Example 7_B | 39.5 | Example 23_A | 30.9 |
| Example 8_A | 20.8 | Example 23_B | 108.3 |
| Example 8_B | 23.5 | Example 24_A | 9.6 |
| Example 9_A | 100.7 | Example 24_B | 4.4 |
| Example 10 | 27 | Example 25_A | 12.3 |
| Example 11_A | 70.2 | Example 25_B | 20.7 |
| Example 11_B | 156.8 | Example 26_A | 8.4 |
| Example 12_A | 131.2 | Example 26_B | 29.3 |
| Example 13_A | 117.7 | Example 27_A | 21.1 |
| Example 13_B | 184.5 | Example 27_B | 69.6 |
| Example 14_A | 165.0 | Example 28_A | 73 |
| Example 14_B | 195.5 | Example 28_B | 50 |

### Experimental Example 3: Anti-Proliferation Experiment on MDA-MB-231 CTG Cells

**Experimental materials:** test compounds; R DMEM medium; fetal bovine serum; penicillin/streptomycin antibiotic; MDA-MB-231 cell line; Envision multi-label analyzer.

### Experimental method:

MDA-MB-231 cells were seeded in a white 96-well plate by adding 80 µL of cell suspension (containing 5000 MDA-MB-231 cells) to each well. The cell plate was incubated in a CO₂ incubator overnight.

Eight concentration points were set for each compound, test compounds were serially 3-fold diluted to the 8th concentration with a multichannel pipette, i.e. from 2 mM to 920 nM, and a duplicate well experiment was performed. 78 µL of medium was added to a intermediate plate, 2 µL of each of serially diluted compounds was transferred to corresponding wells of the intermediate plate, and after mixing, the mixture was transferred to the cell plate at 20 µL per well. The cell plate was incubated in a CO₂ incubator for 3 days. Another cell plate was provided for reading signal values on the day of compound addition and these signal values were used as Max values in data analysis. Promega CellTiter-Glo was added to this cell plate at 25 µL per well and the luminescence signals were stabilized by incubation for 10 min at room temperature. Readings were taken using a PerkinElmer Envision multi-marker analyzer.

Promega CellTiter-Glo was added to the cell plate at 25 µL per well and the luminescence signals were stabilized by incubation for 10 min at room temperature. Readings were taken using a PerkinElmer Envision multi-marker analyzer.

**Data analysis:** the original data was converted to inhibition using the equation **(sample ― Min)/(Max ― Min)** × **100%,** and the IC₅₀ value was then curve fitted using four parameters (obtained from the "log(inhibitor) vs. response-variable slope" model in GraphPad Prism). Table 1 provides the inhibitory activity of the compounds disclosed herein against MDA-MB-231 cell proliferation.

**Experimental results:** the anti-proliferative activities of the compounds disclosed herein against BRCA-wild type MDA-MB-231 cells were determined by the experimental method above, and the half maximal inhibitory concentrations (IC₅₀) of the compounds for *in vitro* anti-proliferation are shown in Table 3.

**Table 3. Inhibitory activity of compounds disclosed herein against BRCA wild type**

| **Compound number** | **MDA-MB-231 (IC₅₀, nM)** |
|---|---|
| Example 6_A | > 10 µm |
| Example 11_A | > 10 µm |
| Example 22_B | > 10 µm |
| Example 24_A | > 10 µm |
| Example 25_A | > 10 µm |
| Example 26_A | > 10 µm |

**Experimental conclusion**: the compounds disclosed herein have little inhibitory activity against BRCA-wild type MDA-MB-231 cells, which shows that the compounds have excellent selectivity.

### Experimental Example 4: Anti-Proliferation Experiment on PARylation

**Experimental materials:** test compounds; F12K medium; Lovo cells; Anti-Poly (ADP-ribose) mouse monoclonal antibody; FITC-labeled goat anti-mouse IgG; hydrogen peroxide; DAPI; PBS; methanol; acetone; Tween-20; skimmed milk powder; Envision multi-marker analyzer.

### Preparation of reagents:

Day one: Lovo cells were plated on a plate at 60,000 cells/well, and then incubated overnight at 37 °C/5% CO₂.
Day two: reagents were prepared:
   1. Washing solution: Tween-20 was added to 1-fold PBS, and the final concentration of Tween-20 was 0.05%.
   2. Blocking solution: skimmed milk powder was added to the washing solution, and the final concentration of skimmed milk powder was 5%.
   3. Cell stationary solution: methanol and acetone were mixed in a ratio of 7:3.

Preparation of test compounds: compound intermediate plate 1: compounds were diluted to final concentrations of 10 µM to 0.13 nM with DMSO and PBS, and the concentration of DMSO was 1%. Compound intermediate plate 2: compounds were diluted to final concentrations of 10 µM to 0.13 nM with DMSO and PBS containing 50 mM hydrogen peroxide, and the concentration of DMSO was 1%.

### Experimental procedures:

1. Cell supernatant was removed, and compound was transferred from the compound intermediate plate 1 to the cell plate at 40 µL per well, and the mixture was incubated at 37 °C for 30 min.
   Compound wells: compound, DMSO 1%;
   Negative and positive controls: 1% DMSO added;
   Blank controls: cell-free wells, PBS added.
2. After the incubation was completed, compound was transferred from the compound intermediate plate 2 to the cell plate at 40 µL per well, and the final concentration of H₂O₂ was 25 mM.
   Compound wells: compound + 25 mM H₂O₂
   Positive and negative controls: 1% DMSO + 25 mM H₂O₂
   Blank controls: cell-free wells, PBS added
4. After the incubation was completed, the cell plate was washed once with PBS pre-cooled on ice and added with 100 µL of pre-cooled cell stationary solution per well. After the cell plate was left to stand at -20 °C for 10 min, the cell stationary solution was shaken off.
5. After being air-dried, the cell plate was washed with PBS at 200 µL per well, and then the PBS was discarded.
6. The cell plate was added with blocking solution at 100 µL per well and incubated at 25 °C for 30 min, after which the blocking solution was shaken off.
7. Anti-PAR antibody which was diluted in blocking solution at a ratio of 1:50, was added to the cell plate at 25 µL per well, and then the mixture was incubated at 25 °C for 60 min.
   Negative control wells: blocking solution added at 25 µL/well
   Blank control wells: blocking solution added at 25 µL/well
8. After the incubation was completed, the cell plate was washed 4 times with 200 µL of washing solution per well, 3 min each time, and then the washing solution was shaken off.
9. Blocking solution containing 1:50 diluted FITC-conjugated goat anti-mouse IgG and 0.5 µg/mL DAPI was added to the cell plate at 25 µL per well, and the mixture was incubated at 25 °C for 60 min.
10. After the incubation was completed, the cell plate was washed 4 times with 200 µL of washing solution per well, 3 min each time.
11. After removal of the liquid, corresponding fluorescence values were read on Envision: FITC: 480 nm and 530 nm; DAPI: 360 nm and 460 nm.

**Data analysis**: the original data was normalized using the equation (FITC - negative control)/(DAPI - blank control), and the normalized data was converted to inhibition using the equation (sample - positive control)/(negative control - positive control) × 100%, and the IC₅₀ value was then curve fitted using four parameters (obtained from the "log(inhibitor) vs. response-variable slope" model in GraphPad Prism). Table 1 provides the inhibitory activity of the compounds disclosed herein.

**Experimental results:** the half inhibitory concentrations (IC₅₀) of the compounds disclosed herein against PARrylation are shown in Table 4.

**Table 4. Inhibitory activity of compounds disclosed herein against PARrylation**

| **Compound number** | **Parylation (IC₅₀, nM)** |
|---|---|
| Example 6_A | 19 |
| Example 11_A | 27 |
| Example 24_A | 23 |
| Example 25_A | 19 |
| Example 26_A | 25 |

**Experimental conclusion:** the compounds disclosed herein have significant inhibitory activity against PARrylation.

### Experimental Example 5: Study on Plasma Protein Binding Rate

The protein binding rates of the compounds disclosed herein in plasma of human, CD-1 mice and SD rats were determined. 796 µL of blank plasma was taken from human, CD-1 mice and SD rats, and added with 4 µL of test compound working solution (400. µM) or warfarin working solution (400 µM) to achieve a final concentration of 2 µM of both the test compound and the warfarin in the plasma sample. The samples were mixed well. The final concentration of organic phase DMSO was 0.5%; 50 µL of test compound and warfarin plasma sample was pipetted into a sample receiving plate (three parallels), and a corresponding volume of blank plasma or buffer was immediately added such that the final volume of each sample well was 100 µL. The volume ratio of plasma to dialysis buffer was 1:1, and 400 µL of stop solution was added to these samples, which were used as TO samples for determination of recovery rate and stability. The TO samples were stored at 2-8 °C for subsequent treatment with other dialyzed samples; 150 µL of the test compound and warfarin plasma sample was added to a drug delivery end of each dialysis well, and 150 µL of blank dialysis buffer was added to a corresponding receiving end of the dialysis well. The dialysis plate was then sealed with a gas permeable membrane and placed in a humid, 5% CO₂ incubator and incubated at 37 °C for 4 h while shaking at about 100 rpm. After the dialysis was completed, 50 µL of the dialyzed buffer sample and dialyzed plasma sample were pipetted to a new sample receiving plate. A corresponding volume of corresponding blank plasma or buffer was added to the samples such that the final volume of each sample well was 100 µL, and the volume ratio of plasma to dialysis buffer was 1:1. All samples were subjected to LC/MS analysis after protein precipitation, and the protein binding rates and the recovery rates were calculated by the following formulas: protein unbinding rate (%) = 100 × drug concentration passing through dialysis membrane/drug concentration not passing through dialysate; protein binding rate (%) = 100 - protein unbinding rate (%); recovery rate (%) = 100 × (drug concentration passing through dialysis membrane + drug concentration not passing through dialysate)/total drug concentration before dialysis.

**Experimental results:** the results are shown in Table 5.

**Experimental conclusion:** the compounds disclosed herein have good plasma protein binding rate.

**Experimental conclusion:** the compounds disclosed herein have an appropriate plasma protein binding rate.

**Table 5. Plasma protein binding rates of compounds disclosed herein in different species**

| **Compound number** | **Plasma protein binding rate** | | |
|---|---|---|---|
| | **Human** | **CD-1 mice** | **SD rats** |
| Example 6_A | 81.9% | 79.2% | 82.4% |
| Example 11_A | 76.5% | 89.9% | 96.5% |
| Example 24_A | 92.7% | 96.3% | 96.4% |
| Example 25_A | 85.1% | 89.6% | 92.3% |
| Example 26_A | 94.3% | 92.0% | 90.3% |

### Experimental Example 6: Study on Inhibition Against Cytochrome P450 Isoenzyme

The inhibition of the test compounds against different subtypes of the human cytochrome P450 isoenzyme was determined. Test compounds, a standard inhibitor (100× final concentration) and a mixed substrate working solution were prepared; the microsomes frozen in a refrigerator at -80 °C were taken out and thawed. 2 µL of a solution of the test compound and the standard inhibitor was added to corresponding wells, and 2 µL of a corresponding solvent was added to a non-inhibitor control (NIC) well and a blank control (Blank) well; then, 20 µL of a solution of mixed substrate was added to corresponding wells except for the Blank well (adding 20 µL of PB to the Blank well); a human liver microsome solution (marking the date after use and immediately putting back to a refrigerator) was prepared and then added to all wells at 158 µL per well; the sample plate was put into a 37 °C water bath for pre-incubation, and then a coenzyme factor (NADPH) solution was prepared; after 10 min, the NADPH solution was added to all the wells at 20 µL per well, and the sample plate was shaken to mix the mixture well and then incubated in a 37 °C water bath for 10 min; at corresponding time points, 400 µL of cold acetonitrile solution (internal standard: 200 ng/mL tolbutamide and labetalol) was added to stop the reaction; after being mixed well, the mixture in the sample plate was centrifuged at 4,000 rpm for 20 min to precipitate proteins; 200 µL of supernatant was collected and added into 100 µL of water, and the mixture was mixed well and then assayed by LC/MS/MS.

**Experimental results:** the results are shown in Table 6.

**Conclusion:** the compounds disclosed herein show no or weak inhibitory effect against 5 CYP enzymes.

**Table 6. Results of inhibition of test compounds against cytochrome P450 isoenzyme**

| **Test compound** | **IC₅₀ (µM)** | | | | |
|---|---|---|---|---|---|
| | **CYP1A2** | **CYP2C9** | **CYP2C19** | **CYP2D6** | **CYP3A4-M** |
| Example 6 | >50 | >50 | 24.6 | 10.4 | >50 |
| Example 11_A | >50 | >50 | 15.5 | 19.2 | 35.0 |
| Example 24_A | 29.5 | >50 | 17.2 | 6.98 | >50 |
| Example 25_A | >50 | >50 | 8.33 | 11.6 | 32.9 |
| Example 26_A | >50 | 49.7 | 3.31 | 20.2 | 14.1 |

### Experimental Example 7. Metabolic Stability in Liver Microsomes

**Experimental objective:** to test the metabolic stability of test compounds in liver microsomes of three species. **Experimental method:** 1 µM test compound and a microsome (0.5 mg/mL) were incubated at 37 °C in the presence of an NADPH regeneration system; positive controls were testosterone (3A4 substrate), propylamine propiophenone (2D6 substrate) and diclofenac (2C9 substrate), and also at 37 °C, a positive control was incubated with a microsome (0.5 mg/mL) in the presence of an NADPH regenerating system; the reaction was stopped by direct mixing of the sample with cold acetonitrile containing an internal standard at various time points (0, 5, 10, 20, 30 and 60 min); the compound and microsome were incubated for 60 min in the absence of an NADPH regenerating system; one parallel (n = 1) was set at each time point; the samples were analyzed by LC/MS/MS; the concentration of the compound was characterized by the ratio of the analyte peak area to the internal standard peak area.

**Experimental results:** the results are shown in Table 7.

**Table 7. Stability of test compounds disclosed herein in liver microsomes of different species**

| **Compound number** | **Residual content after 60 min of incubation** | | |
|---|---|---|---|
| | **Human** | **Rat** | **Mouse** |
| Example 6_A | 53.7% | 55.4% | 45.6% |
| Example 11_A | 73.8% | 58.1% | 57.1% |
| Example 24_A | 60.4% | 60.6% | 52.3% |
| Example 25_A | 35.9% | 31.8% | 40.7% |
| Example 26_A | 29.6% | 34.5% | 57.7% |

### Experimental Example 8: Single-Dose Pharmacokinetic Study in Mice

**Experimental objective:** to evaluate the pharmacokinetic behavior by using male C57BL/6 mice as test animals and determining the drug concentrations of the compounds in the plasma, liver and cerebrospinal fluid after single-dose administration.

**Experimental method:** healthy adult male C57BL/6 mice were selected for intragastric administration. A candidate compound was mixed with an appropriate amount of 10% DMSO/90% (20% hydroxypropyl-β-cyclodextrin), vortexed and sonicated to prepare a 0.5 mg/mL clear solution for later use. After the mice were administered intravenously at 1 mg/kg and orally at 5 mg/kg, whole blood was collected at certain time points, and plasma was separated, and liver and cerebrospinal fluid were collected. After pretreatment of the samples, the drug concentration was measured by LC-MS/MS, and pharmacokinetic parameters were calculated using Phoenix WinNonlin software.

**Experimental results:** the results are shown in Table 8.

**Experimental conclusion:** the test compounds have good AUC₀₋ₗₐₛₜ and bioavailability in mice.

**Table 8. Results of pharmacokinetic experiment of test compounds in mice and rats**

| **Results of pharmacokinetic experiment (IV: 1 mg/kg PO: 5 mg/kg)** | **Rucaparib** | **Example 6** | **Example 11** | **Example 24_A** | **Example 25_A** | **Example 26_A** |
|---|---|---|---|---|---|---|
| Clearance (mL/min/kg) | 99.9 | 57.3 | 16.1 | 34.9 | 41.0 | 60.7 |
| Apparent volume of distribution (L/kg) | 13.1 | 7.44 | 2.27 | 4.21 | 9.44 | 8.22 |
| AUC₀₋ₗₐₛₜ (intravenous injection, nM. h) | 255 | 807 | 2782 | 1302 | 958 | 679 |
| AUC₀₋ₗₐₛₜ (oral, nM. h) | 145 | 1100 | 7919 | 1382 | 1186 | 1286 |
| Half life (h) | 1.78 | 1.70 | 2.20 | 2.03 | 3.02 | 2.15 |
| Maximum concentration (nM) | 24.8 | 273 | 1630 | 433 | 240 | 285 |
| Bioavailability (%) | 14.6 | 27.3 | 53.9 | 21.2 | 24.8 | 37.9 |

### Experimental Example 9: In Vivo Pharmacodynamic Study of Compounds in Subcutaneous Xenograft Tumor BALB/c Nude Mouse Model of Human Breast Cancer MDA-MB-436 Cells

**Experimental objective:** to study the efficacy *in vivo* of the test compound in subcutaneous xenograft tumor BALB/c nude mouse model of human breast cancer MDA-MB-436 cells.

### Experimental design:

**Table 9. Animal grouping and administration regimen in in vivo pharmacodynamic experiment of test compounds**

| **N¹** | **Compound treatment** | **Dosage (mg/kg)** | **Administration volume parameter (µL/g)²** | **Route of administration** | **Frequency of administration** |
|---|---|---|---|---|---|
| 6 | Vehicle | -- | 10 | PO | QD × 28 days |
| 6 | Example 25_A | 12.5 | 10 | PO | QD × 28 days |
| 6 | Example 25_A | 25 | 10 | PO | QD × 28 days |
| 6 | Example 25_A | 50 | 10 | PO | QD × 28 days |

| | | | | | |
|---|---|---|---|---|---|
| Note: 1. N: the number of mice in each group; 2. administration volume: 10 µL/g based on the weight of mice. If body weight decreases by more than 15%, the administration regimen should be adjusted accordingly. 3. QD: once daily; PO: oral administration. | | | | | |

**Experimental materials:** week age and body weight: female BALB/c nude mice, 6-8 weeks old, 18-22 g of body weight. The experiment started after 3-7-days of adaptive feeding. The animal information card of each cage provides the following information about the animals: number, sex, strain, receiving date, administration regime, experiment number, group and starting date of the experiment. All the cages, padding and drinking water were sterilized before use. The cages, feed and drinking water were changed twice a week. The experimental animals were identified with ear tags. Test samples: Example 24_A and Example 25_A. All the test samples were prepared with 10% DMSO + 90% (20% HP-β-CD) as vehicle, and the blank control group was administered with the vehicle alone.

### Experimental method:

1. Cell culturing. Human breast cancer MDA-MB-436 cells (ATCC, Manassas, VA, catalog No.: HTB-130) were cultured in an RPMI-1640 culture medium containing 10% fetal bovine serum and 1% Anti-anti through *in vitro* monolayer culture in an incubator at 37 °C/5% CO₂. The cells were digested with trypsin-EDTA twice a week for passaging as per conventional practice. At a cell saturation of 80%-90% and a required number, the cells were collected, counted and inoculated.
2. Tumor cell inoculation (tumor inoculation). 0.2 mL (1×10⁷ cells) of MDA-MB-436 cells (along with matrigel in a volume ratio of 1:1) was subcutaneously inoculated on the right back of each mouse, and the mice were randomly grouped when the average tumor volume was 318 mm³.
3. Daily observation of experimental animals: animals were monitored daily for health and death, and routine examinations include observation of the effect of tumor growth and drug treatment on the daily performance of the animals, such as behavioral activities, food and water intake, weight changes, appearance, or other abnormal conditions.
4. Tumor measurements and experimental indices: the experimental indices were to investigate whether the tumor growth was inhibited, the tumor growth was delayed or the tumor was cured. Tumor diameters were measured twice weekly using a vernier caliper. The tumor volume was calculated using the following formula: V = 0.5a × b², where a and b represent the long diameter and short diameter of the tumor, respectively. The anti-tumor therapeutic effect of the compound was evaluated by TGI (%) or relative tumor proliferation rate T/C (%). TGI (%) refers to the rate of tumor growth inhibition. Calculation of TGI (%): TGI (%) = [(1 - (average tumor volume at the end of administration in a treatment group - average tumor volume at the start of administration of the treatment group))/(average tumor volume at the end of treatment of the solvent control group - average tumor volume at the start of treatment of the solvent control group)] × 100%. The calculation formula for relative tumor proliferation rate T/C (%) was as follows: T/C (%) = T_{RTV}/C_{RTV} × 100% (T_{RTV}: RTV of treatment group; C_{RTV}: RTV of negative control group). Relative tumor volume (RTV) was calculated based on the results of tumor measurement. The calculation formula was: RTV = Vt /V0, wherein V0 was the average tumor volume measured at the time of grouping and administration (i.e., d0), Vt was the average tumor volume at a certain measurement, and the data of T_{RTV} and C_{RTV} used were obtained on the same day.
5. Statistical analysis: including mean and standard error of mean (SEM) of tumor volume at each time point for each group. The treatment group showed the best treatment effect on day 27 after the administration at the end of the experiment, and therefore statistical analysis was performed based on the data to evaluate the differences between groups. The experimental data were analyzed using a one-way ANOVA method and a Games-Howell method. All data analysis was performed with SPSS 17.0. p < 0.05 was defined as a significant difference. **Experimental results:** the results are shown in Table 10.

**Table 10. Evaluation of anti-tumor efficacy of test compound (based on tumor volume calculated on day 27 after administration)**

| **Groups** | **Tumor volume (mm³)^{a} (day 27)** | **T/C^{b} (%)** | **TGI^{b} (%)** | ***p* value^{c}** |
|---|---|---|---|---|
| Vehicle | 1586±267 | -- | -- | -- |
| Example 25_A (12.5 mg/kg) | 377±149 | 23.79 | 95.34 | 0.051 |
| Example 25_A (25 mg/kg) | 132±32 | 8.31 | 114.69 | 0.025 |
| Example 25_A (50 mg/kg) | 91±14 | 5.76 | 117.86 | 0.023 |

| | | | | |
|---|---|---|---|---|
| Note: a. mean±SEM. b. Tumor growth inhibition was calculated based on T/C and TGI. c. The *p* value is the significance of difference between the tumor volume of each treatment group and the tumor volume of the vehicle group on day 27. | | | | |

**Experimental conclusion:** the compound disclosed herein has good tumor inhibition effect.

## Claims

1. A compound of formula (II), an isomer thereof or a pharmaceutically acceptable salt thereof, 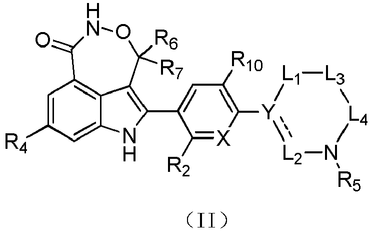 wherein,
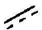 is selected from the group consisting of a single bond and a double bond;
X is selected from the group consisting of CR₃ and N;
Y is selected from the group consisting of CR₁ and C;
L₁ is selected from the group consisting of a single bond and -(CR₈R₉)ₙ;
L₂ is selected from the group consisting of a single bond, -CR₈R₉- and =CH-;
L₁ and L₂ are not single bonds at the same time;
when L₂ is selected from a single bond, 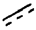 is selected from a single bond;
L₃ and L₄ are each independently selected from -CR₈R₉-;
n is 1 or 2;
R₁ is selected from the group consisting of H, D, F, Cl, Br, I and C₁₋₃ alkyl, wherein the C₁₋₃ alkyl is optionally substituted with 1, 2 or 3 Rₐ, and when L₂ is selected from =CH-, R₁ is absent;
R₂ and R₁₀ are each independently selected from the group consisting of H, F, Cl, Br, I and C₁₋₃ alkyl, wherein the C₁₋₃ alkyl is optionally substituted with 1, 2 or 3 R_{b};
R₃ is selected from the group consisting of H, F, Cl, Br, I, CN and C₁₋₃ alkyl, wherein the C₁₋₃ alkyl is optionally substituted with 1, 2 or 3 R_{c};
R₄ is selected from the group consisting of H and F;
R₅ is selected from the group consisting of H and C₁₋₃ alkyl, wherein the C₁₋₃ alkyl is optionally substituted with 1, 2 or 3 R_{d};
R₆ and R₇ are each independently selected from the group consisting of H and D;
R₈ and R₉ are each independently selected from the group consisting of H, F, Cl, Br, I and C₁₋₃ alkyl, wherein the C₁₋₃ alkyl is optionally substituted with 1, 2 or 3 Rₑ, or
R₈ and R₉, together with a same carbon atom connected thereto, form ring A optionally substituted with 1, 2 or 3 R_{g};
ring A is selected from the group consisting of C₃₋₈ cycloalkyl and 3-8 membered heterocycloalkyl;
Rₐ, R_{b}, R_{c}, R_{d}, Rₑ and R_{g} are each independently selected from the group consisting of F, Cl, Br, I, OH, CN, NH₂, COOH, C(=O)NH₂, CH₃, CH₃CH₂, CF₃, CHF₂, CH₂F, NHCH₃ and N(CH₃)₂;
the 3-8 membered heterocycloalkyl comprises 1, 2, 3 or 4 atoms or groups of atoms each independently selected from the group consisting of O, N, S and NH.

2. The compound, the isomer thereof or the pharmaceutically acceptable salt thereof according to claim 1, selected from formula (II-1) 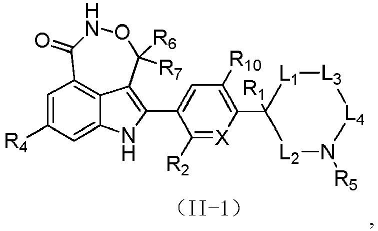 wherein
R₁, R₂, X, R₄, R₅, R₆, R₇, R₁₀, L₁, L₂, L₃ and L₄ are as defined in claim 1.

3. The compound, the isomer thereof or the pharmaceutically acceptable salt thereof according to claim 1, wherein R₁ is selected from the group consisting of H, D, F and CH₃.

4. The compound, the isomer thereof or the pharmaceutically acceptable salt thereof according to claim 1, wherein R₂ and R₁₀ are each independently selected from the group consisting of H and F.

5. The compound, the isomer thereof or the pharmaceutically acceptable salt thereof according to claim 1, wherein R₃ is selected from the group consisting of H, F, CN, Cl and CF₃; preferably, R₃ is selected from the group consisting of H and F.

6. The compound, the isomer thereof or the pharmaceutically acceptable salt thereof according to claim 1, wherein R₅ is selected from the group consisting of H, methyl, ethyl, propyl and isopropyl, wherein the methyl, ethyl, propyl and isopropyl are optionally substituted with 1, 2, or 3 R_{d}; preferably, R₅ is selected from the group consisting of H, methyl, 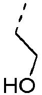 and isopropyl.

7. The compound, the isomer thereof or the pharmaceutically acceptable salt thereof according to claim 1, wherein L₁ is selected from the group consisting of a single bond and -CR₈R₉-.

8. The compound, the isomer thereof or the pharmaceutically acceptable salt thereof according to claim 1, wherein L₁ is selected from -CR₈R₉-, and L₂ is selected from -CR₈R₉-.

9. The compound, the isomer thereof or the pharmaceutically acceptable salt thereof according to claim 1, wherein L₁ is selected from -CR₈R₉-, and L₂ is selected from a single bond.

10. The compound, the isomer thereof or the pharmaceutically acceptable salt thereof according to claim 1, wherein R₈ and R₉ are each independently selected from the group consisting of H and F; preferably, both R₈ and R₉ are selected from H, or one of R₈ and R₉ is selected from H and the other is selected from F.

11. The compound, the isomer thereof or the pharmaceutically acceptable salt thereof according to claim 1, wherein R₆ and R₇ are both H.

12. The compound, the isomer thereof or the pharmaceutically acceptable salt thereof according to claim 1, wherein the structural unit 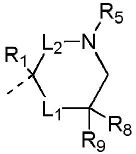 is selected from the group consisting of

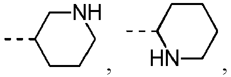

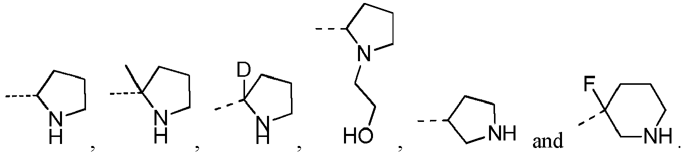

13. The compound, the isomer thereof or the pharmaceutically acceptable salt thereof according to claim 1, wherein the structural unit 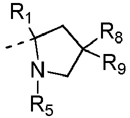 is selected from the group consisting of

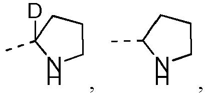

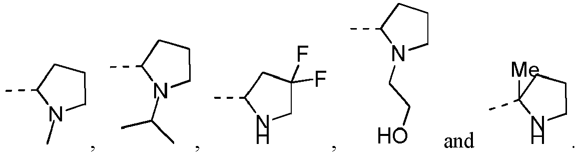

14. The compound, the isomer thereof or the pharmaceutically acceptable salt thereof according to claim 1, wherein the structural unit 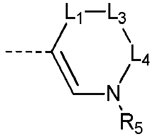 is selected from

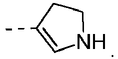

15. The compound, the isomer thereof or the pharmaceutically acceptable salt thereof according to claim 1, wherein the structural unit 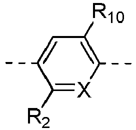 is selected from the group consisting of

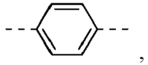

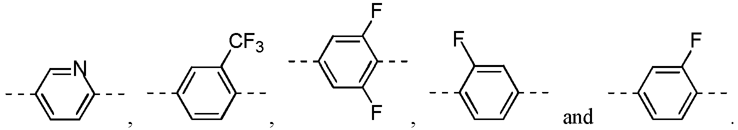

16. The compound, the isomer thereof or the pharmaceutically acceptable salt thereof according to claim 1, wherein the structural unit 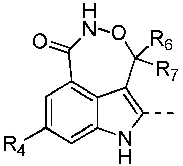 is selected from the group consisting of

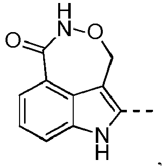

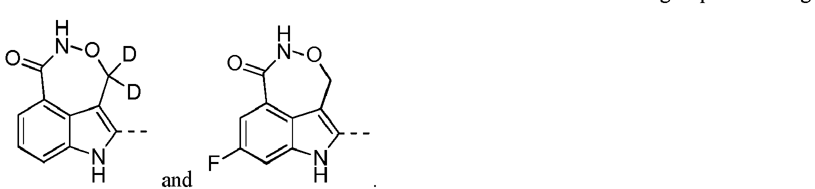

17. A compound of a formula below, an isomer thereof or a pharmaceutically acceptable salt thereof, selected from the group consisting of

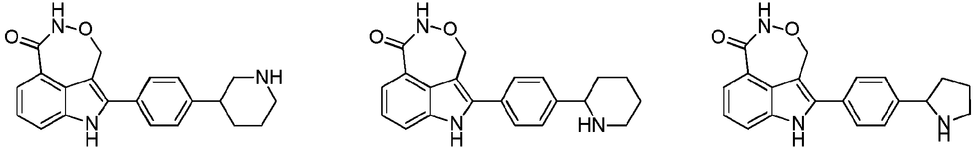

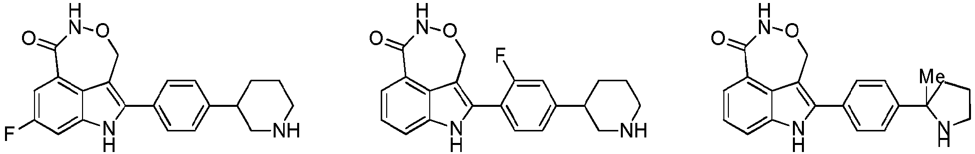

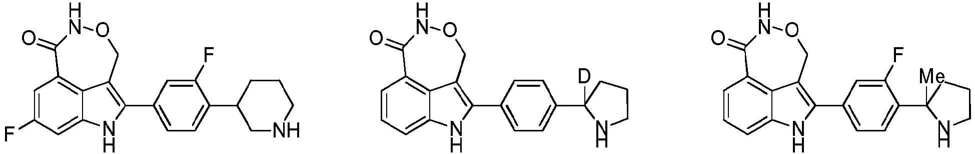

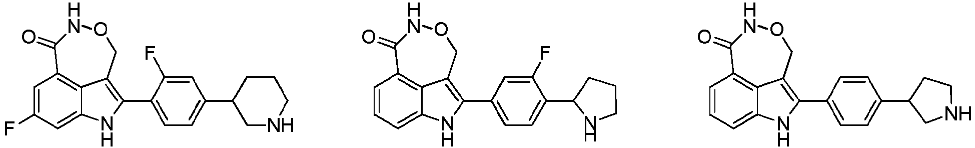

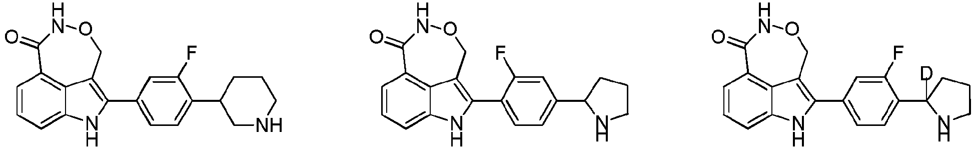

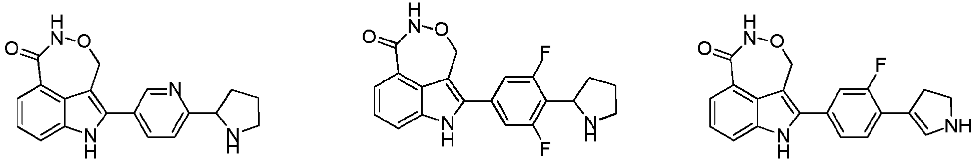

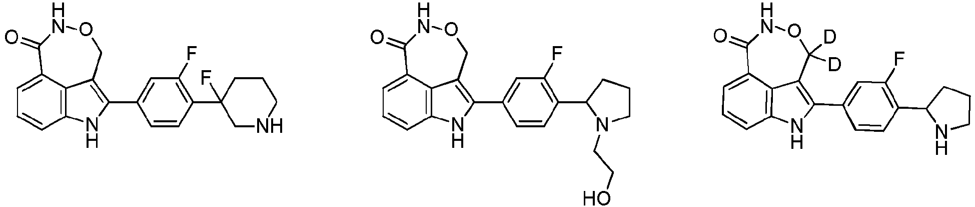

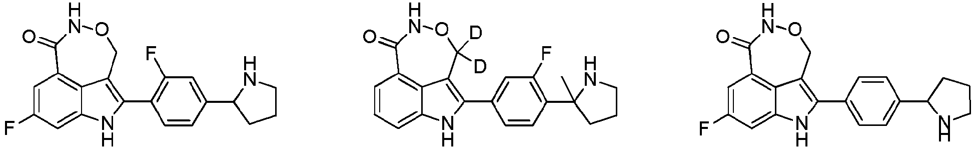

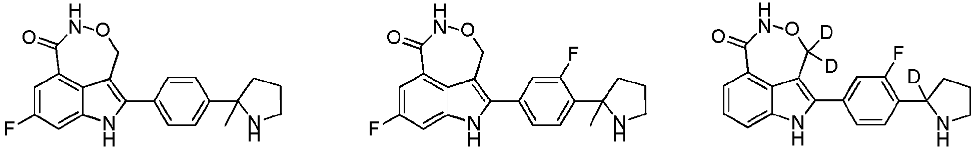

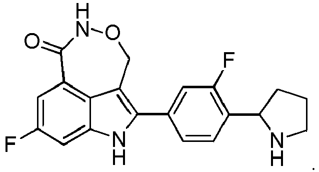

18. The compound, the isomer thereof or the pharmaceutically acceptable salt thereof according to claim 17, selected from the group consisting of

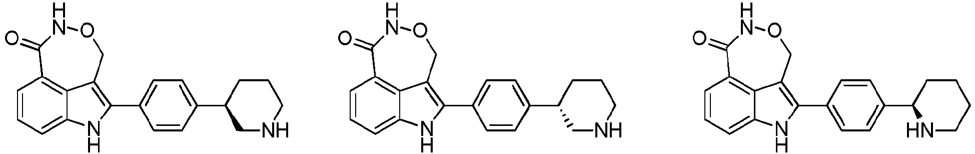

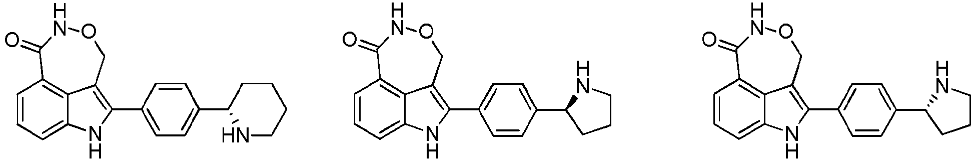

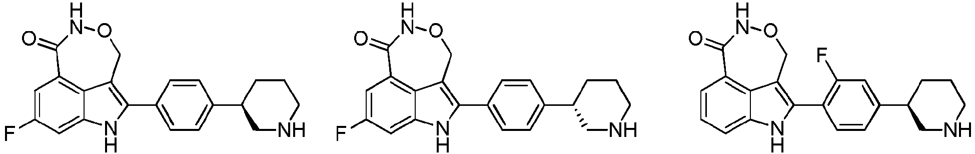

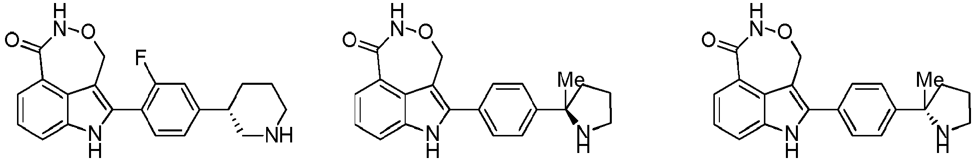

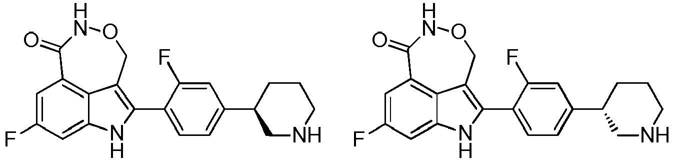

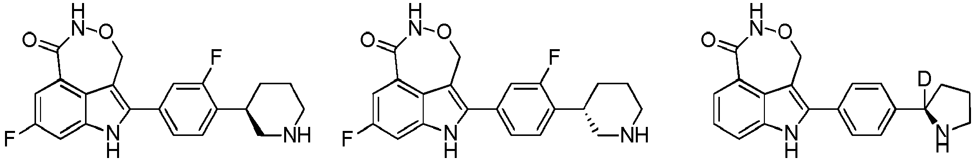

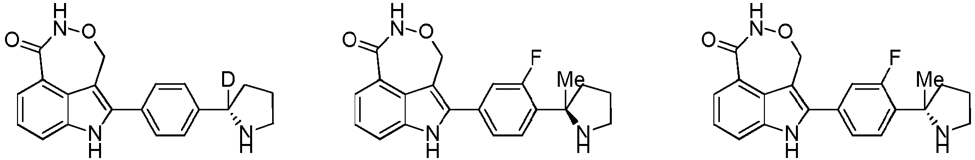

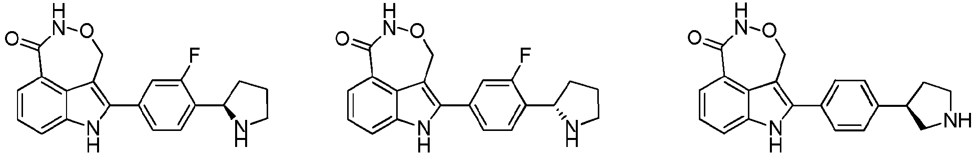

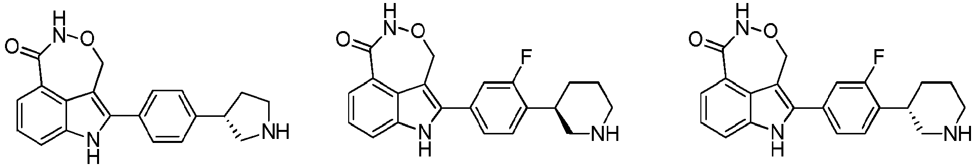

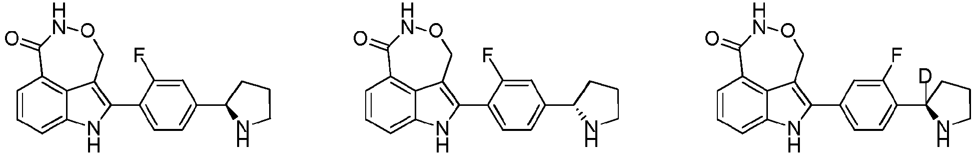

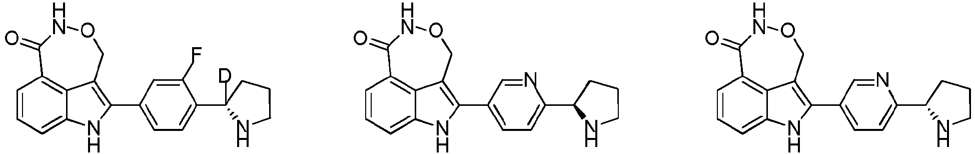

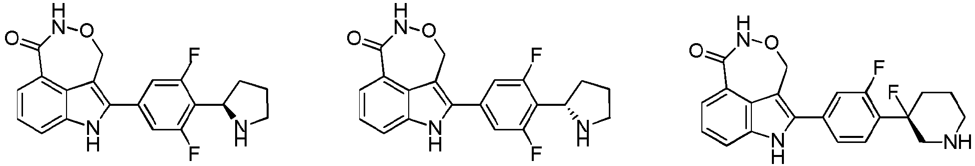

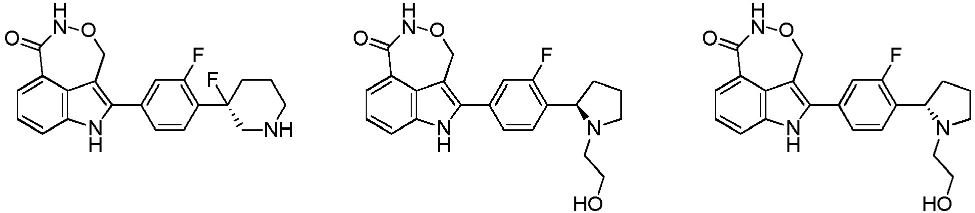

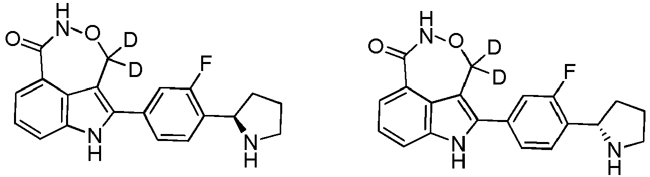

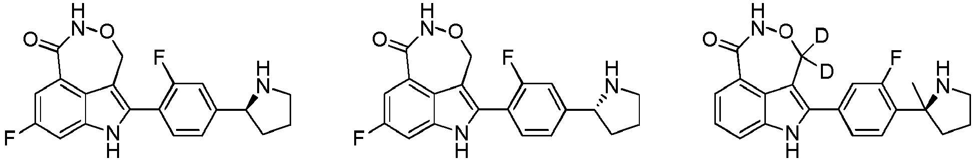

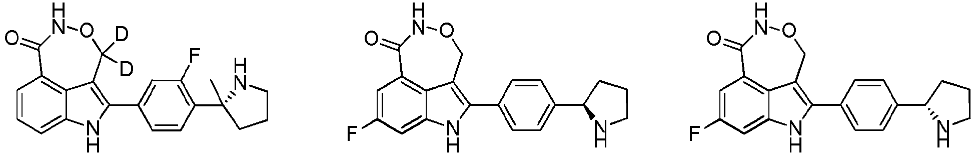

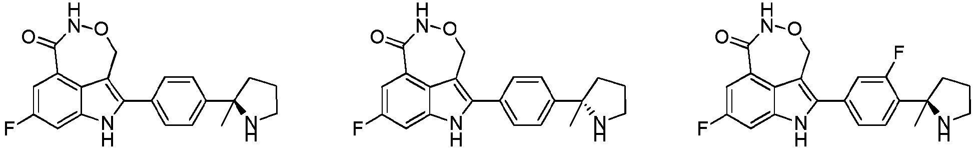

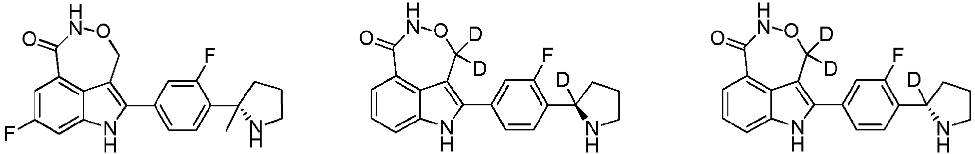

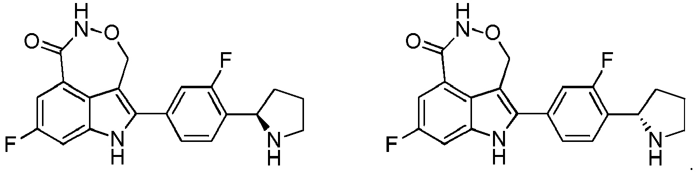

19. A pharmaceutical composition comprising a therapeutically effective amount of the compound, the isomer thereof or the pharmaceutically acceptable salt thereof according to any one of claims 1-18 and a pharmaceutically acceptable carrier.

20. Use of the compound, the isomer thereof or the pharmaceutically acceptable salt thereof according to any one of claims 1-18 in preparing a medicament for use in treating a disease related to PARP receptor.

21. A method for treating a disease related to PARP receptor, comprising administering to a mammal, preferably a human, in need of such treatment a therapeutically effective amount of the compound, the isomer thereof or the pharmaceutically acceptable salt thereof according to any one of claims 1-18.
